# EUROPEAN PATENT SPECIFICATION

(11) **EP 2 488 186 B1**
(45) Date of publication and mention of the grant of the patent: **24.05.2017**
(21) Application number: 10823668.8
(22) Date of filing: 15.10.2010
(51) Int. Cl.: G01N 33/574, A61K 39/00, A61P 35/00, C12N 15/113, A61K 31/7088

(54) **ANTI-NEOPLASTIC USES OF ARTEMIN ANTAGONISTS**
ANTINEOPLASTISCHE VERWENDUNG VON ARTEMINANTAGONISTEN
UTILISATIONS ANTINÉOPLASIQUES D'ANTAGONISTES D'ARTÉMINE

(30) Priority: 16.10.2009 US 252513 P
(43) Date of publication of application: 22.08.2012
(73) Proprietor: National University of Singapore, Singapore 119077 (SG)
(72) Inventor: LOBIE, Peter Edward, Singapore 117456 (SG)
(74) Representative: Casley, Christopher Stuart
(86) International application number: PCT/NZ2010/000207
(87) International publication number: WO 2011/046457

(56) References cited:
- WO-A1-2009/002193
- WO-A2-2009/020964
- J KANG ET AL: "Artemin is estrogen regulated and mediates antiestrogen resistance in mammary carcinoma", ONCOGENE, vol. 29, no. 22, 22 March 2010 (2010-03-22), pages 3228-3240, XP055065477, ISSN: 0950-9232, DOI: 10.1038/onc.2010.71
- VIJAY PANDEY ET AL: "Artemin Reduces Sensitivity to Doxorubicin and Paclitaxel in Endometrial Carcinoma Cells through Specific Regulation of CD24.", TRANSLATIONAL ONCOLOGY, vol. 3, no. 4, 1 January 2010 (2010-01-01) , pages 218-229, XP055065485, ISSN: 1936-5233
- KANG, J. ET AL.: 'Artemin is Oncogenic for Human Mammary Carcinoma Cells' ONCOGENE vol. 28, 2009, pages 2034 - 2045
- WARNER A H ET AL: "Artemin is an RNA-binding protein with high thermal stability and potential RNA chaperone activity", ARCHIVES OF BIOCHEMISTRY AND BIOPHYSICS, ACADEMIC PRESS, US, vol. 424, no. 2, 15 April 2004 (2004-04-15), pages 189-200, XP004496874, ISSN: 0003-9861, DOI: 10.1016/J.ABB.2004.02.022
- BALOH R H ET AL: "ARTEMIN, A NOVEL MEMBER OF THE GDNF LIGAND FAMILY, SUPPORTS PERIPHERAL AND CENTRAL NEURONS AND SIGNALS THROUGH THE GFRALPHA3-RET RECEPTOR COMPLEX", NEURON, CAMBRIDGE, MA, US, vol. 21, no. 6, 1 December 1998 (1998-12-01), pages 1291-1302, XP000990780, DOI: 10.1016/S0896-6273(00)80649-2

## Description

### FIELD OF THE INVENTION

The invention relates to methods for the prophylaxis and treatment of breast cancer using one or more antagonists of artemin function, such as anti-artemin antibodies and antibody fragments, and uses of these antagonists. In particular, the invention relates to the resensitisation of therapy-resistance breast cancer cells to anti-cancer therapies by antagonism of artemin functionality.

### BACKGROUND OF THE INVENTION

Resistance of tumor cells to anti-cancer therapies, such as therapies utilising chemotherapeutic agents, is a significant and serious problem in the clinical management of breast cancer. Systemic anti-cancer agents are generally active at the beginning of therapy in approximately 90% of primary breast cancers and approximately 50% of metastases. However, progression occurs after a variable period of time, such that resistance to therapy is not only common but expected. Reversal of resistance is thus a major goal in breast cancer treatment strategies.

WO 2009/002193 describes polypeptides, polynucleotides, and antibodies, for Artemin and related ligands, including Persephin (PSPN).

Kang et al., Oncogene, 2009, Vol. 28, pp. 2034-2045, describes the finding that Artemin is oncogenic for human mammary carcinoma cells.

Kang et al., Oncogene, 2010, Vol. 29, No. 22, pp. 3228-3240, which was published after the priority date of the present patent application, describes the finding that Artemin is estrogen regulated and mediates antiestrogen resistance in mammary carcinoma.

Pandey et al., Translational Oncology, 2010, Vol. 3, No. 4, pp. 218-229, which was published after the priority date of the present patent application, describes the finding that Artemin reduces sensitivity to Doxorubicin and Paclitaxel in endometrial carcinoma cells through specific regulation of CD24.

There is a continuing need for new approaches to improving the treatment of breast cancer, including new approaches to restore or enhance the anti-cancer activity of chemotherapeutics and other cancer therapies.

It is an object of the present invention to go some way to achieving the abovementioned improved breast cancer treatments, or to at least provide the public with useful choice.

### SUMMARY OF THE INVENTION

Accordingly, in a first aspect the present invention provides an antagonist of artemin function for use in reversing, wholly or in part, the resistance of a breast cancer-burdened patient to a cancer therapeutic, wherein the antagonist of artemin function is an antisense polynucleotide, an RNAi polynucleotide, or an antibody capable of inhibiting artemin functionality. In particular, the antibody may be a monoclonal antibody (mAb).

In some cases, the cancer therapeutic to which resistance has been induced or mediated may be a chemotherapeutic. In particular, the chemotherapeutic to which said tumours are resistant may be an estrogen receptor antagonist or an aromatase inhibitor. In particular embodiments, the estrogen receptor antagonist may be tamoxifen, toremifene, idoxifene, arzoxifene, raloxifene, or fulvestrant, or the aromatase inhibitor may be formestane, anastrozole, letrozole, vorozole, or exemestane.

In some cases, the cancer therapeutic to which said tumours are resistant may be radiation therapy.

In some cases, reversing resistance comprises resensitising to treatment one or more breast cancer cells that are resistant to treatment with a cancer therapeutic.

In a second aspect, the present invention provides an antagonist of artemin function for use in pre-treating a tumour-burdened patient prior to administration to the patient of an amount of a cancer therapeutic effective to induce an anti-tumour effect in said patient, wherein the antagonist of artemin function is an antisense polynucleotide, an RNAi polynucleotide, or an antibody capable of inhibiting artemin functionality.

In a third aspect, the present invention provides a composition for use in reversing resistance to treatment with a cancer therapeutic in a cancer cell, the composition comprising an antagonist of artemin function, wherein the antagonist of artemin function is an antisense polynucleotide, an RNAi polynucleotide, or an antibody capable of inhibiting artemin functionality.

In some cases, the composition for use of the third aspect of the invention additionally comprises a therapeutic agent, wherein the composition is for use in inhibiting one or more breast cancer cells that are resistant to treatment with a cancer therapeutic. In particular, the inhibition may be selected from the group consisting of: inhibition of growth, including inhibition of anchorage-independent growth, inhibition of cell survival, inhibition of proliferation, inhibition of mitosis, inhibition of cell-cycle progression, inhibition of metastasis, inhibition of cell motility, and induction of apoptosis.

In a fourth aspect the present invention provides a method of determining or monitoring resistance to a therapeutic agent in one or more cancer cells *in vitro,* the method comprising contacting one or more cancer cells with an antagonist of artemin function and the therapeutic agent, and measuring inhibition of the one or more cancer cells, wherein the antagonist of artemin function is an antisense polynucleotide, an RNAi polynucleotide, or an antibody capable of inhibiting artemin functionality.

In a fifth aspect, the present invention provides a method of determining the presence or extent of BCL2-mediated resistance in cancer cells exhibiting resistance or of distinguishing in one or more cancer cells BCL2-mediated resistance to a cancer therapy from non-BCL2-mediated resistance to the cancer therapy, the method comprising administration to one or more cancer cells *in vitro* of an effective amount of an antagonist of artemin function and a cancer therapeutic to which the cancer cells are resistant and measuring one or more of cancer cell survival, cancer cell motility, cancer cell proliferation, cancer cell mitosis, cell-cycle progression, cancer cell metastasis, or cancer cell apoptosis, wherein the antagonist of artemin function is an antisense polynucleotide, an RNAi polynucleotide, or an antibody capable of inhibiting artemin functionality.

In accordance with any of the above aspects of the present invention, the following may apply. The breast cancer-burdened patient may have breast cancer in situ. The breast cancer-burdened patient may have invasive breast cancer.

In various embodiments the resistance is or has been wholly or partly induced or mediated by a function of artemin.

In accordance with any aspect of the present invention, the antagonist of artemin function is an anti-sense polynucleotide, an RNAi polynucleotide, or an antibody capable of inhibiting artemin functionality.

In one embodiment, the antibody is a monoclonal antibody (mAb).

In various embodiments, the cancer therapeutic to which resistance has been induced or mediated is a chemotherapeutic.

In various embodiments, for example when the patient has one or more mammary tumours, the chemotherapeutic to which said tumours are resistant is an estrogen receptor antagonist.

In various embodiments, the estrogen receptor antagonist is tamoxifen, toremifene, idoxifene, arzoxifene, raloxifene, or fulvestrant.

In various embodiments, for example when the patient has one or more mammary tumours, the chemotherapeutic to which said tumours are resistant is an aromatase inhibitor.

In various embodiments, the aromatase inhibitor is formestane, anastrozole, letrozole, vorozole, or exemestane.

In various embodiments, the chemotherapeutic to which said tumours are resistant is paclitaxel or doxorubicin.

In various embodiments, the cancer therapeutic is radiation therapy, including external beam radiotherapy, stereotactic radiotherapy, 3D conformal radiotherapy, intensity-modulated radiotherapy, particle therapy, or radioisotope therapy.

In various embodiments, the patient has tumours for which BCL2 is a marker of resistance.

The following embodiments may relate to any of the aspects of the invention as described herein.

In one embodiment, the breast cancer is estrogen receptor (ER) positive or comprises one or more ER positive cells. In one embodiment, administration of an antagonist of artemin function to an ER positive cell reduces anchorage independent growth, including E2-stimulated anchorage independent growth.

In another embodiment, the breast cancer is estrogen receptor (ER) negative or comprises one or more ER negative cells.

In various embodiments, the antagonist of artemin function is an anti-artemin antibody, such as an anti-artemin polyclonal antibody or an anti-artemin monoclonal antibody, a chimeric anti-artemin antibody, a CDR-grafted anti-artemin antibody, a humanized anti-artemin antibody, an anti-artemin Fab, an anti-artemin Fab', an anti-artemin F(ab')2, an anti-artemin Fv, an anti-artemin disulfide linked Fv, an anti-artemin scFv, an anti-artemin single domain antibody, an anti-artemin diabody, an anti-artemin multispecific antibody, an anti-artemin dual specific antibody, and an anti-artemin bispecific antibody.

In another embodiment, the antagonist of artemin function is an antisense polynucleotide, an siRNA polynucleotide, an iRNA, or a short-hairpin RNA (shRNA) polynucleotide.

Also disclosed herein is an antagonist of artemin function, which comprises the extracellular domain of a receptor of which artemin is a ligand, including the extracellular domain of a receptor of which artemin is a high affinity ligand. In one example of the disclosure, the antagonist of artemin function is the extracellular domain of the GFRα3 receptor, or a functional variant or fragment thereof.

In various embodiments, the chemotherapeutic agent is selected from the group comprising tamoxifen, fulvestrant, paclitaxel, or doxorubicin. In other embodiments the chemotherapeutic is selected from the group comprising toremifene, idoxifene, arzoxifene, raloxifene, formestane, anastrozole, letrozole, vorozole, and exemestane.

In various embodiments, the chemotherapeutic agent is selected from the group comprising mitotic inhibitors, such as vinca alkaloids, including vincristine, vinblastine, vinorelbine, vindesine, vinflunine, podophyllotoxin, taxanes, including docetaxel, larotaxel, ortataxel, paclitaxel, and tesetaxel, and epothilones, such as ixabepilone; topoisomerase I inhibitors, such as topotecan, irinotecan, camptothecin, rubitecan, and belotecan, topoisomerase type II inhibitors, including amsacrine, etoposide, etoposide phosphate, and teniposide, anthracyclines, such as aclarubicin, daunorubicin, doxorubicin, epirubicin, idarubicin, amrubicin, pirarubicin, valrubicin, and zorubicin, and anthracenediones, such mitoxantrone and pixantrone; antimetabolites, including dihydrofolate reductase inhibitors, such as aminopterin, methotrexate, pemetrexed, thymidylate synthase inhibitors, such as raltitrexed and pemetrexed, adenosine deaminase inhibitors, including pentostatin, halogenated or ribonucleotide reductase inhibitors, such as cladribine, clofarabine, and fludarabine, thiopurines, including thioguanine and mercaptopurine, thymidylate synthase inhibitors, including fluorouracil, capecitabine, tegafur, carmofur, and floxuridine, DNA polymerase inhibitors, such as cytarabine, ribonucleotide reductase inhibitors, such as gemcitabine, hypomethylating agents, including azacitidine, and decitabine, and ribonucleotide reductase inhibitors, such as hydroxyurea; cell-cycle nonspecific antineoplastic agents, including alkylating agents such as nitrogen mustards, including mechlorethamine, cyclophosphamide, ifosfamide, trofosfamide, chlorambucil, melphalan, prednimustine, bendamustine, uramustine, estramustine, nitrosoureas, including carmustine, lomustine, semustine, fotemustine, nimustine, ranimustine, and streptozocin, alkyl sulfonates, including busulfan, mannosulfan, and treosulfan, aziridines, including carboquone, thioTEPA, triaziquone, and triethylenemelamine, alkylating-like agents, including platinum agents such as cisplatin, carboplatin, oxaliplatin, nedaplatin, triplatin tetranitrate, satraplatin, hydrazines, such as procarbazine, triazenes, such as dacarbazine, temozolomide, altretamine, and mitobronitol, and streptomycins, such as actinomycin, bleomycin, daunomycin,mitomycin, and plicamycin; photosensitizers, including aminolevulinic acid, methyl aminolevulinate, efaproxiral, and porphyrin derivatives, such as porfimer sodium, talaporfin, temoporfin, and verteporfin; enzyme inhibitors, including farnesyltransferase inhibitors such as tipifarnib, cyclin-dependent kinase inhibitors, such as alvocidib and seliciclib, proteasome inhibitors, such as bortezomib, phosphodiesterase inhibitors, such as anagrelide, IMP dehydrogenase inhibitors, such as tiazofurine, lipoxygenase inhibitors, such as masoprocol, and PARP inhibitors, such as olaparib; receptor antagonists, such as endothelin receptor antagonists including atrasentan, retinoid X receptor antagonists, such as bexarotene, and testolactone; and other chemotherapeutics, including amsacrine, trabectedin, retinoids such as alitretinoin and tretinoin, arsenic trioxide, asparagine depleters such as asparaginase or pegaspargase, celecoxib, demecolcine, elesclomol, elsamitrucin, etoglucid, and lonidamine.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

Other aspects and embodiments of the invention are described herein below.

### BRIEF DESCRIPTION OF THE DRAWINGS

These and other aspects of the present invention, which should be considered in all its aspects, will become apparent from the following description, which is given by way of example only, with reference to the accompanying figures, in which:

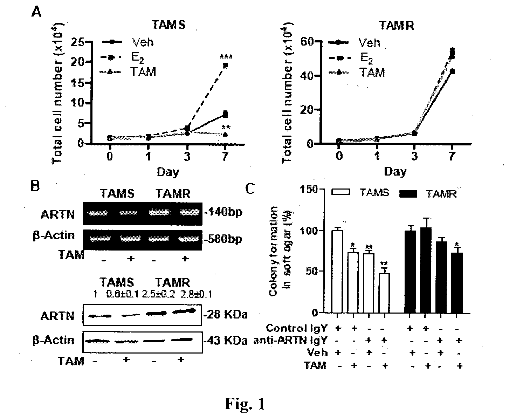

**Figure 1****.** Shows that breast cancer cells that have acquired tamoxifen resistance are resensitised to chemotherapy by antagonism of artemin, as described herein in Example 1. **(A)** TAMS and TAMR cells treated with either DMSO (vehicle), 10 nM E₂ or 1 µM tamoxifen over a period of 7 days. **(B)** TAMS and TAMR cells treated with either DMSO (vehicle) or 1 µM tamoxifen for 72 hours. Top panel: *ARTN*mRNA level as measured by semi-quantitative RT-PCR. Bottom panel: soluble whole cellular extracts as identified by SDS-PAGE and probed for artemin. **(C)** Colony formation in soft agar of TAMS and TAMR cells treated with 1 µM tamoxifen alone or in combination with 400 µg/ml anti-artemin IgY for 10 days.

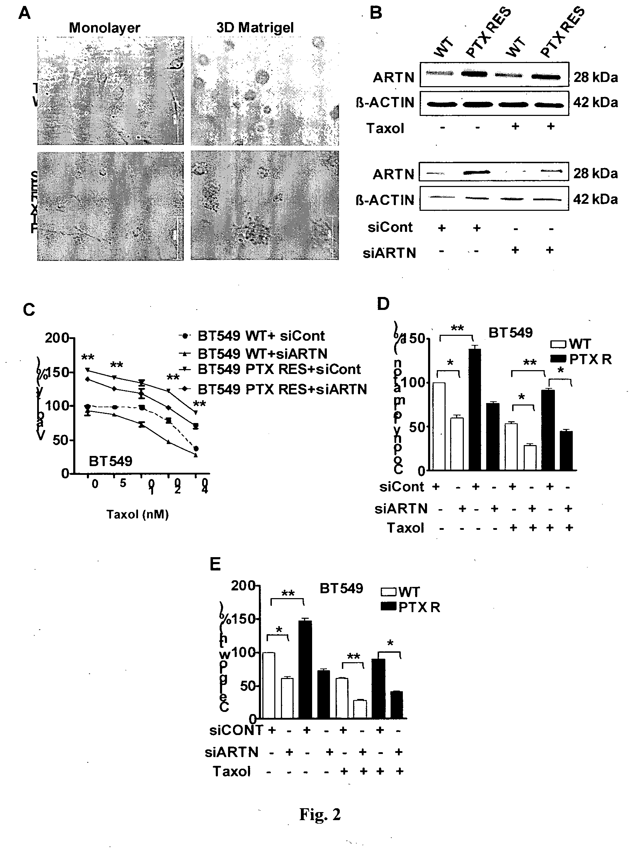

**Figure 2****.** Depletion of ARTN restores paclitaxel sensitivity in paclitaxel resistant mammary carcinoma cells, as described herein in Example 2. **(A)** Cellular and 3D morphology in monolayer and matrigel culture of BT549 wild type and BT549 paclitaxel resistant cells respectively. **(B)** Western blot for ARTN expression with or without paclitaxel in BT549 wild type and BT549 paclitaxel resistant cells, and western blot to demonstrate siRNA mediated depletion of ARTN in BT549 wild type and paclitaxel resistant cells. **(C)** Cell viability assays in monolayer culture comparing BT549 wild type and paclitaxel resistant cells ± siARTN. **(D)** Colony formation in soft agar between BT549 wild type and paclitaxel resistant cells ±siARTN. (E) Growth in 3D matrigel between BT549 wild type and paclitaxel resistant cells ±siARTN. *, p <0.05; **, p <0.01; ***, p <0.001.

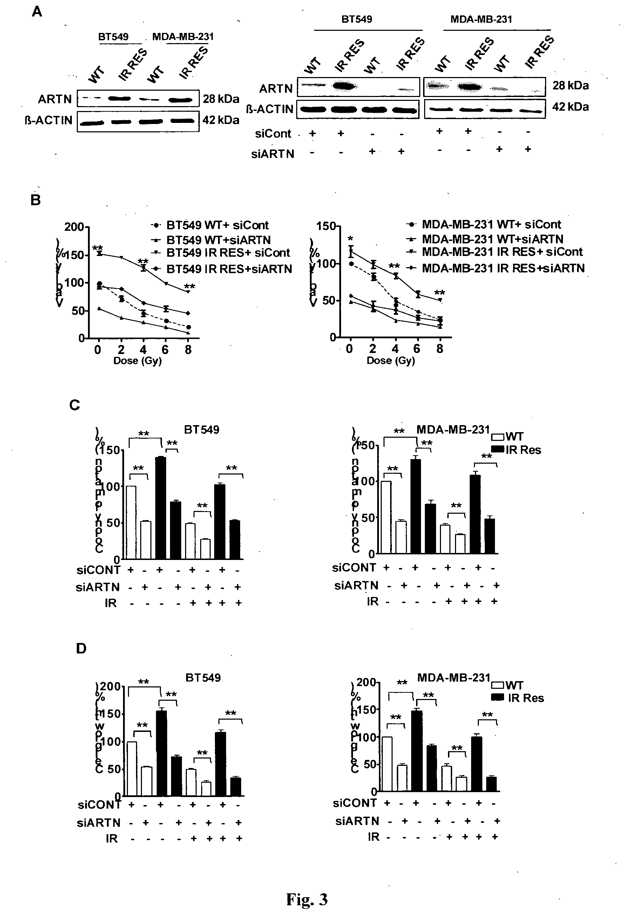

**Figure 3****.** Depletion of ARTN restores ionizing radiation (IR) sensitivity in IR resistant mammary carcinoma cells, as described herein in Example 3. **(A)** Western blot for ARTN expression with or without IR in BT549 wild type and BT549 IR resistant cells, and western blot to demonstrate siRNA mediated depletion of ARTN in BT549 and MDA-MB-231 wild type and IR resistant cells. **(B)** Cell viability assays in monolayer culture between BT549 (left panel) and MDA-MB-231 (right panel) wild type and IR resistant cells ±siARTN. **(C)** Colony formation in soft agar between BT549 (left panel) and MDA-MB-231 (right panel) wild type and IR resistant cells ±siARTN. **(D)** Growth in 3D matrigel between BT549 (left panel) and MDA-MB-231 (right panel) wild type and IR resistant cells ±siARTN. ***, *p* <0.05; **, *p <* 0.01; ***, *p* <0.001.

### DETAILED DESCRIPTION OF THE INVENTION

The following is a description of the present invention, including preferred embodiments thereof, given in general terms. The invention is further elucidated from the disclosure given under the section "Examples" which provides experimental data supporting the invention and specific examples thereof.

### Definitions

The term "antibody" should be understood in the broadest possible sense and is intended to include intact monoclonal antibodies and polyclonal antibodies. It is also intended to cover modified antibodies so long as they exhibit the desired biological activity. Antibodies encompass immunoglobulin molecules and immunologically active portions of immunoglobulin (Ig) molecules, i.e., molecules that contain an antigen binding site that specifically binds (immunoreacts with) an antigen. These include, but are not limited to, polyclonal, monoclonal, chimeric, single chain, Fc, Fab, Fab', and Fab2 fragments, and a Fab expression library. Antibody molecules relate to any of the classes IgG, IgM, IgA, IgE, IgD, and IgY, which differ from one another by the nature of heavy chain present in the molecule. These include subclasses as well, such as IgG1, IgG2, and others. The light chain may be a kappa chain or a lambda chain. In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4.

Reference herein to antibodies includes a reference to all classes, subclasses, and types. Also included are chimeric antibodies, for example, monoclonal antibodies or modifications thereof that are specific to more than one source, e.g., a mouse or human sequence. Further included are camelid antibodies or nanobodies. It will be understood that each reference to "antibodies" or any like term, herein includes intact antibodies, as well as any modifications thereof. Specifically contemplated are such mutant, variant, or derivative antibody formats as are known in the art. Nonlimiting embodiments of these are discussed below.

It has been shown that the antigen-binding function of an antibody can be performed by fragments of a full-length antibody. Such antibody embodiments may also be bispecific, dual specific, or multi-specific formats; specifically binding to two or more different antigens. Examples of binding fragments encompassed within the term "antibody" as used herein, and more specifically the term "antigen-binding portion" of an antibody include (i) a Fab fragment, a monovalent fragment consisting of the VL, VH, CL and CH2 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546, Winter et al., PCT publication WO 90/05144 A1), which comprises a single variable domain; and (vi) an isolated complementarity determining region (CDR). Furthermore, although the two domains of the Fv fragment, VL and VH, are coded for by separate genes, they can be joined using recombinant methods by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed by the term "antibody" and specifically by the term "antigen-binding portion". Diabodies are bivalent, bispecific antibodies in which VH and VL domains are expressed on a single polypeptide chain, but linked with a linker that is too short to allow for pairing between the two domains on the same polypeptide chain. The domains are thus forced to pair with complementary domains of another chain, creating two antigen binding sites (see e.g., Holliger, P., et al. (1993) Proc. Natl. Acad. Sci. USA 90:6444-6448; Poljak, R. J., et al. (1994) Structure 2:1121-1123).

The term "antisense" should be interpreted broadly. It is intended to mean any nucleic acid (preferably RNA, but including single stranded DNA) capable of binding to a transcript for artemin. It includes oligonucleotides and their derivatives (as well as salts thereof where salt-forming groups are present) that are specifically hybridizable with DNA or RNA, preferably mRNA. Such an oligonucleotide or oligonucleotide derivative can comprise nucleotide units or nucleotide analogues/derivatives thereof sufficient in number and identity to allow such hybridization. In most cases, antisense oligonucleotides and their derivatives specifically bind (hybridise) to the complementary sequence of DNA, pre-mRNA or mature mRNA, as defined by Watson-Crick base pairing.

"Altered" polynucleotides, as used herein, include those with deletions, insertions, or substitutions of different nucleotides resulting in polynucleotides that encode the same or functionally equivalent. The polypeptides and antibodies may also be "altered" and contain deletions, insertions, or substitutions of amino acid residues which produce a silent change and result in functionally equivalent sequences. Deliberate amino acid substitutions may be made on the basis of similarity in polarity, charge, solubility, hydrophobicity, hydrophilicity, and/or the amphipathic nature of the residues as long as at least one biological activity (e.g., effect on cell proliferation, cell survival, cell motility, and/or oncogenicity) or immunogenic/immunological activity of the sequence is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine. Guidance in making substitutions and/or deletions or additions can be obtained, for example, by sequence comparisons to homologues, orthologues, or paralogues, as shown in the figures, herein.

The term "antigen binding site" or "antigen binding portion" refers to the part of the immunoglobulin molecule, or fragment, or modification thereof, that participates in antigen interaction. The antigen binding site is generally formed by amino acid residues of the N-terminal variable ("V") regions of the heavy ("H") and light ("L") chains. Three highly divergent stretches within the V regions of the heavy and light chains, referred to as "hypervariable regions," are interposed between more conserved flanking stretches known as "framework regions". Thus, the term "framework region" or "FR" refers to amino acid sequences which are naturally found between, and adjacent to, hypervariable regions in immunoglobulins. In an antibody molecule, the three hypervariable regions of a light chain and the three hypervariable regions of a heavy chain are disposed relative to each other in three dimensional space to form an antigen binding surface. The antigen binding surface is generally complementary to the three-dimensional surface of a bound antigen, and the three hypervariable regions of each of the heavy and light chains are referred to as "complementarity-determining regions," or "CDRs." The assignment of amino acids to each domain is in accordance with accepted definitions (see, e.g., Kabat Sequences of Proteins of Immunological Interest, National Institutes of Health, Bethesda, MD, 1987 and 1991; and Chothia & Lesk J. Mol. Biol. 196:901-917, 1987, Chothia et al. Nature 342:878-883, 1989).

"Amino acid sequence", as used herein, refers to a sequence of an oligopeptide, peptide, polypeptide, protein, or antibody, and any fragment thereof, and to any naturally occurring, recombinant, synthetic, or semi-synthetic molecules. The sequences of the invention comprise at least 5, 6, 7, 8, 9, 10, 11, or 12 amino acids, preferably at least 5 to 10, 5 to 15, 10 to 15, or 12 to 15 amino acids. Preferably, the sequences retain the biological activity (e.g., effect on cell proliferation, cell survival, cell motility, and/or oncogenicity) or the immunogenicity/immuno logical activity of the original amino acid sequence. It will be understood that "amino acid sequence" and like terms are not limited to the complete, original sequence associated with the full-length molecule, but include also any modifications thereof.

When used in reference to artemin, the terms "antagonist", "antagonism" and the like are intended to refer to countering, reducing, or ablating one or more biological activities of artemin. While it may be desirable to completely inhibit activity, this need not be essential. "Antagonism" may occur at the level of expression and production (e.g., transcriptional or translational levels) or by targeting function. For example, antagonism as used herein contemplates a decrease, for example, in RNA levels (e.g., decreased transcription, increased turnover, and/or decreased stability), in polypeptide levels (e.g., decreased translation, increased turnover, and/or decreased stability) in post-translational modification required for activity, or a decrease in activity. It will be appreciated that antagonism may be effected indirectly or directly.

Accordingly, an "antagonist of artemin function", an "antagonist of artemin" or an "antagonist of artemin functionality" is an agent capable of countering, decreasing or ablating one or more biological activities of artemin, such as in a cell to which the agent is contacted, provided that such antagonist of artemin function is as defined in the claims. It will be recognised by those skilled in the art that to be effective in the methods of the present invention, an antagonist of artemin function need not mediate its effect directly on artemin, but may antagonise a biological activity of artemin indirectly. In one example, an antagonist of artemin function may be effective by decreasing or ablating artemin levels, for example, by decreasing transcription or translation of artemin mRNA. In another example, an antagonist of artemin function may decrease or block binding of artemin to a ligand or binding partner, such as a receptor, such as GFRα3. In another example an antagonist of artemin function may decrease or block binding or interaction of artemin-bound receptor with a signal transduction molecule to which the artemin-bound receptor would otherwise bind, such as artemin-bound GFRα3 to RET. In certain embodiments, an antagonist of artemin function may also decrease or block the activities or expression levels of downstream or upstream agents in the relevant pathway. In particular aspects, the antagonists of artemin function of the invention, as defined in the claims, are useful for resensitising one or more cancer cells to an anti-cancer treatment, such as those directed to inhibiting cell proliferation, cell survival, cell motility, and/or oncogenicity, especially for cancer cells, as described herein. In particular, the agents may be useful for one or more of: decreasing cell proliferation (e.g., by decreasing cell division), increasing cell death (e.g., by increasing apoptosis or necrosis), or decreasing cellular invasion and/or metastasis (e.g., by decreasing cytoskeletal activity, cell motors).

"Artemin" as used herein refers to the GDNF family ligand artemin and can include similar sequences and/or activities, including, but not limited to isoforms of pre-pro-artemin or isoform precursors, such as isoforms of human artemin precursors including human artemin isoform 1 precursor (Entrez Protein IDs: NP_003967 and NP_476432, SEQ ID NO: 1), human artemin isoform 2 precursor (Entrez Protein ID: NP_476501, SEQ ID NO: 2), and human artemin isoform 3 precursor (Entrez Protein IDs: NP_476431 and NP_001129687, SEQ ID NO: 3), isoforms of pro-artemin, and isoforms of mature artemin, such as isoforms of mature human artemin (SEQ ID NO: 4-6). Accordingly, artemin polynucleotides include polynucleotides capable of encoding artemin, including human artemin, and are exemplified by but not limited to human artemin transcript variant 1 (GenBank accession #: NM_003976), human artemin transcript variant 2 (GenBank accession #: NM_057091), human artemin transcript variant 3 (GenBank accession #: NM_057160), human artemin transcript variant 4 (GenBank accession #: NM_057090), human artemin transcript variant 5 (GenBank accession #: NM_001136215), and the gene encoding human artemin (GeneID: 9048). For use with the invention, human sequences are preferred, but other homo logs and orthologs can also be used. It will be understood that each reference to these factors (e.g., artemin and isoforms thereof), herein, will include the full length sequences as well as any fragments, or modifications (including variants) thereof. Artemin has also been referred to as neublastin and enovin

The terms "cancer" and "cancerous" refer to a physiological condition in mammals that is typically characterized by abnormal or unregulated cell proliferation, cell survival, cell motility, and/or oncogenicity. Cancer and cancer pathology can be associated, for example, with metastasis, interference with the normal functioning of neighbouring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc. Specifically included are breast cancers and precancerous conditions, which can include epithelial tumours, nonepithelial tumours, carcinomas, for example, carcinomas in situ, as well as invasive breast cancers.

The term "coding region" or "open reading frame" (ORF) refers to the sense strand of a genomic DNA sequence or a cDNA sequence that is capable of producing a transcription product and/or a polypeptide under the control of appropriate regulatory sequences. The coding sequence is identified by the presence of a 5' translation start codon and a 3' translation stop codon. When inserted into a genetic construct, a "coding sequence" is capable of being expressed when it is operably linked to promoter and terminator sequences.

The term "comprising" as used in this specification means "consisting at least in part of'. When interpreting each statement in this specification that includes the term "comprising", features other than that or those prefaced by the term may also be present. Related terms such as "comprise" and "comprises" are to be interpreted in the same manner.

The terms "complementary" or "complementarity" as used herein in reference to nucleic acids refers to the natural binding of polynucleotides under permissive salt and temperature conditions by base-pairing. For the sequence A-G-T, the complementary sequence is T-C-A, the reverse complement is A-C-T, and the reverse sequence is T-G-A. Complementarity between two single stranded molecules may be partial, in which only some of the nucleic acids bind, or it may be complete when total complementarity exists between the single stranded molecules. The degree of complementarity between nucleic acid strands has significant effects on the efficiency and strength of hybridization between nucleic acid strands. This is of particular importance in amplification reactions, which depend upon binding between nucleic acids strands and in the design and use of iRNAs and PNAs.

The term "derivative," as used herein, refers to the chemical modification of a polynucleotide, or a polynucleotide complementary thereto. Such modifications include, for example, replacement of hydrogen by an alkyl, acyl, or amino group. In preferred aspects, a polynucleotide derivative encodes a polypeptide which retains the biological or immunological function of the natural molecule. A derivative polypeptide or antibody is one which is modified by glycosylation, pegylation, or any similar process which retains one or more biological functions (e.g., effect on cell proliferation, cell survival, cell motility, and/or oncogenicity) or immunogenic/immunological function of the sequence from which it was derived. In reference to antibodies, the term "derivatives" includes, for example, hybrid and recombinant antibodies. "Hybrid" and "recombinant" versions of an antibody include, for example, humanised antibodies, diabodies, triabodies, and single chain antibodies.

The term "domain" refers to a unit of a protein or protein complex, comprising a polypeptide subsequence, a complete polypeptide sequence, or a plurality of polypeptide sequences where that unit has a defined function. The function is understood to be broadly defined and can be antigen binding activity, ligand binding activity, catalytic activity, or the like, or can have a stabilizing effect on the structure of the protein.

As used herein, the term "epitope" includes any polypeptide or peptide determinant capable of specific binding to an immunoglobulin, or a related molecule, e.g., an scFv, or a T cell receptor. Epitopic determinants generally include chemically active surface groupings of molecules such as amino acids and/or sugar side chains, and usually have specific three dimensional structural characteristics, as well as specific charge characteristics. Epitopes include two main types, namely, sequential epitopes (SEs), where the antibody binds to a contiguous stretch of amino acid residues that are linked by peptide bond, and conformational epitopes (CEs), where the antibody binds to non-contiguous residues, brought together by folding of polypeptide chain. Conformational epitopes are also referred to herein as native epitopes. It is known from the analyses of the crystal structures of antigen-antibody complexes that, to be recognized by an antibody, the residues must be generally accessible for interactions, and thus presented near the surface of antigen. A commercially available algorithm was used to predict SE and CE.

An antibody is said to specifically bind an antigen when the dissociation constant is ≤ 1 µM; preferably ≤ 100 nM, and most preferably ≤ 10 nM. In certain aspects, an antibody can specifically bind with or react with a ligand as described herein. For an antibody, "specifically bind" or "specifically immunoreacts with" is meant that the antibody reacts with one or more antigenic determinants of the desired antigen and does not react (i.e., bind) with other polypeptides or binds at much lower affinity (e.g., Kd > 10-6) with other polypeptides.

The term "expression" includes production of polynucleotides and polypeptides, in particular, the production of RNA (e.g., mRNA) from a gene or portion of a gene, and includes the production of a polypeptide encoded by an RNA or gene or portion of a gene, and the appearance of a detectable material associated with expression. For example, the formation of a complex, for example, from a polypeptide-polypeptide interaction, polypeptide-nucleotide interaction, or the like, is included within the scope of the term "expression". Another example is the binding of a binding ligand, such as a hybridization probe or antibody, to a gene or other polynucleotide or oligonucleotide, a polypeptide or a protein fragment, and the visualization of the binding ligand. Thus, increased intensity of a spot on a microarray, on a hybridization blot such as a Northern blot, or on an immunoblot such as a Western blot, or on a bead array, or by PCR analysis, is included within the term "expression" of the underlying biological molecule.

The term "expression construct" refers to a genetic construct that includes the necessary elements that permit transcribing the inserted polynucleotide molecule, and, optionally, translating the transcript into a polypeptide. An expression construct typically comprises in a 5' to 3' direction: a promoter, functional in the host cell into which the construct will be introduced, the polynucleotide to be expressed, and a terminator functional in the host cell into which the construct will be introduced.

Expression constructs contemplated herein may be inserted into a replicable vector for cloning or for expression, or may be incorporated into the host genome.

A "fragment" of a polynucleotide sequence provided herein is a subsequence of contiguous nucleotides that is preferably at least 15 nucleotides in length. The fragments of the invention preferably comprises at least 20 nucleotides, more preferably at least 30 nucleotides, more preferably at least 40 nucleotides, more preferably at least 50 nucleotides and most preferably at least 60 contiguous nucleotides of a polynucleotide of the invention. A fragment of a polynucleotide sequence can be used in antisense, gene silencing, triple helix or ribozyme technology, or as a primer, a probe, included in a microarray, or used in polynucleotide-based selection methods.

The term "fragment" in relation to promoter polynucleotide sequences is intended to include sequences comprising cis-elements and regions of the promoter polynucleotide sequence capable of regulating expression of a polynucleotide sequence to which the fragment is operably linked.

Preferably fragments of polynucleotide sequences of the invention comprise at least 20, more preferably at least 30, more preferably at least 40, more preferably at least 50, more preferably at least 100, more preferably at least 200, more preferably at least 300, more preferably at least 400, more preferably at least 500, more preferably at least 600, more preferably at least 700, more preferably at least 800, more preferably at least 900 and most preferably at least 1000 contiguous nucleotides of a polynucleotide of the invention.

The terms "functional variant" and "functional fragment" as used herein, for example in respect of artemin, or in respect of a polypeptide antagonist of artemin function, refer to polypeptide sequences different from the specifically identified sequence(s), wherein one or more amino acid residues is deleted, substituted, or added, or a sequence comprising a fragment of the specifically identified sequence(s). Functional variants may be naturally occuring allelic variants, or non-naturally occuring variants. Functional variants may be from the same or from other species and may encompass homologues, paralogues and orthologues. Functional variants or functional fragments of the polypeptides possess one or more of the biological activities of the native specifically identified polypeptide, such as an ability to elicit one or more biological effects elicited by the native polypeptide. For example, a functional fragment of artemin may bind to or agonise one or more receptors bound or agonised by artemin.

Functional variants or functional fragments may have greater or lesser activity than the native polypeptide. In one example, one or more of the biological activities of the specifically identified native polypeptide possessed by the functional variant or functional fragment may be present to a greater or lesser degree in the functional variant or functional fragment than is found in the native polypeptide. In another example, each of the biological activities of the specifically identified native polypeptide possessed by the functional variant or functional fragment is present to a greater or lesser degree in the functional variant or functional fragment than is found in the native polypeptide. In still a further example, it may be desirable to provide a functional variant or functional fragment in which one or more of the biological activities of the native polypeptide is maintained or is present to a greater degree than is found in the native polypeptide, but one or more other biologicial activities of the native polypeptide is not present or is present to a lesser degree than is found in the native polypeptide. Examples of such functional fragments include the anti-artemin antibody fragments described herein.

Methods and assays to determine one or more biological effects elicited by the polypeptides specified herein are well known in the art and examples are described herein, and such methods and assays can be used to identify or verify one or more functional variants or functional fragments of, for example, artemin or a polypeptide antagonist of artemin function. For example, an assay of the ability of artemin to increase one or more oncogenic traits in a cell, such as those described herein in the Examples, is amenable to identifying one or more functional variants or functional fragments of artemin. Similarly, an assay of the ability of an antagonist of artemin function, such as a polypeptide antagonist of artemin function including an anti-artemin antibody, is amenable to identifying one or more functional variants of the antagonist of artemin function.

Examples of functional fragments include polypeptide fragments that comprise amino acid sequences that are responsible for biological activity, for example, receptor binding, antigen binding, or the like.

The term "primer" refers to a short polynucleotide, usually having a free 3'OH group, that is hybridized to a template and used for priming polymerization of a polynucleotide complementary to the template. Such a primer is preferably at least 5, more preferably at least 6, more preferably at least 7, more preferably at least 8, more preferably at least 9, more preferably at least 10, more preferably at least 11, more preferably at least 12, more preferably at least 13, more preferably at least 14, more preferably at least 15, more preferably at least 16, more preferably at least 17, more preferably at least 18, more preferably at least 19, more preferably at least 20 nucleotides in length.

The term "probe" refers to a short polynucleotide that is used to detect a polynucleotide sequence that is complementary to the probe, in a hybridization-based assay. The probe may consist of a "fragment" of a polynucleotide as defined herein. Preferably such a probe is at least 5, more preferably at least 10, more preferably at least 20, more preferably at least 30, more preferably at least 40, more preferably at least 50, more preferably at least 100, more preferably at least 200, more preferably at least 300, more preferably at least 400 and most preferably at least 500 nucleotides in length.

The term "homology", as used herein, refers to association to a common ancestor and accordingly to a degree of similarity, identity, or complementarity. There may be partial homology (i.e., a certain % identity) or complete homology (i.e., 100% identity). A partially complementary sequence that at least partially inhibits an identical sequence from hybridizing to a target nucleic acid is referred to using the functional term "substantially homologous." The inhibition of hybridization of the completely complementary sequence to the target sequence may be examined using a hybridization assay (e.g., Southern or northern blot, solution hybridization, and the like) under conditions of low stringency. A substantially homologous sequence or hybridization probe will compete for and inhibit the binding of a completely homologous sequence to the target sequence under conditions of low stringency. This is not to say that conditions of low stringency are such that non-specific binding is permitted; low stringency conditions require that the binding of two sequences to one another be a specific (i.e., selective) interaction.

The term "hybridization", as used herein, refers to any process by which a strand of nucleic acid binds with a complementary strand through base pairing.

An "insertion" or "addition", as used herein, refers to a change in an amino acid or nucleotide sequence resulting in the addition of one or more amino acid residues or nucleotides, respectively, as compared to the naturally occurring molecule.

"Inhibition" or "inhibiting" is intended to refer to reducing or ablating, and contemplates both partial and complete reduction or ablation. "Inhibition" of a biological activity, for example that mediated by a gene product, may occur at the level of expression and production (e.g., transcriptional or translational levels) or by targeting function, for example. The terms "inhibit" or "inhibition" when used in reference to artemin functionality accordingly means a reduction or ablation in one or more functional of or mediated by artemin.

The terms "modified" or "modification" refer to altered sequences and to sequence fragments, variants, and derivatives, as described herein. The term includes polypeptides, polynucleotides, antibodies, and like agents described herein.

The term "monoclonal antibody," "mAb," or "monoclonal antibody composition," as used herein, refers to a population of antibody molecules that contain a molecular species of antibody molecule including a unique light chain gene product and a unique heavy chain gene product. In particular, the complementarity determining regions (CDRs) of the monoclonal antibody are unique. mAbs include an antigen binding site capable of immunoreacting with a particular epitope of the antigen characterized by a unique binding affinity for it.

"Nucleic acid sequence" or "nucleotide sequence" as used herein, refers to a sequence of a polynucleotide, oligonucleotide, or fragments thereof, and to DNA or RNA of natural, recombinant, synthetic or semi-synthetic, origin which may be single or double stranded, and can represent sense or antisense strand, or coding or non-coding regions. The sequences of the invention, preferably, comprise at least 15, 21, 27, 33, 36, 39, 45, 51, 57, or 66 nucleotides, preferably at least 15 to 36, 15 to 66, 36 to 66, or 45 to 66 nucleotides, or at least 100 nucleotides, or at least 1000 nucleotides. It will be understood that each reference to a "nucleic acid sequence" or "nucleotide sequence," herein, will include the original, full length sequence, as well as any complements or modifications thereof. It will be further understood that any reference to a "polynucleotide" (or "oligonucleotide," or "probe," or "primer," etc.) having a particular SEQ ID NO. will encompass both the DNA and the counterpart RNA sequences.

The term "oligonucleotide" refers to a polynucleotide, typically a probe or primer, including, without limitation, single stranded DNAs, single or double stranded RNAs, RNA:DNA hybrids, and double stranded DNAs. Oligonucleotides, such as single stranded DNA probe oligonucleotides, are often synthesized by chemical methods, for example using automated oligonucleotide synthesizers that are commercially available, or by a variety of other methods, including in vitro expression systems, recombinant techniques, and expression in cells and organisms.

The term "patient" or "subject" includes human and non-human animals. Non-human animals include, but are not limited to, birds and mammals, in particular, mice, rabbits, cats, dogs, pigs, sheep, goats, cows, and horses.

"Peptide nucleic acid" or "PNA" as used herein, refers to an antisense molecule or anti-gene agent which comprises bases linked via a peptide backbone.

As used herein, the phrase "pharmaceutically acceptable diluents, carriers, and/or excipients" is intended to include substances that are useful in preparing a pharmaceutical composition, and may be co-administered with an agent in accordance with the invention while allowing same to perform its intended function. These are generally safe, non-toxic, and neither biologically nor otherwise undesirable. Examples of pharmaceutically acceptable diluents, carriers, and/or excipients include solutions, solvents, dispersion media, delay agents, emulsions, and the like. Diluents, carriers, and/or excipients may contain minor amounts of additives such as substances that enhance isotonicity and chemical stability.

The term "polynucleotide" (e.g., "artemin" which can be used to discuss a polynucleotide), when used in the singular or plural, generally refers to any nucleic acid sequence, e.g., any polyribonucleotide or polydeoxribonucleotide, which may be unmodified RNA or DNA or modified RNA or DNA. This includes, without limitation, single and double stranded DNA, DNA including single and double- stranded regions, single and double stranded RNA, and RNA including single and double stranded regions, hybrid molecules comprising DNA and RNA that may be single stranded or, more typically, double stranded or include single and double stranded regions. Also included are triple-stranded regions comprising RNA or DNA or both RNA and DNA. Specifically included are mRNAs, cDNAs, and genomic DNAs, and any fragments thereof. The term includes DNAs and RNAs that contain one or more modified bases, such as tritiated bases, or unusual bases, such as inosine. The polynucleotides of the invention can encompass coding or non-coding sequences, or sense or antisense sequences, or iRNAs such as siRNAs. It will be understood that each reference to a "polynucleotide" or like term, herein, will include the full length sequences as well as any complements or modifications thereof.

"Polypeptide" as used herein, refers to an oligopeptide, peptide, or protein, or fragment thereof, and to naturally occurring, recombinant, synthetic, or semi-synthetic molecules. Where these terms are recited herein to refer to an amino acid sequence of a naturally occurring protein molecule, the terms are not meant to limit the amino acid sequence to the complete, original sequence for the full length molecule. It will be understood that each reference to "polypeptide" or like term, herein, will include the full length sequence, as well as any modifications thereof.

The terms "resensitising", "resensitisation", "resensitise" and grammatical equivalents thereof refers to the development or presence of a response to a stimulus where no response was previously present or evident, or to the development or presence of a greater response to a stimulus where a lesser response was previously present or evident. For example, when used with reference to a cell's response to an agent (e.g., response to contact with or exposure to the agent), resensitising the cell to the agent means the development of a response in the cell to the agent when previously no response or a lesser response was present. Methods to determine, for example, resensitisation of a cell to an agent are well known in the art, and examples of such methods are presented herein. Typically, resensitisation is by a statistically significant amount - that is, the response to the stimulus when resensitised is significantly greater than the response to the stimulus in the absence of resensitisation. Again, examples of statistically significant resensitisation and methods of assessing statistical significance are presented herein in the Examples.

The terms "stringent conditions" or "stringency," as used herein, refer to the conditions for hybridization as defined by the nucleic acid, salt, and temperature. These conditions are well known in the art and may be altered in order to identify or detect identical or related polynucleotide sequences. See, e.g., Sambrook, J. et al. (1989) Molecular Cloning, A Laboratory Manual, Cold Spring Harbor Press, Plainview, NY, and Ausubel, F. M. et al. (1989) Current Protocols in Molecular Biology, John Wiley & Sons, New York, NY. Numerous equivalent conditions comprising either low or high stringency depend on factors such as the length and nature of the sequence (e.g., DNA, RNA, base composition), nature of the target (e.g., DNA, RNA, base composition), milieu (e.g., in solution or immobilized on a solid substrate), concentration of salts and other components (e.g., formamide, dextran sulfate and/or polyethylene glycol), and temperature of the reactions (e.g., within a range from about 5°C below the melting temperature of the probe to about 20°C to 25°C below the melting temperature). One or more factors be may be varied to generate conditions of either low or high stringency different from, but equivalent to, the above listed conditions.

The terms "substantially purified" or "isolated" as used herein, refer to nucleic or amino acid sequences that are removed from their cellular, recombinant, or synthetic environment, and are at least 60% free, preferably 75% free, and most preferably at least 90% free or at least 99% free from other components with which they are associated in their environment. "Isolated" polynucleotides and polypeptides have been identified and separated from at least one contaminant nucleic acid molecule with which they are associated in their natural state. Accordingly, it will be understood that isolated polynucleotides and polypeptides are in a form which differs from the form or setting in which they are found in nature. It will further be appreciated that "isolated" does not necessarily reflect the exact extent (e.g., a specific percentage) to which the sequence has been purified.

"Treatment" and like terms refer to methods and compositions to prevent, cure, or ameliorate a medical disorder (e.g., medical disease, condition, or syndrome), or reduce at least a symptom of such disorder. In particular, this includes methods and compositions to prevent or delay onset of a medical disorder; to cure, correct, reduce, slow, or ameliorate the physical or developmental effects of a disorder; to preventing recurrence or relapse of a disorder or symptoms of disorder; or to prevent, end, reduce, or ameliorate the pain or suffering caused the disorder. The term "treatment" is to be considered in its broadest context. The term does not necessarily imply that the subject is treated until total recovery. Accordingly, "treatment" as used herein broadly includes inhibiting, reducing or preventing cell proliferation, cell survival, cell motility, and/or oncogenicity; ameliorating the symptoms or severity of cell proliferation, cell survival, cell motility, and/or oncogenicity; or preventing or otherwise reducing the risk of developing cell proliferation, cell survival, cell motility, and/or oncogenicity, for example cancer, and in particular, breast cancer, colon cancer, prostate cancer, endometrial cancer, lung cancer, stomach cancer, liver cancer, or another cancer.

The term "variant" as used herein refers to polynucleotide or polypeptide sequences different from the specifically identified sequences, wherein one or more nucleotides or amino acid residues is deleted, substituted, or added. Variants may be naturally occuring allelic variants, or non-naturally occurring variants. Variants may be from the same or from other species and may encompass homologues, paralogues and orthologues. In certain embodiments, variants of the polynucleotides and polypeptides possess biological activities that are the same or similar to those of the wild type polynucleotides or polypeptides. The term "variant" with reference to polynucleotides and polypeptides encompasses all forms of polynucleotides and polypeptides as defined herein.

### Polynucleotide and polypeptide variants

The term "polynucleotide(s)," as used herein, means a single or double-stranded deoxyribonucleotide or ribonucleotide polymer of any length but preferably at least 15 nucleotides, and include as non-limiting examples, coding and non-coding sequences of a gene, sense and antisense sequences complements, exons, introns, genomic DNA, cDNA, pre-mRNA, mRNA, rRNA, siRNA, miRNA, tRNA, ribozymes, recombinant polypeptides, isolated and purified naturally occurring DNA or RNA sequences, synthetic RNA and DNA sequences, nucleic acid probes, primers and fragments. A number of nucleic acid analogues are well known in the art and are also contemplated.

### Polynucleotide variants

Variant polynucleotide sequences preferably exhibit at least 50%, more preferably at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to a specified polynucleotide sequence. Identity is found over a comparison window of at least 20 nucleotide positions, preferably at least 50 nucleotide positions, at least 100 nucleotide positions, or over the entire length of the specified polynucleotide sequence.

Polynucleotide sequence identity can be determined in the following manner. The subject polynucleotide sequence is compared to a candidate polynucleotide sequence using BLASTN (from the BLAST suite of programs, version 2.2.10 [Oct 2004]) in b12seq (Tatiana A. Tatusova, Thomas L. Madden (1999), "Blast 2 sequences - a new tool for comparing protein and nucleotide sequences", FEMS Microbiol Lett. 174:247-250), which is publicly available from NCBI (ftp://ftp.ncbi.nih.gov/blast/). The default parameters of bl2seq are utilized except that filtering of low complexity parts should be turned off.

The identity of polynucleotide sequences may be examined using the following unix command line parameters:
bl2seq -i nucleotideseq1 -j nucleotideseq2 -F F -p blastn

The parameter -F F turns off filtering of low complexity sections. The parameter -p selects the appropriate algorithm for the pair of sequences. The bl2seq program reports sequence identity as both the number and percentage of identical nucleotides in a line "Identities = ".

Polynucleotide sequence identity may also be calculated over the entire length of the overlap between a candidate and subject polynucleotide sequences using global sequence alignment programs (e.g. Needleman, S. B. And Wunsch, C. D. (1970) J. Mol. Biol. 48, 443-453). A full implementation of the Needleman-Wunsch global alignment algorithm is found in the needle program in the EMBOSS package (Rice, P. Longden, I. And Bleasby, A. EMBOSS: The European Molecular Biology Open Software Suite, Trends in Genetics June 2000, vol 16, No 6. pp.276-277) which can be obtained from http://www.hgmp.mrc.ac.uk/Software/EMBOSS/. The European Bioinformatics Institute server also provides the facility to perform EMBOSS-needle global alignments between two sequences on line at http:/www.ebi.ac.uk/emboss/align/.

Alternatively the GAP program may be used which computes an optimal global alignment of two sequences without penalizing terminal gaps. GAP is described in the following paper: Huang, X. (1994) On Global Sequence Alignment. Computer Applications in the Biosciences 10, 227-235.

Polynucleotide variants of the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.10 [Oct 2004]) from NCBI (ftp://ftp.ncbi.nih.gov/blast/).

The similarity of polynucleotide sequences may be examined using the following unix command line parameters:
bl2seq -i nucleotideseq1 -j nucleotideseq2 -F F -p tblastx

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. The size of this database is set by default in the bl2seq program. For small E values, much less than one, the E value is approximately the probability of such a random match.

Variant polynucleotide sequences preferably exhibit an E value of less than 1 x 10⁻¹⁰, more preferably less than 1 x 10⁻²⁰, less than 1 x 10⁻³⁰, less than 1 x 10⁻⁴⁰, less than 1 x 10⁻⁵⁰, less than 1 x 10⁻⁶⁰, less than 1 x 10⁻⁷⁰, less than 1 x 10⁻⁸⁰, less than 1 x 10⁻⁹⁰, less than 1 x 10⁻¹⁰⁰, less than 1 x 10⁻¹¹⁰, less than 1 x 10⁻¹²⁰ or less than 1 x 10⁻¹²³ when compared with any one of the specifically identified sequences.

Alternatively, variant polynucleotides of the present invention hybridize to a specified polynucleotide sequence, or complements thereof under stringent conditions.

The term "hybridize under stringent conditions", and grammatical equivalents thereof, refers to the ability of a polynucleotide molecule to hybridize to a target polynucleotide molecule (such as a target polynucleotide molecule immobilized on a DNA or RNA blot, such as a Southern blot or Northern blot) under defined conditions of temperature and salt concentration. The ability to hybridize under stringent hybridization conditions can be determined by initially hybridizing under less stringent conditions then increasing the stringency to the desired stringency.

With respect to polynucleotide molecules greater than about 100 bases in length, typical stringent hybridization conditions are no more than 25 to 30°C (for example, 10°C) below the melting temperature (Tm) of the native duplex (see generally, Sambrook et al., Eds, 1987, Molecular Cloning, A Laboratory Manual, 2nd Ed. Cold Spring Harbor Press; Ausubel et al., 1987, Current Protocols in Molecular Biology, Greene Publishing,). Tm for polynucleotide molecules greater than about 100 bases can be calculated by the formula Tm = 81.5 + 0.41% (G + C)-log (Na+). (Sambrook et al., Eds, 1987, Molecular Cloning, A Laboratory Manual, 2nd Ed. Cold Spring Harbor Press; Bolton and McCarthy, 1962, PNAS 84:1390). Typical stringent conditions for polynucleotide of greater than 100 bases in length would be hybridization conditions such as prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 0.2X SSC, 0.1% SDS at 65°C.

With respect to polynucleotide molecules having a length less than 100 bases, exemplary stringent hybridization conditions are 5 to 10°C below Tm. On average, the Tm of a polynucleotide molecule of length less than 100 bp is reduced by approximately (500/oligonucleotide length)°C.

With respect to the DNA mimics known as peptide nucleic acids (PNAs) (Nielsen et al., Science. 1991 Dec 6;254(5037):1497-500) Tm values are higher than those for DNA-DNA or DNA-RNA hybrids, and can be calculated using the formula described in Giesen et al., Nucleic Acids Res. 1998 Nov 1;26(21):5004-6. Exemplary stringent hybridization conditions for a DNA-PNA hybrid having a length less than 100 bases are 5 to 10°C below the Tm.

Variant polynucleotides of the present invention also encompasses polynucleotides that differ from the sequences of the invention but that, as a consequence of the degeneracy of the genetic code, encode a polypeptide having similar activity to a polypeptide encoded by a polynucleotide of the present invention. A sequence alteration that does not change the amino acid sequence of the polypeptide is a "silent variation". Except for ATG (methionine) and TGG (tryptophan), other codons for the same amino acid may be changed by art recognized techniques, e.g., to optimize codon expression in a particular host organism.

Polynucleotide sequence alterations resulting in conservative substitutions of one or several amino acids in the encoded polypeptide sequence without significantly altering its biological activity are also included in the invention. A skilled artisan will be aware of methods for making phenotypically silent amino acid substitutions (see, e.g., Bowie et al., 1990, Science 247, 1306). In some embodiments, polynucleotide sequence alterations resulting in non-conservative amino acid substitutions desirably result in a functional variant as contemplated herein, and such sequence alterations are also included in the invention.

Variant polynucleotides due to silent variations and conservative substitutions in the encoded polypeptide sequence may be determined using the publicly available bl2seq program from the BLAST suite of programs (version 2.2.10 [Oct 2004]) from NCBI (ftp://ftp.ncbi.nih.gov/blast/) via the tblastx algorithm as previously described.

### Polypeptide Variants

The term "variant" with reference to polypeptides encompasses naturally occurring, recombinantly and synthetically produced polypeptides. Variant polypeptide sequences preferably exhibit at least 50%, more preferably at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to a sequence of the present invention. Identity is found over a comparison window of at least 20 amino acid positions, preferably at least 50 amino acid positions, at least 100 amino acid positions, or over the entire length of a polypeptide of the invention.

Polypeptide sequence identity can be determined in the following manner. The subject polypeptide sequence is compared to a candidate polypeptide sequence using BLASTP (from the BLAST suite of programs, version 2.2.10 [Oct 2004]) in bl2seq, which is publicly available from NCBI (ftp://ftp.ncbi.nih.gov/blast/). The default parameters of bl2seq are utilized except that filtering of low complexity regions should be turned off.

Polypeptide sequence identity may also be calculated over the entire length of the overlap between a candidate and subject polynucleotide sequences using global sequence alignment programs. EMBOSS-needle (available at http:/www.ebi.ac.uk/emboss/align/) and GAP (Huang, X. (1994) On Global Sequence Alignment. Computer Applications in the Biosciences 10, 227-235) as discussed above are also suitable global sequence alignment programs for calculating polypeptide sequence identity.

Polypeptide variants of the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.10 [Oct 2004]) from NCBI (ftp://ftp.ncbi.nih.gov/blast/). The similarity of polypeptide sequences may be examined using the following unix command line parameters:
bl2seq -i peptideseq1 -j peptideseq2 -F F -p blastp

Variant polypeptide sequences preferably exhibit an E value of less than 1 x 10⁻¹⁰, more preferably less than 1 x 10⁻²⁰, less than 1 x 10⁻³⁰, less than 1 x 10⁻⁴⁰, less than 1 x 10⁻⁵⁰, less than 1 x 10⁻⁶⁰, less than 1 x 10⁻⁷⁰, less than 1 x 10⁻⁸⁰, less than 1 x 10⁻⁹⁰, less than 1 x 10⁻¹⁰⁰, less than 1 x 10⁻¹¹⁰, less than 1 x 10⁻¹²⁰ or less than 1 x 10⁻¹²³ when compared with any one of the specifically identified sequences.

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. For small E values, much less than one, this is approximately the probability of such a random match.

Conservative substitutions of one or several amino acids of a described polypeptide sequence without significantly altering its biological activity are also included in the invention. A skilled artisan will be aware of methods for making phenotypically silent amino acid substitutions (see, e.g., Bowie et al., 1990, Science 247, 1306). Likewise, functional variants resulting from substitution of one or more amino acids, including non-conservative substitutions, are included in the invention.

A polypeptide variant of the present invention also encompasses that which is produced from the nucleic acid encoding a polypeptide, but differs from the wild type polypeptide in that it is processed differently such that it has an altered amino acid sequence. For example a variant may be produced by an alternative splicing pattern of the primary RNA transcript to that which produces a wild type polypeptide.

The invention also encompasses variants which retain at least one biological activity (e.g., effect on cell proliferation, cell survival, cell motility, and/or oncogenicity) or immunogenic/immunological function. A preferred variant is one having at least 80%, and more preferably at least 90%, sequence identity to a disclosed sequence. A most preferred variant is one having at least 95%, at least 97%, at least 98%, or at least 99% sequence identity to a sequence disclosed herein. The percentage identity is determined by aligning the two sequences to be compared as described below, determining the number of identical residues in the aligned portion, dividing that number by the total number of residues in the inventive (queried) sequence, and multiplying the result by 100. A useful alignment program is AlignX (Vector NTI).

The term "vector" refers to a polynucleotide molecule, usually double stranded DNA, which is used to transport the genetic construct into a host cell. The vector may be capable of replication in at least one additional host system, such as *E. coli.*

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, Molecular Cloning: A Laboratory Manual, 2nd edition, Sambrook et al., 1989; also, Molecular Cloning: A Laboratory Manual, 3rd Edition, Sambrook et al., 2000; Oligonucleotide Synthesis, MY Gait, ed., 1984; Animal Cell Culture, R.I. Freshney, ed., 1987; Methods in Enzymology, Academic Press, Inc.; Handbook of Experimental Immunology, 4th edition, D .M. Weir & CC. Blackwell, eds., Blackwell Science Inc., 1987; Gene Transfer Vectors for Mammalian Cells, JAM. Miller & MAP. Calos, eds., 1987; Current Protocols in Molecular Biology, FEM. Ausubel et al., eds., 1987; and PCR: The Polymerase Chain Reaction, Mullis et al., eds., 1994.

### Breast cancer

Breast cancer (also referred to as mammary cancer) is the most common cancer in women. Breast cancer usually originates in the ducts (e.g., ductal carcinoma *in situ*) and lobules (e.g. lobular carcinoma *in situ*) of the breast. Progression of the disease and invasion can occur rapidly, with the cancer commonly spreading to axillary (underarm) lymph nodes. This can be followed by metastasis - the widespread distribution to other regions of the body, frequently to bone, lungs and liver.

The most common type of breast cancer is invasive (or infiltrating) ductal carcinoma (IDC). IDC typically originates in a duct then penetrates the wall of the duct to continue growing in the fatty tissue of the breast. Metastasis through the lymphatic system and bloodstream can follow.

Another common invasive breast cancer is Invasive lobular carcinoma (ILC), which starts in the milk-producing lobules and, like IDC, can metastasize to other parts of the body.

A number of less common types of breast cancer exist, including inflammatory breast cancer, triple-negative breast cancer, mixed tumor breast cancer, medullary carcinoma, metaplastic carcinoma, mucinous carcinoma, Paget disease of the nipple, tubular carcinoma, papillary carcinoma, adenoid cystic carcinoma (adenocystic carcinoma), phyllodes tumor, and angiosarcoma of the breast.

Primary risk factors associated with breast cancer include family history, age, sex, estrogen levels, diet, smoking, and obesity.

The present invention provides methods and compositions suitable for use in the treatment and diagnosis of breast cancer that is resistant to one or more therapies. Treatment of breast cancer can involve surgery, radiation therapy, hormonal therapy or chemotherapy.

Chemotherapy agents for treatment of breast cancer can be classified into three groups: Anthracyclines (which deform DNA structure), Taxanes (which prevent cancer cell division) and Alkylating agents (which cause alkylation of DNA). Examples of anthracyclines include Epirubicin (Ellence®) and Doxorubicin (Adriamycin®). Taxanes include Paclitaxel (Taxol®) and Docetaxel (Taxotere®), while alkylating agents include Cyclophosphamide (Cytoxin®). Other commonly used chemotherapeutic agents for treating breast cancer include Methotrexate (Amethopterin®, Mexate®, Folex®), Fluorouracil (Fluorouracil®, 5-Fu, Adrucil®), Vinorelbine (Navelbine®), Gemcitabine (Gemzar®), and Capecitabine (Xeloda®).

Hormone therapy of breast cancer can be classified into selective estrogen-receptor modulators (SERMs), aromatase inhibitors, biologic response modifiers, and other hormonal therapies. These are discussed briefly below.

SERMS act as estrogen receptor antagonists in cancer cells. Examples include Tamoxifen, Raloxifene (Evista®), and Arzoxifene. Aromatase Inhibitors act by binding to the enzyme aromatase, thus inhibiting production of estrogen by the enzyme. This causes cancer cells to starve, preventing cell growth and division. Examples include Exemestane (Aromasin®), Letrozole (Femera®), Anastrozole (Arimidex®) and Megestrol (Megace®). Biologic Response Modifiers act by binding to certain proteins expressed in cancer cells, preventing further growth of the tumour. One example is Trastuzumab (Herceptin®), a monoclonal antibody which binds to HER2 in cancer cells. Other hormone therapies include Goserelin Acetate (Zoladex®), a synthetic lutenizing hormone-releasing hormone, which blocks release of estrogen in breast cancer cells, and Fulvestrant (Faslodex®), which destroys estrogen receptors in cancer cells.

Radiation therapy can be an effective standard of care for treatment of all stages of breast cancer, however, it is typically recommended following surgery (lumpectomy or mastectomy). Surgery for treatment of breast cancer is rarely a viable option due to the non-specific nature of the disease, and the fact that rarely is the cancer localised to one spot in an organ.

A number of different mechanisms by which breast cancer cells become resistant to therapy have been proposed. Fundamentally, resistance to therapy is caused in part by genetic amplification, where each treatment with a single agent selects for those cancer cells that are increasingly resistant to therapy. This effectively decreases the responsiveness of such cells to other therapies.

More specifically, multiple mechanisms of resistance have been identified, including decreased drug accumulation, decreased drug influx, increased drug efflux, altered intracellular drug trafficking, increased inactivation of drug or toxic intermediates, increased repair of therapy-induced damage or increased tolerance to therapy-induced damage, decreased drug activation, alterations in one or more drug targets, co-factor or metabolite levels, downstream effectors of cytotoxicity, signaling pathways or apoptotic responses to drug, altered gene expression (including by mutation, amplification, or deletion, altered transcription, posttranscription processing, or translation), formation of pharmacological or anatomic drug barriers (so-called "tumor sanctuaries"), and host-drug interactions, including increased drug inactivation by normal tissues, decreased drug activation by normal tissues, and relatively increased normal tissue drug sensitivity (toxicities).

The specific mechanisms by which breast cancer cells develop resistance to therapeutic agents remain unclear. However, recent studies suggest the role of various apoptotic or cell survival pathways.

The extrinsic apoptotic pathway is activated by members of the tumour necrosis factor family and respective ligands. The intrinsic mitochondrial pathway is typically activated in response to intracellular stress signals as a result of for example, DNA damage, or extracellular stimuli, such as radiation, chemotherapeutic drugs and viral infection. The Bcl-2 family of proteins, which regulate the release of cytochrome c, play an important role in the regulation of intrinsic apoptosis and overexpression of Bcl-2 has been implicated in cancer chemoresistance.

The involvement of p53 in cellular defence to various stresses such as oncogenes, chemotherapeutic agents, hypoxia, and aberrant growth factor signalling is widely reported. In particular, the implication of p53 in cell cycle control and progression suggests its expression could be an important determinant of sensitivity towards specific chemotherapy drugs.

Cell survival pathways are many and varied and involve a diverse range of physiologic stimuli. It has recently been reported that the phosphatidylinositol 3-kinase/AKT (PI3K/AKT) pathway plays a role in resistance to anti-tumour drugs. Specifically, it was reported that expression of the tumour supressor gene, PTEN, significantly increases doxorubicin chemosensitivity through induction of apoptosis while knock-down of PI3K p110-P stimulates tumour growth (through regulation of cyclin E and Bcl-2).

Another mechanism involving cyclooxygenases (Cox) and prostaglandins has been postulated, and involves activating transcription of anti-apoptotic genes such as IAPs (inhibitor of apoptosis proteins).

For example, tamoxifen resistance in breast cancer patients has been attributed to a number of different mechanisms. Examples of such mechanisms include mutations in estrogen receptors (ER) affecting the ability of the drug to bind to ERs and elicit a response, reduction in the intracellular availability of tamoxifen through decreased influx or increased efflux, increased metabolism of tamoxifen to agonistic metabolites and overexpression of HER2 in cancer cells.

The present invention recognises that in a variety of breast cancers, functional antagonists of artemin are effective to resensitise cancer cells to therapy.

### Functional antagonists of Artemin

Artemin has been shown by the applicants to be expressed in a number of breast cancer cell lines, including MCF-7, BT549, and MDA-231. The present invention recognises that antagonists of artemin function, as defined in the claims, are useful in the treatment of breast cancer through the resensitisation of breast cancer to one or more therapies, such as one or more therapies to halt progression, metastasis, or recurrence of breast cancer.

The data disclosed herein demonstrates that antagonists of artemin function, specifically anti-artemin antibodies and anti-artemin siRNA, resensitised therapy-resistant breast cancer cells to effectively inhibit cell invasion and anchorage-independent growth, two important biological activities in cancer growth and metastasis, in ER+ and ER- mammary carcinoma cells. As such, antagonists of artemin function represent ideal new reagents for breast cancer treatment and diagnosis. Antagonists of artemin function, such as for example anti-artemin antibodies, that counter, decrease or inhibit a biological activity of artemin can be used to resensitise breast cancer cells to anti-cancer therapies, including those directed to inhibiting cancer cell proliferation, inhibiting cancer cell survival, inhibiting cancer cell motility, and/or inhibiting oncogenicity. These functional antagonists can be administered in conjunction with other agents, for example, chemical compounds (e.g., small molecules and chemotherapeutic agents), receptor agonists or antagonists, other antibodies, iRNAs, and radiotherapies.

### Anti-artemin antibodies

The invention encompasses methods and compositions that utilise antibodies for artemin, for example, antibodies that bind to at least a portion of or a modified sequence of artemin (also referred to herein as "anti-artemin antibodies"). In certain embodiments, anti-artemin antibodies may be used to antagonise one or more functions mediated by artemin. For example, an anti-artemin antibody may interfere with or decrease the binding of artemin to a receptor. In another example, an anti-artemin antibody may interfere with or decrease the binding or interaction of artemin-bound receptor with a signal transduction molecule to which artemin-bound receptor would otherwise bind or interact with in the absence of the anti-artemin antibody. In certain aspects of the invention, anti-artemin antibodies may be used to inhibit artemin. It will be understood that, for the purposes of the invention, a fragment or modification of an antibody need not act fully as an antibody. That is to say, the fragment or other modification need not be capable of recruiting immune system cells to the site of binding to artemin in vivo. It is not necessary to produce neutralising antibodies. Those of ordinary skill in the art to which the invention relates will recognise methods to generate antibody fragments. Antibody fragments of the invention can encompass a portion of one of the intact antibodies, generally the antigen binding or variable region of the antibody. However, by way of general example, fragments may be generated by proteolytic digestion of intact antibodies, or the fragments may be directly produced via recombinant nucleic acid technology.

It is understood that the basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ea., 2nd ed. Raven Press, N.Y. (1989)). The variable regions of each light/heavy chain pair form the antibody binding site.

Antibodies suitable for use in the invention can be characterised on the basis of isotype and epitope mapping, biochemical characterisations, and, by functional characterisations such as their ability to block binding of artemin to one or more receptors, or block binding of artemin-bound receptor to a signal transduction molecule. Reagents that block ligand binding or activation have application in methods for treatment of various disorders as disclosed herein. Antibodies that recognize artemin and/or a related ligand, but fail to block receptor binding are also contemplated. In various embodiments such antibodies may be used in detecting and purifying such ligands, as well as in methods for diagnosing and monitoring progression of the disorders in a subject as detailed herein after. Such antibodies might also find use in analysing the post-translational modifications of the ligand, or other modified sequences thereof.

Humanisation of antibodies may be used to reduce the immunogenicity of antibodies generated in other animals. Production of humanised antibodies or humanization of antibodies can be achieved using techniques known in the art, for example in the case of humanisation of murine antibodies by epitope-guided selection. The most frequently used strategies for the humanization of rodent monoclonal antibodies are CDR grafting (Reichmann, L., M. Clark, H. Waldman, and G. Winter. 1998. Reshaping human antibodies for therapy. Nature 332L323-327; Jones PT, Dear PH, Foote J, Neuberger MS, Winter G. 1986. Replacing the complementarity determining regions in a human antibody with those from a mouse. Nature 321:522-25) and resurfacing (Pedersen, J. T., A. H. Henry, S. J. Searle, B. C. Guild, M. Roguska, and A. R. Rees. 1994. Comparison of surface accessible residues in human and murine immunoglobulin Fv domains. Implication for humanization of murine antibodies. J. Mol. Biol. 235:959-973). Humanization of antibodies can also be achieved epitope-guided selection (Wang et al, J. Immunological Methods 241: 171-184, 2000). The methods of Maynard, J., and Georgiou, G. 2000. Antibody engineering. Annu Rev Biomed Eng 2: 339-376, provide further examples.

Humanised antibodies can be produced based on a rational design approach and iterative optimization, i.e., site-directed mutagenesis of framework residues aided by computer modelling. Other selective humanization strategies using phage display may be used. See, e.g., Baca, M., L. G. Presta, S. J. O'Connor, and J. A. Wells. 1997. Antibody humanization using monovalent phage display. J. Biol. Chem. 272:10678-10684; Hoogenboom, H. R., A. P. de Bruine, S. E. Hufton, R. M. Hoet, J. W. Arends, and R. C. Roovers. 1998. Antibody phage display technology and its applications. Immunotechnology. 4:1-20; Jespers, L. S., A. Roberts, S. M. Mahler, G. Winter, and H. R. Hoogenboom. 1994. Guiding the selection of human antibodies from phage display repertoires to a single epitope of an antigen. Biotechnology (NY) 12:899-903; Rader, C. And C. F. Barbas, III. 1997. Phage display of combinatorial antibody libraries. Curr. Opin. Biotechnol. 8:503-508; Rader, C., D. A. Cheresh, and C. F. Barbas, III. 1998. A phage display approach for rapid antibody humanization: designed combinatorial V gene libraries. Proc. Natl. Acad. Sci. U. S. A 95:8910-8915; Rosok, M. J., D. E. Yelton, L. J. Harris, J. Bajorath, K. E. Hellstrom, I. Hellstrom, G. A. Cruz, K. Kristensson, H. Lin, W. D. Huse, and S. M. Glaser. 1996. A combinatorial library strategy for the rapid humanization of anticarcinoma BR96 Fab. J. Biol. Chem. 271:22611-22618.

In other aspects, human antibodies can be produced using a transgenic animal strain reconstituted with human immunoglobulin loci, e.g., XenoMouse strains. Such strains make it possible to generate fully human antibodies in an animal host (Mendez, M. J., L. L. et al. 1997. Functional transplant of megabase human immunoglobulin loci recapitulates human antibody response in mice. Nat. Gen. 15:146-156). XenoMouse animals, in particular, comprise yeast artificial chromosomes (YACs) containing 66 human heavy-chain and 32 kappa light-chain immunoglobulin genes in their genome, where endogenous heavy-chain and kappa loci are functionally inactivated by targeted deletion (Mendez et al., supra). The mice express human mu, delta, gamma 2, and kappa chains and mouse lambda chains, with a human kappa-to-mouse lambda ratio of 75:1 (Mendez et al., supra). XenoMouse animals have been previously shown to produce human antibodies to protein antigens (Green, L. L., et al. 1994. Antigen-specific human monoclonal antibodies from mice engineered with human Ig heavy and light chain YACs. Nat. Gen. 7:13-21; Mendez et al., supra), and may also respond to T-independent antigens such as polysaccharides. As one example, two or more genetically distinct groups of XenoMouse animals can used to produce antibodies, for example, Xm2a-3 strains, reconstituted with one double YAC containing both heavy- and light-chain genes; and Xm2a-5 strains, reconstituted with two YACs, one with heavy-chain and the other with light-chain genes (Jakobovits, A. 1995. Production of fully human antibodies by transgenic mice. Curr. Opin. Biotechnol. 6:561-566; see, also, Russell ND et al., Production of protective human antipneumococcal antibodies by transgenic mice with human immunoglobulin loci. Infect Immun. 2000 Apr;68(4):1820-6).

Those of skill in the art to which the invention relates will appreciate the terms "diabodies" and "triabodies". These are molecules which comprise a heavy chain variable domain (VH) connected to a light chain variable domain (VL) by a short peptide linker that is too short to allow pairing between the two domains on the same chain. This promotes pairing with the complementary domains of one or more other chains and encourages the formation of dimeric or trimeric molecules with two or more functional antigen binding sites. The resulting antibody molecules may be monospecific or multispecific (e.g., bispecific in the case of diabodies). Such antibody molecules may be created from two or more antibodies using methodology standard in the art to which the invention relates (see, e.g., Todorovska et al. Design and application of diabodies, triabodies, and tetrabodies for cancer targeting. J. Immunol. Methods. 2001 Feb 1;248(1-2):47-66; Holliger P, Prospero T, and Winter G, Diabodies: small bivalent and bispecific antibody fragments, Proc Natl Acad Sci USA, 90, 6444-6448, 1993; and Tomlinson I and Holliger P, Methods for generating multivalent and bispecific antibody fragments, Methods Enzymol, 326, 461-479, 2000).

The production of antibodies may be carried out according to standard methodology in the art. For example, in the case of the production of polyclonal antibodies the methodology described by Bean (Eric S. Bean (2001) Polyclonal Antibodies. In: Basic Methods in Antibody Production and Characterization antibodies. Howard, G, and Bethel D. (ed.), CRC Press, 5:21-50, 2000) may be used. Monoclonal antibodies and corresponding hybridomas may be prepared, for example, in accordance with the methodology of Stewart (Sandy J. Stewart (2001) Monoclonal Antibody Production. In: Basic Methods in Antibody Production and Characterization antibodies. Howard, G. And Bethel D. (ed.), CRC Press, 6:51-68, 2000) or in Monocolonal Antibody Production Techniques and Applications, Lawrence B Schook eds., Marcel Dekker Inc., New York, 1987. Hybridomas may be subcloned, grown, and maintained using standard techniques in the art. For example, they may be grown and maintained in vitro in media such as DMEM or RPMI-1640. Alternatively, this may be done in vivo as ascites tumours in an animal of choice.

Antibodies of use in the invention may also be produced via standard recombinant techniques, see, e.g., Siegel (2002) Siegel DL, Recombinant monoclonal antibody technology, Transfus Clin Biol, 9(1):15-22 2002; Welschof, M., C. Christ, I. Hermes, A. Keller, C. Kleist, and M. Braunagel. 2003. Generation and screening of a modular human scFv expression library from multiple donors. Methods Mol. Biol. 207:103-121). The inventors consider recombinant techniques to be a preferable means of producing antibodies on a commercial scale. Polynucleotides encoding an antibody may be readily identified on the basis of the amino acid sequence of the antibody, the genetic code, and the understood degeneracy therein. Polynucleotides encoding antibodies may be isolated from hybridoma cells, for example, and subsequently characterised using procedures standard in the art. For example, a polynucleotide probe may be designed based on the amino acid sequence of a portion of an antibody and then used to isolate genes encoding the heavy and/or light chains of the antibody. Alternatively, polynucleotides may be generated by standard chemical synthesis methodology, for example, using phosphoramidite and solid phase chemistry. The amino acid sequence of an antibody of the invention may be determined using standard methodology; for example, Edman degradation and HPLC or mass spectroscopy analysis, may be used.

It should be appreciated that anti-artemin antibodies suitable for use in the invention include antibody fragments, as well as modifications of the antibodies specifically referred to herein, and as such the nucleic acid encoding the antibodies may be appropriately modified. For example, the coding sequence for heavy-and light-chain constant domains may be replaced with a homologous human domain. Alternatively, the CDRs may be transplanted to a homologous human beta-sheet framework. In this way, antibody modifications, such as humanised antibodies may be generated via recombinant techniques. As mentioned herein, antibodies suitable for use in the invention may be produced via standard recombinant procedures. Accordingly, the present invention also contemplates polynucleotides encoding an antibody, antibody fragment, or modification thereof, constructs comprising same, and host cells comprising said constructs. Polynucleotides in accordance with the invention may be DNA, RNA, or cDNA, for example, double stranded or single stranded, sense or antisense sequences, as described herein.

The antibodies suitable for use in the invention may be isolated for example, from culture supernatants, ascites fluid, or serum using standard procedures known in the art to which the invention relates. Isolation or purification may also occur via one or more of procedures such as affinity chromatography, ion exchange chromatography, interaction chromatography, gel filtration chromatography, thiophilic gel chromatography, chromatofocusing, Protein-A or G Sepharose columns, hydroxyapatite columns, detergent extraction, electrophoresis, osmotic shock treatment, inclusion body purification, ammonium sulphate precipitation, centrifugation with liquid polymers, filtration, and dialysis. A recombinant antibody in accordance with the invention may be recovered from a transformed host cell, or culture media, or transgenic organism using a variety of techniques that are standard in the art. It will be appreciated that the amino acid sequence of an antibody of the invention may be determined using standard methodology, for example, using Edman degradation and HPLC, or mass spectroscopy analysis (M.W. Hunkapiller, R.M. Hewick, W.J. Dreyer, and L.E. Hood. 1983. High-Sequencing with a Gas-Phase Sequenator. Methods Enzymol. 91: 399).

Standard methods can be used to identify amino acid sequences useful for antibody production. Antigenic segments of a polypeptide can be predicted, for example, by Abie Pro 3.0: Peptide Antibody Design (hypertext transfer protocol://world wide web.changbioscience.com/abie/abie.html). More particularly, antigenic segments can be predicted according to the Hopp-Woods scale (Hopp TP, Woods KR. Prediction of protein antigenic determinants from amino acid sequences. Proc Natl Acad Sci USA. 1981 Jun;78(6):3824-8.) and/or the Kyte and Doolittle scale (Kyte J, Doolittle RF. A simple method for displaying the hydropathic character of a protein. J Mol Biol. 1982 May 5;157(1):105-32.). Particular computer programs include MAPAG (Aguilar RC, Retegui LA, Roguin LP Int J Biomed Comput. 1994 Nov-Dec;37(3):225-35); PEOPLE (Alix AJ.: Vaccine. 1999 Sep;18(3-4):311-4); and HYDRPHIL (E A Mesri et al. J Clin Microbiol. 1990 June; 28(6): 1219-1224). Such epitopes may be conformational specific, in that they may include non-contiguous residues, and may constitute various portions of the predicted antigenic sequences, or a combination of one or more portions of the predicted antigenic sequences, or one or more of the full length sequences. Antigenic sequences can comprise any combination of amino acids or their derivatives that would form a similar 3-D structure (e.g., surface residues) as would be encountered in the native polypeptide.

For artemin, sequence analysis can be used to predict antigenic sequences useful for antibody production. The sequence information for these ligands is widely available (see, e.g., Rosenblad C, Gronborg M, Hansen C, Blom N, Meyer M, Johansen J, Dago L, Kirik D, Patel UA, Lundberg C, Trono D, Bjorklund A, Johansen TE. In vivo protection of nigral dopamine neurons by lentiviral gene transfer of the novel GDNF-family member neublastin/artemin. Mol Cell Neurosci. 2000 Feb;15(2):199-214. Erratum in: Mol Cell Neurosci 2001 Sep;1B(3):332-3). In exemplary sequence analysis, Abie Pro 3.0: Peptide Antibody Design (hypertext transfer protocols/world wide web.changbioscience.com/abie/abie.html) shows that are two highly antigenic segments according to the Hopp-Woods scale and the Kyte and Doolittle scale (Hopp TP, Woods KR. Prediction of protein antigenic determinants from amino acid sequences. Proc Natl Acad Sci U S A. 1981 Jun;78(6):3824-8; Kyte J, Doolittle RF. A simple method for displaying the hydropathic character of a protein. J Mol Biol. 1982 May5;157(1):105-32).

These antigenic sequences for artemin include PPPQPSRPAPPPPAPPSALPRGGRAAR AGGPGSRARAAGARG (residues 1-43, relative to the largest processed mature protein of 140 amino acids; SEQ ID NO: 7) and GALRPPPGSRPVSQP (residues 92-106; SEQ ID NO: 8). In addition, 27 antigenic peptides of 14 amino acids are predicted as follows: PPPQPSRPAPPPPA (residues 1-14; SEQ ID NO: 9); PPQPSRPAPPPPAP (residues 2-15; SEQ ID NO: 10); PQPSRPAPPPPAPP (residues 3-16; SEQ ID NO: 11); QPSRPAPPPPAPPS (residues 4-17; SEQ ID NO: 12); PSRPAPPPPAPPSA (residues 5-18; SEQ ID NO: 13); SRPAPPPPAPPSAL (residues 6-19; SEQ ID NO: 14); RPAPPPPAPPSALP (residues 7-20; SEQ ID NO: 15); PAPPPPAPPSALPR (residues 8-21; SEQ ID NO: 16); APPPPAPPSALPRG (residues 9-22; SEQ ID NO: 17); PPPPAPPSALPRGG (residues 10-23; SEQ ID NO: 18); PPPAPPSALPRGGR (residues 11-24; SEQ ID NO: 19); PPAPPSALPRGGRA (residues 12-25; SEQ ID NO: 20); APPSALPRGGRAAR (residues 14-27; SEQ ID NO: 21); PPSALPRGGRAARA (residues 15-28; SEQ ID NO: 22); PSALPRGGRAARAG (residues 16-29; SEQ ID NO: 23); LPRGGRAARAGGPG (residues 19-32; SEQ ID NO: 24); PRGGRAARAGGPGS (residues 20-33; SEQ ID NO: 25); RGGRAARAGGPGSR (residues 21-34; SEQ ID NO: 26); GGRAARAGGPGSRA (residues 22-35; SEQ ID NO: 27); GRAARAGGPGSRAR (residues 23-36; SEQ ID NO: 28); RAARAGGPGSRARA (residues 24-37; SEQ ID NO: 29); ARAGGPGSRARAAG (residues 26-39; SEQ ID NO: 30); AGGPGSRARAAGAR (residues 28-41; SEQ ID NO: 31); GGPGSRARAAGARG (residues 29-43; SEQ ID NO: 32); GALRPPPGSRPVSQ (residues 92-105; SEQ ID NO: 33); ALRPPPGSRPVSQP (residues 93-106; SEQ ID NO: 34).

In addition to therapeutic use, antibodies directed against artemin may find use in diagnostic applications of the invention. Persons of ordinary skill in the art will readily appreciate methods for determining the efficacy of an antibody in resensitising one or more cancer cells to a therapy, for example in preventing, decreasing, or inhibiting cell proliferation, cell survival, cell motility, and/or oncogenicity, and thus will appreciate how such methods can be utilised in diagnostic applications. However, by way of example, the methodology described elsewhere herein, including one or more of the assays referred to in the examples section, may be used.

### Nucleotide functional antagonists of artemin

Those of skill in the art will recognise that functional antagonists of artemin may readily be achieved using nucleotide agents well known in the art as inhibitors of gene expression or of gene product expression or function.

### siRNA, shRNA and antisense polynucleotides

In situations were a genotype is associated with upregulated expression of a gene, therapy utilising, for example, RNAi, siRNA, shRNA, or antisense methodologies can be implemented to decrease the abundance of mRNA and so decrease the expression of said gene.

"RNAi molecule" or an "siRNA" refers to a nucleic acid that forms a double stranded RNA, which double stranded RNA has the ability to reduce or inhibit expression of a gene or target gene when the siRNA is expressed in the same cell as the gene or target gene. "siRNA" thus refers to the double stranded RNA formed by the complementary strands. The complementary portions of the siRNA that hybridize to form the double stranded molecule typically have substantial or complete identity. In one embodiment, an siRNA refers to a nucleic acid that has substantial or complete identity to a target gene and forms a double stranded siRNA. The sequence of the siRNA can correspond to the full length target gene, or a subsequence thereof. Typically, the siRNA is at least about 15-50 nucleotides in length (e.g., each complementary sequence of the double stranded siRNA is 15-50 nucleotides in length, and the double stranded siRNA is about 15-50 base pairs in length, preferable about preferably about 20-30 base nucleotides, preferably about 20-25 nucleotides in length, e.g., 20, 21, 22, 23, 24, 25, 26, 27, 28, 29, or 30 nucleotides in length.

"shRNA" or "short hairpin RNA" refers to a nucleic acid that forms a double stranded RNA with a tight hairpin loop, which has the ability to reduce or inhibit expression of a gene or target gene, by cleavage into siRNA.

An "antisense" polynucleotide is a polynucleotide that is substantially complementary to a target polynucleotide and has the ability to specifically hybridize to the target polynucleotide. Antisense polynucleotides may be used to decrease expression of a target gene.

In addition to therapeutic use, nucleotide antagonists, such as siRNA, shRNA, or anti-antisense polynucleotides directed against artemin may find use in diagnostic applications of the invention. Persons of ordinary skill in the art will readily appreciate methods for determining the efficacy of such antagonists in resensitising one or more cancer cells to a therapy, for example in preventing, decreasing, or inhibiting cell proliferation, cell survival, cell motility, and/or oncogenicity, and thus will appreciate how such methods can be utilised in diagnostic applications. However, by way of example, the methodology described elsewhere herein, including one or more of the assays referred to in the examples section, may be used.

### Artemin polynucleotides

The invention encompasses the use of artemin polynucleotides for preparing expression vectors and host cells, and for preparing antagonists of artemin functionality including antisense polynucleotides, and iRNAs including shRNAs, and siRNAs.

Artemin polynucleotides for use in the present invention comprise one or more sequence selected from the group consisting of: (a) sequences comprising a coding sequence for at least one amino acid sequence selected from the group consisting of SEQ ID NO: 1-34, or modifications thereof; (b) complements, reverse sequences, and reverse complements of a coding sequence for at least one amino acid sequence selected from the group consisting of SEQ ID NO: 1-34, or modifications thereof; (c) open reading frames contained in the coding sequence for at least one amino acid sequence selected from the group consisting of SEQ ID NO: 1-34, or their modifications (d) functional domains of a coding sequence for at least one amino acid sequence selected from the group consisting of SEQ ID NO: 1-34, or modifications thereof; (e) sequences comprising at least a specified number of contiguous nucleotides of a coding sequence for at least one amino acid sequence selected from the group consisting of SEQ ID NO: 1-34, or modifications thereof; and (f) sequences comprising at least a specified number of contiguous nucleotides of a sequence selected from the group consisting of GenBank accession #: NM_003976, NM 057091, NM_057160, NM_057090, NM_001136215, or NM_057090, or complements, or modified sequences thereof. In one particular embodiment, the invention encompasses an isolated polynucleotide comprising a coding sequence for at least one amino acid sequence selected from the group consisting of SEQ ID NO: 1-34. Oligonucleotide probes and primers and their modifications are also provided. All of these polynucleotides and oligonucleotide probes and primers are collectively referred to herein, as polynucleotides of the invention.

It will be appreciated by those skilled in the art that as a result of the degeneracy of the genetic code, a multitude of nucleotide sequences encoding artemin polypeptides, some bearing minimal homology to the nucleotide sequences of any known and naturally occurring gene, may be produced. Thus, the invention contemplates each and every possible variation of nucleotide sequence that could be made by selecting combinations based on possible codon choices. These combinations are made in accordance with the standard triplet genetic code as applied to naturally occurring amino acid sequences, and all such variations are to be considered as being specifically disclosed.

Nucleotide sequences for artemin, or modifications thereof, are preferably capable of hybridizing to the nucleotide sequence of the naturally occurring sequence under appropriately selected conditions of stringency. However, it may be advantageous to produce nucleotide sequences, or modifications thereof, possessing a substantially different codon usage. Codons may be selected to increase the rate at which expression of the polypeptide occurs in a particular prokaryotic or eukaryotic host in accordance with the frequency with which particular codons are utilized by the host. Other reasons for substantially altering the nucleotide sequence and its derivatives without altering the encoded amino acid sequences include the production of RNA transcripts having more desirable properties, such as a greater half-life, than transcripts produced from the naturally occurring sequence.

The invention also encompasses production of polynucleotides for artemin, or modifications thereof, entirely by synthetic chemistry. After production, the synthetic sequence may be inserted into any of the many available expression vectors and cell systems using reagents that are well known in the art. Moreover, synthetic chemistry may be used to introduce mutations into a nucleotide sequence, or any derivatives thereof. Also encompassed by the invention are polynucleotide sequences that are capable of hybridizing to the claimed nucleotide sequences, and in particular, those shown in a sequence selected from the group consisting of GenBank accession #: NM_003976, N_057091, N_057160, N_057090, N_001136215, or NM_057090, or complements, or modified sequences thereof, under various conditions of stringency as taught in Wahl, G. M. And S. L. Berger (1987; Methods Enzymol. 152:399-407) and Kimmel, A. R. (1987; Methods Enzymol. 152:507-511).

Methods for DNA sequencing which are well known and generally available in the art may be used to practice any of the embodiments of the invention. The methods may employ such enzymes as the Klenow fragment of DNA polymerase I, SEQUENASE (U.S. Biochemical Corp, Cleveland, OH), Taq polymerase (Perkin Elmer), thermostable T7 polymerase (Amersham Pharmacia Biotech, Piscataway, NJ), or combinations of polymerases and proofreading exonucleases such as those found in the ELONGASE Amplification System (Life Technologies, Gaithersburg, MD). Preferably, the process is automated with machines such as the Hamilton Micro Lab 2200 (Hamilton, Reno, NV), Peltier Thermal Cycler (PTC200; MJ Research, Watertown, MA) the ABI Catalyst and 373 and 377 DNA Sequencers (Perkin Elmer), or the Genome Sequencer 20TM (Roche Diagnostics).

The nucleic acid sequences may be extended utilizing a partial nucleotide sequence and employing various methods known in the art to detect upstream sequences such as promoters and regulatory elements. For example, one method which may be employed, "restriction-site" PCR, uses universal primers to retrieve unknown sequence adjacent to a known locus (Sarkar, G. (1993) PCR Methods Applic. 2:318-322). In particular, genomic DNA is first amplified in the presence of primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Capillary electrophoresis systems which are commercially available may be used to analyze the size or confirm the nucleotide sequence of sequencing or PCR products. In particular, capillary sequencing may employ flowable polymers for electrophoretic separation, four different fluorescent dyes (one for each nucleotide), which are laser activated, and detection of the emitted wavelengths by a charge coupled device camera. Output/light intensity may be converted to electrical signal using appropriate software (e.g., GENOTYPER and Sequence NAVIGATOR, Perkin Elmer) and the entire process from loading of samples to computer analysis and electronic data display may be computer controlled. Capillary electrophoresis is especially preferable for the sequencing of small pieces of DNA which might be present in limited amounts in a particular sample.

### Compositions for use in the invention

The antagonist of artemin function (e.g., an anti-artemin antibody or antibody fragment) may be used on its own, or in the form of compositions in combination with one or more pharmaceutically acceptable diluents, carriers, and/or excipients. The antagonist of artemin function or composition comprising the antagonist of artemin function may be used in combination with an anti-cancer therapeutic agent, for example, an anti-cancer therapeutic agent against which one or more cancer cells is resistant. Those skilled in the art to which the invention relates will readily appreciate a variety of pharmaceutically acceptable diluents, carriers, and/or excipients which may be employed in compositions of the invention. As will be appreciated, the choice of such diluents, carriers, and/or excipients will be dictated to some extent by the nature of the agent to be used, the intended dosage form of the composition, and the mode of administration. By way of example, in the case of administration of polynucleotides, such as vectors adapted to express antibodies or antibody fragments, suitable carriers include isotonic solutions, water, aqueous saline solution, aqueous dextrose solution, and the like.

In addition to standard diluents, carriers, and/or excipients, a pharmaceutical composition of the invention may be formulated with additional constituents, or in such a manner, so as to enhance the activity of the agent or help protect the integrity of the agent. For example, the composition may further comprise adjuvants or constituents which provide protection against degradation, or decrease antigenicity of an agent, upon administration to a subject. Alternatively, the agent may be modified so as to allow for targeting to specific cells, tissues, or tumours.

Agents may be formulated to incorporate a sustained-release system. Inasmuch as this is the case, compositions may include semi-permeable polymer matrices in the form of shaped articles, e.g., films, or microcapsules. Sustained-release matrices include polylactides (U.S. Pat. No. 3,773,919; EP 58,481), copolymers of L-glutamic acid and gamma-ethyl-L-glutamate (Sidman et al., 1983, Biopolymers: 22: 547-56), poly(2-hydroxyethyl methacrylate) (Langer et al., 1981, J. Biomed. Mater. Res.: 15: 267), ethylene vinyl acetate (Langer et al., 1981, J. Biomed. Mater. Res.: 15: 267), or poly-D-(-)-3-hydroxybutyric acid (EP 133,988).

Agents of the invention may also be formulated into liposomes. Liposomes comprising the compound may be prepared using techniques known in the art to which the invention relates. By way of example see: DE 3,218,121, EP 52,322, EP 36,676, EP 88,046, EP 143,949, EP 142,641, Japanese Pat. Appln. 83-118008, U.S. Pat. Nos. 4,485,045 and 4,544,545, and EP 102,324. Ordinarily, the liposomes are of the small (from or about 200 to 800 Angstroms) unilamellar type in which the lipid content is greater than about 30 mol percent cholesterol, the selected proportion being adjusted for the most efficacious therapy. Agents of use in the invention may also be pegylated to increase their lifetime.

Additionally, it is contemplated that a composition in accordance with the invention may be formulated with other ingredients which may be of benefit to a subject in particular instances. The antagonist of artemin function may be co-administered with other agents, e.g., chemical compounds, such as small molecules, as well as receptor agonists or antagonists, other antibodies, antisense polynucleotides, and iRNAs. In addition, there may be benefit in incorporating, where appropriate, one or more anti-neoplastic agents. Examples of such agents include: alkylating agents, for example, chlorambucil (e.g., Leukeran™), cyclophosphamide (e.g., Endoxan™, Cycloblastin™, Neosar™, Cyclophosphamide™), ifosfamide (e.g., Holoxan™, Ifex™, Mesnex™), thiotepa (e.g., Thioplex™, Thiotepa™); and antimetabolites/S-phase inhibitors, for example, methotrexate sodium (e.g., Folex™, Abitrexate™, Edertrexate™), 5-fluorouracil (e.g., Efudix™, Efudex™), hydroxyurea (e.g., Droxia™, Hydroxyurea, Hydrea™), amsacrine, gemcitabine (e.g., Gemzar™), dacarbazine, thioguanine (e.g., Lanvis™).

Also included are antimetabolites/mitotic poisons, for example, etoposide (Etopophos™, Etoposide, Toposar™), vinblastine (e.g., Velbe™, Velban™), vindestine (e.g., Eldesine™), vinorelbine (e.g., Navelbine™), paclitaxel (e.g., Taxol™); antibiotic-type agents, for example, doxorubicin (e.g., Rubex™), bleomycin (e.g., Blenoxane™), dactinomycin (e.g., Cosmegen™), daunorubicin (e.g., Cerubidin™), mitomycin (e.g., Mutamycin™); hormonal agents, for example, aminoglutethimide (e.g., Cytadren™), anastrozole (e.g., Arimidex™), estramustine (e.g., Estracyt™, Emcyt™), goserelin (e.g., Zoladex™), hexamethylmelanine (e.g., Hexamet™), letrozole (e.g., Femara™), anastrozole (e.g., Arimidex™), and tamoxifen (e.g., Estroxyn™, Genox™, Novaldex™, Soltamox™, Tamofen™).

Further included are combinations of any two or more anti-neoplastic agents (for example, Adriamycin/5-fluorouracil/cyclophosphamide (FAC); and cyclophosphamide/methotrexate/5-fluorouracil (CMF)). Particularly useful are combinations that include, for example, at least two or more agents such as cyclophosphamide (e.g., CYTOXAN), methotrexate (e.g., RHEUMATREX), 5-fluorouracil (e.g., ADRUCIL), doxorubicin (e.g., ADRIAMYCIN), and cyclophosphamide (e.g., CYTOXAN). Useful for metastatic disease are agents such as capecitabine (e.g., XELODA), doxorubicin (e.g., ADRIAMYCIN), including its liposomal formulation, gemcitabine (e.g., GEMZAR), the taxanes, including paclitaxel (e.g., TAXOL) and docetaxel (e.g., TAXOTERE), vinorelbine (e.g., NAVELBINE), and trastuzumab (e.g., HERCEPTIN). Persons of ordinary skill in the art to which the invention relates will readily appreciate examples of other agents which may be of benefit. Agents of the invention may also be formulated with compounds and agents, other than those specifically mentioned herein, in accordance with accepted pharmaceutical practice.

As will be appreciated, in the case of administration of polynucleotides (e.g., expression vectors for nucleotide antagonists of artemin function, or expression vectors for antibodies or antibody fragments), they may be packaged into viral delivery systems, which viral systems may themselves be formulated into compositions as herein described. Persons of skill in the art to which the invention relates may appreciate a variety of suitable viral vectors having regard to the nature of the invention described herein. However, by way of example, retroviral vectors, adenoviral vectors, and adeno-associated virus (AAV) can be used. Persons of skill in the art to which the invention relates will readily appreciate methods which may be employed to implement such vectors in the present invention. However, by way of example only: the use of retroviral vectors is reported in Miller et al., 1993, Meth. Enzymol. 217:581-599, and Boesen et al., 1994, Biotherapy 6:291-302; the use of adenoviral vectors is reported for example in Kozarsky and Wilson, 1993, Current Opinion in Genetics and Development 3:499-503; Rosenfeld et al., 1991, Science 252:431-434; Rosenfeld et al., 1992, Cell 68:143-155; Mastrangeli et al., 1993, J. Clin. Invest. 91:225-234; PCT Publication WO 94/12649; and Wang, et al., 1995, Gene Therapy 2:775-783; and, the use of AAV has been reported in Walsh et al., 1993, Proc. Soc. Exp. Biol. Med. 204:289-300; U.S. Patent No. 5,436,146.

In accordance with the mode of administration to be used, and the suitable pharmaceutical excipients, diluents and/or carriers employed, compositions of the invention may be adapted into customary dosage forms such as solutions, orally administrable liquids, injectable liquids, tablets, coated tablets, capsules, pills, granules, suppositories, transdermal patches, suspensions, emulsions, sustained release formulations, gels, aerosols, liposomes, powders and immunoliposomes. The dosage form chosen will reflect the mode of administration desired to be used, the disorder to be treated and the nature of the agent to be used. Particularly preferred dosage forms include orally administrable tablets, gels, pills, capsules, semisolids, powders, sustained release formulations, suspensions, elixirs, aerosols, ointments, or solutions for topical administration, and injectable liquids. Skilled persons will readily recognise appropriate dosage forms and formulation methods. Generally, compositions are prepared by contacting or mixing specific agents and ingredients with one another. Then, if necessary, the product is shaped into the desired formulation. By way of example, certain methods of formulating compositions may be found in references such as Gennaro AR: Remington: The Science and Practice of Pharmacy, 20th ed., Lippincott, Williams & Wilkins, 2000.

The amount of an antagonist of artemin function in a composition can vary widely depending on the type of composition, size of a unit dosage, kind of carriers, diluents and/or excipients, and other factors well known to those of ordinary skill in the art. In general, the final composition can comprise from 0.0001 percent by weight (% w) to 100% w of the antagonist of artemin function, preferably 0.001 % w to 10% w, with the remainder being any other active agents present and/or carrier(s), diluent(s) and/or excipient(s). For example, the anti-artemin antibodies or antibody fragments described herein may be present in the composition in an amount of about 0.001 to 99.99 wt %, more preferably about 0.01 to 20 wt %, and still more preferably about 1 to 5 wt %. In particular, the antibody or antibody fragment can be present at a concentration of 1-100 mg/ml, e.g., 10 mg/ml. These antibodies or antibody fragments can be provided in the form of lyophilized or aqueous solutions. Other amounts may also be suitable.

Carriers generally, and pharmaceutically-acceptable carriers in particular, are well known in the art. The carrier may be provided in a separate sterile container or in admixture with the antibody. Typically, saline, aqueous alcoholic solutions, albumin-saline solutions, and propylene glycol solutions are suitable. However, others may also be utilized. Acceptable carriers, excipients or stabilizers are nontoxic to recipients at the dosages and concentrations employed, and include buffers such as phosphate, citrate, or acetate at a pH typically of 5.0 to 8.0, most often 6.0 to 7.0; salts such as sodium chloride, potassium chloride, etc. to make isotonic; antioxidants, preservatives, low molecular weight polypeptides, proteins, hydrophilic polymers such as polysorbate 80, amino acids, carbohydrates, chelating agents, sugars, and other standard ingredients known to those skilled in the art (Remington's Pharmaceutical Science 16th edition, Osol, A. Ed. 1980).

The antagonist of artemin function for use in the invention can also be provided as a composition along with a carrier or diluent for use in vitro, preferably a pharmaceutically-acceptable carrier or diluent for use in vivo and ex vivo. The antagonist of artemin function for use in the invention can be provided as substantially pure from undesired contaminants. This means that the antagonist of artemin function is typically at least about 50% w/w (weight/weight) pure, as well as being substantially free from interfering proteins and contaminants. Preferably the antagonist of artemin function is at least 90, 95% or 99% w/w pure. Pharmaceutical compositions for are usually sterile, substantially isotonic and prepared in accordance with Good Manufacturing Practices of the FDA or similar body.

When utilized for therapeutic purposes the antagonists of artemin function, such as anti-artemin antibodies or antibody fragments should be of a purity suitable for human administration. The composition may contain other ingredients as is known in the art. In particular, other therapeutic drugs, carriers or diluents, immunological adjuvants and the like may be also be added. When the composition described above is utilized for in vivo imaging or diagnosis, it may comprise about 0.001 to 99.9 wt % antagonist of artemin function, and more preferably about 0.01 to 25 wt % antagonist of artemin function. Typically, when the composition is utilized for therapeutic purposes it may contain about 0.001 to 99.9 wt % antagonist of artemin function, and more preferably about 0.01 to 30 wt % antagonist of artemin function. When utilized for the ex vivo targeting of cells in bodily fluids such as spinal fluid, the composition may comprise about 0.0001 to 50 wt %, and preferably about 0.01 to 20 wt % antagonist of artemin function. When applied to the in vitro diagnosis of cancer cells the composition of the invention may comprise about 0.001 to 35 wt % antagonist of artemin function, and more preferably about 0.01 to 10 wt % antagonist of artemin function. Other amounts, however, may also be suitable.

In certain embodiments, particularly where the antagonist of artemin function is an anti-artemin antibody, the patient may be imaged in vivo and/or diagnosed by administration of the antagonist of artemin function in radiolabeled form, in an amount effective to reach the locus of the cancer, and further non-invasive detection of any localization of the labeled antagonist of artemin function at the tumor cells. In certain aspects, the antagonist of artemin function may be administered in an amount of about 0.001 to 5000 mg/kg weight per treatment, more preferably about 0.01 to 5000 µg/kg weight per treatment, and more preferably about 0.1 to 500 µg/kg weight per treatment. However, other amounts may also be utilized. Radiolabels that may be utilized are ¹¹¹In, ¹²⁵I, ⁹⁹mTc, and ¹³¹I, among others. These radioisotopes may be detected with a PET scanner, and with an NMR imaging and/or radioactivity counting apparatus that are in wide use by the medical community, depending on the radio label utilized.

For immunotoxins, one will generally desire to prepare it into a pharmaceutical composition that may be administered parenterally. This is done by using for the last purification step a medium with a suitable pharmaceutical composition. Such formulations will typically include pharmaceutical buffers, along with excipients, stabilizing agents and such like. The pharmaceutically acceptable compositions will be sterile, non-immunogenic and non-pyrogenic. Details of their preparation are well known in the art and are further described herein. It will be appreciated that endotoxin contamination should be kept minimally at a safe level, for example, less that 0.5 ng/mg protein. Suitable pharmaceutical compositions in accordance with the invention will generally comprise from about 10 to about 100 mg of the immunotoxin admixed with an acceptable pharmaceutical diluent or excipient, such as a sterile aqueous solution, to give a final concentration of about 0.25 to about 2.5 mg/ml with respect to the antibody-toxin conjugate.

### Methods of treatment

The studies presented herein investigate the reversal of resistance to chemotherapeutics and radiotherapy in breast cancer cells. Without wishing to be bound by any theory, the applicants predict antagonism of artemin functionality being applicable to the reversal of resistance to treatment with a variety of breast cancer therapies.

Also disclosed herein is a method of preventing, reducing, or inhibiting cell proliferation, cell survival, and/or cell motility by antagonising artemin functionality by reversing resistance to a therapy in one or more breast cancer cells. Preferably, the method is for the treatment of a breast cancer characterised by aberrant cell proliferation, cell survival, and/or cell motility in subject. These aberrant characteristics may occur in one or more cell type within a subject and can include metastatic disorders.

In various embodiments, the breast cancer therapy includes treatment targeted to epithelial tumours (e.g., from cells lining ducts or lobules) or nonepithelial tumours (e.g., from the supporting stroma), such as angiosarcomas, primary stromal sarcomas, and phyllodes tumor. In other exemplary embodiments, the breast cancer thereapy includes treatment targeted to carcinomas, for example, carcinomas *in situ*, including lobular carcinoma in situ (LCIS), ductal carcinoma *in situ*, as well as those targeted to invasive cancers, including adenocarcinomas, and those targeted to rare forms of breast cancer including medullary, mucinous, and tubular carcinomas, Paget's disease of the nipple, and metastatic breast cancer.

It will be appreciated by those of general skill in the art to which the invention relates, having regard to the nature of the invention and the results reported herein, that the present invention is applicable to a variety of different animals. Accordingly, the diagnostic and treatment methods can apply to any animal of interest. In particular, the invention is applicable to mammals, more particularly humans.

Persons of ordinary skill in the art to which the invention relates will appreciate various means and agents for use in inhibiting artemin functionality. By way of example, antagonists of artemin function such as antibodies directed against artemin, or functional modifications of such antibodies may be used. Exemplary antagonistic agents are described in detail herein. Antagonists of use in the invention will antagonise one or more biological effects of artemin and preferably exhibit the ability to reverse resistance to a therapy in one or more breast cancer cells, with the proviso that the antagonists are as defined in the claims. Those skilled in the art will recognise that such antagonists may thereby enable therapy to 1) the ability to prevent, reduce or inhibit cellular proliferation; 2) the ability to prevent, reduce or inhibit cellular survival; 3) the ability to prevent, reduce or inhibit cellular motility; 4) the ability to prevent, reduce or inhibit expression or activity of artemin and/or a related ligand; 5) the ability to prevent, decrease, reduce or control metastasis of breast tumours.

A number of immunotherapeutic strategies involving innate or acquired immunity may be used to antagonise artemin functionality, including (a) local application of a live bacterial vaccine; (b) use of cytokines; (c) active immunization against artemin; (d) passive therapy with antibodies against artemin and (e) adoptive transfer of effector cells (e.g., T cells). Active immunization against artemin can be used to induce immune responses, or passive immunization with a humanised monoclonal antibody directed against artemin may be employed. For general guidance, see, e.g., Riethmüller G, et al., Monoclonal antibody therapy for resected Dukes' C colorectal cancer: seven-year outcome of a multicenter randomized trial. J Clin Oncol 1998;16:1788-1794; Riethmüller G, et al., Randomized trial of monoclonal antibody for adjuvant therapy of resected Dukes' C colorectal carcinoma. German Cancer Aid 17-1A Study Group. Lancet 1994;343:1177-1183; Herlyn DM, et al., Inhibition of growth of colorectal carcinoma in nude mice by monoclonal antibody. Cancer Res 1980;40:717-721.

For immunization, pure antigens are often ineffective in inducing an acquired (i.e., antigen-specific) immune response unless certain adjuvants are used to stimulate innate immunity. Therefore, numerous approaches are designed to stimulate innate immunity at the site of vaccinations by the use of chemical and/or bacterial agents. Synthetic peptides used in vaccines can be designed for particular MHC haplotypes (see, e.g., Toes RE et al. Peptide vaccination can lead to enhanced tumor growth through specific T-cell tolerance induction. Proc Natl Acad Sci USA 1996;93:7855-7860). Antigenic peptides can be loaded onto heat-shock protein (or as recombinant virus-like particles) to increase the efficacy of immunization. Generally, effective induction of an immune response requires antigen presentation in an environment that provides appropriate help or secondary signals.

Several experimental designs use dendritic cells pulsed with virus-specific or tumor-associated peptides to induce tumor-reactive T cells and rejection of transplanted tumor cells. Dendritic cells can be loaded with synthetic antigenic peptides or recombinant proteins. Dendritic cells can also be loaded with one or more of: native peptides stripped from tumor cell surfaces; tumor-derived, peptide-loaded heat-shock proteins; tumor-derived mRNA; or fused tumor cells (for review, see Shurin MR. Dendritic cells presenting tumor antigen. Cancer Immunol Immunother 1996;43:158-164). One advantage of these strategies is that powerful immunity can be induced to (unique) individually distinct tumor antigens, as well as tumor-associated antigens.

For antigens for artemin, recombinant vaccines can be developed using vaccinia, Listeria, or virus-like particles. In other approaches, genetic vaccination can be used, for example, by injecting naked DNA plasmid constructs, intramuscularly encoding the tumor antigen (see e.g., Donnelly JJ, Ulmer JB, Liu MA. DNA vaccines. Life Sci 1997;60:163-172). GM-CSF may also be used to improve the presentation of the antigen by dendritic cells at the site of injection (see, e.g., Syrengelas AD, Chen TT, Levy R. DNA immunization induces protective immunity against B-cell lymphoma. Nat Med 1996;2:1038-1041). In other approaches, vaccination with anti-idiotypic antibodies can be used.

As further approaches, passive antibody therapy or adoptive transfer of tumor-specific T cells can be used. For example, passive immunization with an antibody to artemin can protect against challenge with tumor cells and can be therapeutic when given soon after challenge with the cancer cells (e.g., see Riethmuller G, et al. Monoclonal antibody therapy for resected Dukes' C colorectal cancer: seven-year outcome of a multicenter randomized trial. J Clin Oncol 1998;16:1788-1794; Riethmuüler G, et al. Randomized trial of monoclonal antibody for adjuvant therapy of resected Dukes' C colorectal carcinoma. German Cancer Aid 17-1A Study Group. Lancet 1994;343:1177-1183; Herlyn DM, et al. Inhibition of growth of colorectal carcinoma in nude mice by monoclonal antibody. Cancer Res 1980;40:717-721).

As alternate approaches, anti-idiotypic antibody treatment can be used to induce cancer cells to go into a long-lasting dormant state (see, e.g., Miller RA, Maloney DG, Warnke R, Levy R. Treatment of B-cell lymphoma with monoclonal anti-idiotype antibody. N Engl J Med 1982;306:517-522). In addition, antibodies to tumour cells can be used as carriers for cytokines or cytotoxic agents, such as radiochemicals or natural toxins (see, e.g., Ghetie V, Vitetta E. Immunotoxins in the therapy of cancer: from bench to clinic. Pharmacol Ther 1994;63:209-234; Reisfeld RA, Gillies SD. Recombinant antibody fusion proteins for cancer immunotherapy. Curr Top Microbiol Immunol 1996;213:27-53). The recombinant antibody-cytokine or antibody-toxin fusion proteins may be used to concentrate these agents in the stroma surrounding the tumor cells. As alternatives, bispecific monoclonal antibodies can be engineered to bind effector cells as well as tumor antigens on the cancer cells. Monoclonal antibodies can also be humanized to reduce the stimulation of neutralizing anti-murine antibodies by patients. As still further approaches, adoptive transfer of T cells can be used with longer established tumor loads. T cells that have been isolated from patients can be expanded in vitro with IL-2 and then infused into patients who receive IL-2 as well (see, e.g., Smith CA, et al. Adoptive immunotherapy for Epstein-Barr virus-related lymphoma. Leuk Lymphoma 1996;23:213-220).

The efficacy of an agent in inhibiting artemin functionality may be determined having regard to the description of the invention herein and known methodology. For example, efficacy of agents may be determined by observing their ability to prevent, reduce, or inhibit expression of artemin or to antagonise one or more of the functional effects of artemin. By way of example, the effect of the antagonist of artemin function on one or more of cellular invasion, cellular migration, the level of gene transcription, and the level of expression or lack thereof or genes responsive to artemin may be studied. Such studies may be conducted *in vitro* or *in vivo.* It will be appreciated that for use in the methods and compositions of the present invention, antagonists of artemin functionality that are able to resensitise one or more cancer cells to a therapy are preferred.

The assays described herein, including those described in the Examples, may be used to determine the suitability of an antagonist of artemin function in accordance with the invention. Specifically, RT-PCR and Northern blot analysis can be used to detect expression at the mRNA level, and Western blotting and direct or indirect immunostaining can be used to detect the expression at the protein level. To detect activity, cell-based assays for cell proliferation, cell survival, cell motility, and/or oncogenicity can be used. Regarding inhibition of metastasis, an *in vivo* assay may be used, as described, for example, in Fidler, I. J. (1973) Nat. New Biol. 242, 148-149; and Price J. E. The biology of cancer metastasis. Prog. Clin. Biol. Res., 354A: 237-255, 1990, or Kerbel R. S. What is the optimal rodent model for anti-tumor drug testing? Cancer Metastasis Rev., 17: 301-304, 1998; Killion J. J., Radinsky R., Fidler I. J. Orthotopic models are necessary to predict therapy of transplantable tumours in mice. Cancer Metastasis Rev., 17: 279-284, 1998; and Price J. E. Analyzing the metastatic phenotype. J. Cell. Biochem., 56: 16-22, 1994. Similarly, methods for assessing induction or inhibition of apoptosis are well known in the art, and pro-apoptotic and anti-apoptotic cellular markers are likewise well known.

### Administration routes/regimes

Administration of the antagonist of artemin functionality or compositions of the invention may be by any means capable of delivering the desired activity (e.g., inhibition of artemin functionality) to a target site within the body of a subject. Such agents and compositions can thereby be used to reverse resistance in a cancer cell to a therapy, such as resistance to a therapeutic agent, and so can be used in a therapy to inhibit cell proliferation, cell survival, and/or cell motility at the target site. A target site may be any site within the body which may have or be susceptible to a disorder, and may include one or more cells, tissues or a specific tumor. For example, administration may include parenteral administration routes, systemic administration routes, oral and topical administration. Administration may be by way of injection, subcutaneous, intraorbital, ophthalmic, intraspinal, intracisternal, topical, infusion (using, e.g., slow release devices or minipumps such as osmotic pumps or skin patches), implant, aerosol, inhalation, scarification, intraperitoneal, intracapsular, intramuscular, intratumoral, intranasal, oral, buccal, transdermal, pulmonary, rectal or vaginal delivery. As will be appreciated, the administration route chosen may be dependent on the position of the target site within the body of a subject, as well as the nature of the agent (e.g., an antibody or antibody fragment) or composition being used.

In a specific embodiment, polynucleotides may be administered for example by infection using defective or attenuated retroviral or other viral vectors (see e.g., U.S. Patent No. 4,980,286), or by direct injection of naked DNA, or by use of microparticle bombardment (e.g., a gene gun; Biolistic, DuPont); by coating with lipids or cell-surface receptors or transfecting agents; encapsulation in liposomes, microparticles, or microcapsules; by linkage to a peptide which is known to enter the nucleus; or by administering it in linkage to a ligand subject to receptor-mediated endocytosis (see, e.g., Wu and Wu, 1987, J. Biol. Chem. 262:4429-4432), which can be used to target cell types specifically expressing the receptors, and the like. In addition, nucleic acid-ligand complexes can be formed in which the ligand comprises a fusogenic viral peptide to disrupt endosomes, allowing the nucleic acid molecules to avoid lysosomal degradation.

In yet another embodiment, the polynucleotides can be targeted *in vivo* for cell specific uptake and expression, by targeting a specific receptor, as described for example in WO 92/06180 dated April 16, 1992 (Wu et al.); WO 92/22635 dated December 23, 1992 (Wilson et al.); WO 92/20316 dated November 26, 1992 (Findeis et al.); WO 93/14188 dated July 22, 1993 (Clarke et al.); and, WO 93/20221 dated October 14, 1993 (Young). Alternatively, the polynucleotides can be introduced intracellularly and incorporated within host cell DNA for expression, by homologous recombination Koller and Smithies, 1989, Proc. Natl. Acad. Sci. USA 86:8932-8935; Zijlstra et al., 1989, Nature 342:435-438. Cells into which polynucleotides can be introduced for purposes of the present invention encompass any desired, available cell type. The appropriate cell type will depend on the nature of the disorder to be treated. However, by way of example, the polynucleotide can be introduced to a cancer cell.

As will be appreciated, the dose of an agent or composition administered, the period of administration, and the general administration regime may differ between subjects depending on such variables as the nature of the condition to be treated, severity of symptoms of a subject, the size of any tumour to be treated, the target site to be treated, the mode of administration chosen, and the age, sex and/or general health of a subject. Persons of average skill in the art to which the invention relates will readily appreciate or be able to determine appropriate administration regimes having regard to such factors. It should be appreciated that administration may include a single daily dose or administration of a number of discrete divided doses as may be appropriate. The invention also contemplates the administration regimes which combine different modes or routes of administration. For example, intratumoural injection and systemic administration could be combined.

It should be appreciated that a method of the invention may comprise further steps such as the administration of additional agents or compositions, particularly one or more breast cancer therapeutics, or other agents which may be beneficial to a subject having regard to the condition to be treated. For example, other agents of use in treating proliferative disorders (such as the anti-neoplastic agents mentioned above) may be administered. It should be appreciated that such additional agents and compositions may be administered concurrently with the agents and compositions of the invention, or in a sequential manner. For example, the additional agents or compositions could be administered before or after administration of the antagonist of artemin function or compositions comprising an antagonist of artemin function. It should be appreciated in relation to sequential delivery of agents or compositions, that sequential administration of one agent or composition after the other need not occur immediately, although this may be preferable. There may be a time delay between delivery of the agents or compositions. The period of the delay will depend on factors such as the condition to be treated and the nature of the compositions or agents to be delivered. However, by way of example, the invention contemplates periods of between hours to several days or months.

In particular aspects, the antagonist of artemin function is formulated for parental administration by intravenous infusion or bolus injection, intramuscularly or subcutaneously. Intravenous infusion can be given over as little as 15 minutes, but more often for 30 minutes, or over 1, 2 or even 3 hours. The antagonist of artemin function can also be injected directly into the site of disease (e.g., a tumor), or encapsulated into carrying agents such as liposomes. The dose given will be sufficient to alleviate the condition being treated and is likely to be 0.1 to 5 mg/kg body weight, for example 1, 2, 3 or 4 mg/kg, but may be as high as 10 mg/kg or even 15 or 20 mg/kg. A fixed unit dose may also be given, for example, 50, 100, 200, 500 or 1000 mg, or the dose may be based on the patient's surface area, e.g., 100 mg/m². Usually between 1 and 8 doses, (e.g., 1, 2, 3, 4, 5, 6, 7 or 8) are administered to treat cancer, but 10, 20 or more doses may be given. The antagonist of artemin function can be administered daily, biweekly, weekly, every other week, monthly or at some other interval, depending, e.g. on the half-life of the reagent, for 1 week, 2 weeks, 4 weeks, 8 weeks, 3-6 months or longer. Repeated courses of treatment are also possible, as for chronic administration. The regime of a dosage and intervals of administration is designed to alleviate or at least partially arrest the symptoms of the disease (biochemical, histologic and/or clinical), including its complications and intermediate pathological phenotypes in development of the disease.

In other aspects, the pharmaceutical compositions suitable for use in the invention can be used in prophylaxis of a patient at risk of breast cancer. Such patients include those having genetic susceptibility to breast cancer, patients who have undergone exposure to carcinogenic agents, such as radiation or toxins, and patients who have undergone previous treatment for breast cancer and are at risk of recurrence. A prophylactic dosage is an amount sufficient to eliminate or reduce the risk, lessen the severity, or delay the outset of the disease, including biochemical, histologic and/or clinical symptoms of the disease, its complications and intermediate pathological phenotypes presenting during development of the disease.

In exemplary embodiments, treatment of breast cancer with one or more antagonists of artemin functionality increases the median progression-free survival or overall survival time of patients with breast cancer (especially when relapsed or refractory), and in some embodiments increases such survival by at least 30%, or 40%, but preferably 50%, 60% to 70% or even 100% or longer, compared to the same treatment (e.g., chemotherapy) in the absence of antagonism of artemin functionality. In addition or alternatively, treatment (e.g., standard chemotherapy) with one or more antagonists of artemin functionality, increases in certain embodiments the complete response rate, partial response rate, or objective response rate (complete+partial) of patients with cancers (especially when relapsed or refractory), and in some embodiments by at least 30%, or 40%, but preferably 50%, 60% to 70% or even 100% compared to the same treatment (e.g., chemotherapy) in the absence of antagonism of artemin functionality.

### Diagnostic methods and compositions

As described herein, the methods and compositions of the invention find particular use in anti-breast cancer therapies, for example, therapies for preventing, decreasing, or inhibiting cell proliferation, cell survival, cell motility, and/or oncogenicity, and are directed to reversing resistance in breast cancer cells. In various embodiments the methods of the invention are suitable for the determination or monitoring of resistance in one or more breast cancer cells, including diagnosis of resistance in one or more breast cancer cells in a patient. In one broad embodiment the invention provides a method of inhibiting artemin functionality, for example by blocking the interaction of artemin with one or more receptors, or more broadly, blocking the interaction of artemin or a mediator of artemin functionality with a binding agent, the method comprising contacting an antagonist of artemin function, such as an anti-artemin antibody, antibody fragment, or modification thereof, with the breast cancer cell, and determining the sensitivity of the cell to a therapy . This method may be conducted *in vivo* or *in vitro.* Persons of ordinary skill in the art will readily appreciate methods for determining the efficacy of an antagonist of artemin function in reversing resistance in a breast cancer cell to a therapeutic agent. However, by way of example, the methodology described elsewhere herein, including one or more of the assays referred to in the "Examples" section, may be used.

Information of use in diagnosing or generally monitoring the resistant status of a breast cancer cell or of a breast cancer in a subject may be gained by making a direct comparison of the sensitivity of a cell in a test sample to a therapeutic agent in the presence and in the absence of an antagonist of artemin function, or a comparision of the sensitivity of a cell in a test sample to a therapeutic agent with that of a determined base level or standard.

Preferably, to be indicative of resistance a statistically significant difference between sensitivity under different conditions is observed. However, even where there is no statistically significant difference, results obtained may provide valuable information about the status of a breast cancer cell, a breast tumour-bearing subject, or a breast cancer in a subject. It should be appreciated that the sensitivity of breast cancer cells to various agents may differ depending on cell type, sampling procedure, exposure to other agents, and the like. Those skilled in the art will be well aware of methods to control for such variation.

It should be appreciated that diagnosis or general determination of the resistant status of a breast cancer cell or of a breast cancer in a subject may be made by comparing sensitivity to an agent observed in a test sample against a database of results obtained from a range of other subjects. Instead of utilising a standard or base level of sensitivity obtained from a number of normal subjects, the base level of sensitivity may be determined from a single subject during a period when they were known not to present a medical disorder, or during a period of an active medical disorder. This may be particularly applicable to cases of ongoing and/or intermittent disease, to breast cancer recurrence, or to events or disorders where constant monitoring of the subjects status is required. For example, a base level may be determined during a period of remission from the breast cancer and the diagnostic procedure carried out at various times thereafter to assess status. This may provide valuable information pertaining to progression of breast cancer, or help in assessing whether treatment of the breast cancer is proving successful.

In one embodiment, the invention relates to use of the inhibition of artemin functionality in methods of diagnosing a disorder characterised by resistance to one or more anti-breast cancer therapies. Such disorders are typically associated with cell proliferation, cell survival, cell motility, and/or oncogenicity, and particularly refractory cell proliferation, cell survival, cell motility, and/or oncogenicity. Preferably, the method is for the diagnosis of a breast cancer characterised by aberrant cell proliferation, cell survival, cell motility, and/or oncogenicity in a subject. This aberrant cell growth and/or proliferation may occur in one or more cell type within a subject and can include metastatic or other breast cancers.

In accordance with the invention, an antagonist of artemin function, for example one or more antibodies which specifically bind artemin, may be used for the diagnosis of breast cancers characterized by altered expression of artemin, or in assays to monitor patients being treated. Specifically contemplated are diagnostic methods reliant on one or more anti-artemin antibodies. The antibodies useful for diagnostic purposes may be prepared in the same manner as those described above. Diagnostic assays include methods which utilize the antibody and a label to detect the corresponding peptide or polypeptide in human body fluids or extracts of cells or tissues. The antibodies may be used with or without modification, and may be labeled by joining them, either covalently or non-covalently, with a reporter molecule. A wide variety of reporter molecules which are known in the art may be used, several of which are described herein.

A variety of protocols, including ELISA, RIA, and FACS, are known in the art and provide a basis for diagnosing altered or abnormal levels of expression for artemin. Normal or standard values for expression are established by combining body fluids or cell extracts taken from normal mammalian subjects, preferably human, with antibody under conditions suitable for complex formation. The amount of standard complex formation may be quantified by various methods, but preferably by photometric or fluorometric means. Quantities expressed in subject, control, and disease, samples from biopsied tissues are compared with the standard values. Deviation between standard and subject values establishes the parameters for diagnosing disorders.

The antibodies or antibody fragments may be used to detect and quantitate expression in biopsied tissues in which expression may be correlated with breast cancer, or with sensitivity to a particular treatment, for example treatment with one or more chemotherapeutics. The diagnostic assay may be used to distinguish between absence, presence, and altered expression, and to monitor regulation of levels during therapeutic intervention.

Antagonists of artemin function may be used for the diagnosis of breast cancers which are associated with either increased or decreased expression, and may be used in qualitative or quantitative methods are well known in the art. In a particular aspect, the antagonist of artemin function may be useful in assays that detect activation, induction, or resistance of various breast cancers, particularly those mentioned above. Such assays may also be used to evaluate the efficacy of a particular therapeutic treatment regimen in animal studies, in clinical trials, or in monitoring the treatment of an individual patient.

In order to provide a basis for the diagnosis of a disorder associated with resistance to a breast cancer therapy, such as a chemotherapeutic, a normal or standard profile for expression is established. This may be accomplished by combining body fluids or cell extracts taken from normal subjects, either animal or human, with antagonists of artemin function and the chemotherapeutic. Normal sensitivity to the chemotherapeutic may be quantified by comparing the values obtained from normal subjects, or from a breast cancer cell line known to be sensitive to the chemotherapeutic. Standard values obtained from normal samples may be compared with values obtained from samples from patients who are symptomatic for breast cancer. Deviation between standard and subject values is used to establish the presence of resistant breast cancer, that is, presence of resistance to the chemotherapeutic.

Once the resistant breast cancer is diagnosed and a treatment protocol is initiated, assays may be repeated on a regular basis to evaluate whether the level of sensitivity in the patient begins to approximate that which is observed in the normal patient. The results obtained from successive assays may be used to show the efficacy of treatment over a period ranging from several days to months. With respect to resistant breast cancer, the presence of a relatively high amount of artemin polypeptide in biopsied tissue from an individual may indicate a predisposition for the development of resistance, or may provide a means for detecting resistance prior to the appearance of actual clinical symptoms of resistance. A more definitive diagnosis of this type may allow health professionals to employ preventative measures or aggressive treatment earlier thereby preventing the development or further progression of the breast cancer or the acquisition of resistance to one or more anti-breast cancer therapies.

Those skilled in the art will recognises that insofar as the following specifically discusses the use of anti-artemin antibodies as exemplary antagonists of artemin functionality, other antagonists may be substituted in such uses as appropriate.

Methods which may also be used to quantitate the expression of artemin include radio labeling antibodies, binding with a control polypeptide, and standard curves onto which the experimental results are interpolated. The speed of quantitation of multiple samples may be accelerated by running the assay in an ELISA format where the sequence of interest is presented in various dilutions and a spectrophotometric or colorimetric response gives rapid quantitation.

In further embodiments, antibodies, antibody fragments, or modifications thereof as described herein may be used as reagents in a microarray. The microarray can be used to monitor the expression level of large number of samples simultaneously and to develop and monitor the activities of therapeutic agents. The microarrays may be prepared and used according to the methods known in the art. The microarray substrate may be paper, nylon or any other type of membrane, filter, chip, plate such as a microtiter plate, glass slide, or any other suitable solid support. In one aspect, a gridded array analogous to a dot or slot blot may be used to link samples to the surface of a substrate. In yet another aspect, an array may be produced by hand or by using available devices, materials, and machines (including multichannel pipettors or robotic instruments) and may include about 8, 24, 96, 384, 1536 or 6144 samples, or any other multiple from 2 to 1,000,000, which lends itself to the efficient use of commercially available instrumentation.

In order to conduct sample analysis using the microarrays, polypeptides may be extracted from a biological sample. The biological samples may be obtained from any bodily fluid (e.g., blood, urine, saliva, phlegm, gastric juices, etc.), cultured cells, biopsies, or other tissue preparations. Labeled antibodies or antibody fragments may be incubated with the microarray so that they bind to the polypeptides of the microarray. Incubation conditions can be adjusted so that binding occurs with specificity. After removal of unbound antibodies, a scanner can be used to determine the levels and patterns of label. The scanned images are examined to determine the relative abundance of a polypeptide sequence on the microarray. A detection system may be used to measure the absence, presence, and amount of binding for a number of distinct sequences simultaneously.

In another embodiment, the antibodies suitable for use in the invention may be used for imuunohistochemistry-based applications. In accordance with standard immunohistochemistry techniques, thin slices (e.g., about 4-40 µm) can be taken from the tissue of interest, or the tissue can be used whole. The slicing can be accomplished through the use of a microtome, and slices can be mounted on slides. The tissue can then be treated to rupture the cell membranes, e.g., using detergent such as Triton X-100. Additional unmasking steps can also be used, as well as blocking steps to minimize non-specific binding. For direct immunohistochemistry, the labeled antibody of interest (e.g. FITC conjugated antiserum) is used to bind the antigen in the tissue sections. For indirect immunohistochemistry, an unlabeled primary antibody is used to bind to the tissue antigen, and a labeled secondary antibody is used to react with the primary antibody.

Anti-artemin antibodies and antibody fragments may also be used as *in vivo* diagnostic agents to provide an image of breast cancer cells (e.g., tumors) or respective metastases, such as those that are resistant to one or more anti-breast cancer therapies. Various diagnostic methods such as magnetic resonance imaging (MRI), X-ray imaging, computerized emission tomography and similar technologies may be employed. In this type of imaging, the antibody portion used will generally bind to the breast cancer marker, such as artemin, and the imaging agent will be an agent detectable upon imaging, such as a paramagnetic, radioactive or fluorescent molecule. Many appropriate imaging agents are known in the art, as are methods for their attachment to antibodies (see, e.g., US 5,021,236 and US 4,472,509,). Certain attachment methods involve the use of a metal chelate complex employing, for example, an organic chelating agent such a DTPA attached to the antibody (U.S. Pat. No. 4,472,509). Antibodies also may be reacted with an enzyme in the presence of a coupling agent such as glutaraldehyde or periodate. Conjugates with fluorescein markers are prepared in the presence of these coupling agents or by reaction with an isothiocyanate.

In the case of paramagnetic ions, exemplary agents include chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and erbium (III), with gadolinium being particularly preferred. Ions useful in other contexts, such as X-ray imaging, include but are not limited to lanthanum (III), gold (III), lead (II), and especially bismuth (III). In the case of radioactive isotopes for diagnostic application, useful agents include astatine²¹¹, ¹⁴carbon, ⁵¹chromium, ³⁶chlorine, ⁵⁷cobalt, ⁵⁸cobalt, copper⁶⁷, ¹⁵²Eu, gallium⁶⁷, ³hydrogen, iodine¹²³, iodines¹²⁵, iodine¹³¹, indium¹¹¹, ⁵⁹iron, ³²phosphorus, rhenium¹⁸⁶, rhenium¹⁸⁸, ⁷⁵selenium, ³⁵sulphur, technicium⁹⁹m and yttrium⁹⁰. ¹²⁵I is commonly used, and technicium⁹⁹m and indium¹¹¹ are also often used due to their low energy and suitability for long range detection. Elements particularly useful in MRI include the nuclear magnetic spin-resonance isotopes ¹⁵⁷Gd, ⁵⁵Mn, ¹⁶²Dy, ⁵²Cr, and ⁵⁶Fe, with gadolinium often being preferred.

Radioactively labeled antibodies and antibody fragments for use in the present invention may be produced according to well-known methods in the art. For instance, antibodies can be iodinated by contact with sodium or potassium iodide and a chemical oxidizing agent such as sodium hypochlorite, or an enzymatic oxidizing agent, such as lactoperoxidase. Antibodies according to the invention may be labeled with technetium⁹⁹m by ligand exchange process, for example, by reducing pertechnate with stannous solution, chelating the reduced technetium onto a Sephadex column and applying the antibody to this column or by direct labeling techniques, e.g., by incubating pertechnate, a reducing agent such as SNC¹², a buffer solution such as sodium-potassium phthalate solution, and the antibody. Intermediary functional groups which are often used to bind radioisotopes which exist as metallic ions to antibody are diethylenetriaminepentaacetic acid (DTPA) and ethylene diaminetetracetic acid (EDTA).

A factor to consider in selecting a radionuclide for *in vivo* diagnosis is that the half-life of a nuclide be long enough so that it is still detectable at the time of maximum uptake by the target, but short enough so that deleterious radiation upon the host, as well as background, is minimized. Ideally, a radionuclide used for *in vivo* imaging will lack a particulate emission, but produce a large number of photons in a 140-2000 keV range, which may be readily detected by conventional gamma cameras. Administration of the labeled antibody may be local or systemic and accomplished intravenously, intra-arterially, via the spinal fluid or the like. Administration also may be intradermal or intracavitary, depending upon the body site under examination. After a sufficient time has lapsed for the labeled antibody or fragment to bind to the diseased tissue, in this case cancer tissue, for example 30 min to 48 h, the area of the subject under investigation is then examined by the imaging technique. MRI, SPECT, planar scintillation imaging and other emerging imaging techniques may all be used. The distribution of the bound radioactive isotope and its increase or decrease with time is monitored and recorded. By comparing the results with data obtained from studies of clinically normal individuals, the presence and extent of the diseased tissue can be determined. The exact imaging protocol will necessarily vary depending upon factors specific to the patient, and depending upon the body site under examination, method of administration, type of label used and the like.

### Kits for treatment or diagnosis

The antagonists and compositions of the invention may be used in kits suitable for reversing resistance in breast cancer cells and for the treatment of a breast cancer as defined herein. The antagonists and compositions may also be used in diagnostic kits. Kits can comprise at least one antagonist of artemin function in a suitable container. For example, the kit may include an anti-artemin antibody or antibody fragment, or a modification thereof. The kit may further include one or more ancillary reagents, such as reagents suitable for detecting the presence of a complex between the antagonist and a mediator of artemin functionality, such as between an anti-artemin antibody or antibody fragment and artemin or portion thereof. These can be provided in further separate containers as may be necessary for a particular application. Where a secondary antibody capable of binding to a primary antibody is employed, such secondary antibody can be provided in the kit, for instance in a separate vial or container. The secondary antibody, if present, is typically labeled, and may be formulated in an analogous manner with the antibody formulations described herein. The kit may additionally comprise one or more therapeutic agents, including one or more breast cancer therapeutics, such as one or more chemotherapeutics, including those against which a breast cancer cell may acquire resistance or has acquired resistance.

For a kit comprising an anti-artemin antibody, the antibody composition may be provided either alone or in combination with additional antibodies specific for other epitopes. The antibody or antibody fragment may be labeled or unlabelled, and may be provided with adjunct ingredients, e.g., buffers, such as Tris, phosphate and carbonate, stabilizers, excipients, biocides and/or inert proteins, such as bovine serum albumin. Generally these adjunct materials will be present in less than about 5% weight based on the amount of active antibody, and usually will be present in a total amount of at least about 0.001% weight based on antibody concentration. The antibodies or antibody fragments can be provided as a lyophilized mixture with the adjunct ingredients, or the adjunct ingredients can be separately provided for combination by the user.

In a particular aspect, the kit can include, in an amount sufficient for at least one diagnostic assay, an antagonist of artemin function as a separately packaged reagent. The antagonist of artemin function can be provided as reagent in combination with a solid phase support or bead. The kit can be directed to the isolation of artemin polypeptides or peptides or cells expressing artemin. In specific aspects, a kit comprising an anti-artemin can be directed to FACS analysis. The kit can comprise a hybridoma cell line as disclosed herein, and allow production of an anti-artemin antibody. In particular aspects, a cell culture medium for said hybridoma cell line can be included.

In the case of therapeutic kits, the antagonist of artemin function may be formulated suitable for direct administration to a subject for example, as pharmaceutical compositions. Alternatively, the kit may comprise one or more agents in one container and pharmaceutical diluents, carriers and/or excipients in another; the contents of each container being mixed together prior to administration. Any container suitable for storing and/or administering an agent or composition may be used in a kit of the invention. Suitable containers will be appreciated by persons skilled in the art. By way of example, such containers include vials and syringes. The containers may be suitably sterilised and hermetically sealed. Further, kits of the invention can also comprise instructions for the use and administration of the components of the kit.

The invention is further elucidated with reference to the examples below.

### EXAMPLES

The examples described herein are for purposes of illustrating embodiments of the invention. Other embodiments, methods, and types of analyses are within the scope of persons of ordinary skill in the molecular diagnostic arts and need not be described in detail herein. Other embodiments within the scope of the art are considered to be part of this invention.

### EXAMPLE 1

### Materials and methods

### Cell culture

The mammary carcinoma cell line MCF-7 was obtained from American Type Culture Collection (Manassas, VA).

For estrogen-free cell culture, MCF-7 cells were cultured in phenol red free RPMI-1640 supplemented with 10% dextran coated charcoal stripped fetal bovine serum (CS-FBS) for 3 days before estrogen or antiestrogen treatment.

The tamoxifen resistant (TAMR) cell line was established as described by Shaw *et al.* (2006). Briefly, the parental MCF-7 cells were continuously exposed for 6 months to 1 µM tamoxifen in phenol red free RPMI 1640 medium containing 10% CS-FBS until the cells developed resistance to the growth inhibitory effect of tamoxifen. MCF-7 cells sensitive to tamoxifen, cultured in the same medium without tamoxifen, were designated as tamoxifen sensitive (TAMS) cells.

### Cell number assay

Cells were seeded onto 6-well plates at a density of 5 × 10⁴ cells per well, in triplicate, in 3 ml of complete medium containing 10% or 0.2% FBS. Cells were cultured under these conditions for up to 10 days with medium changed every other day until harvesting. To compare the cell proliferation rate, the total cell number in each well was quantified with a haemocytometer every 2 days. Cell viability was assessed by using trypan blue.

### Cell proliferation assay

MTT (3-[4,5-dimethylthiazol-2-yl]-2,5-diphenyl tetrazolium bromide) assays were used measure cell proliferation. Cells were seeded into 96 well flat bottom tissue culture microplates in 100 µl medium containing 10% FBS at a concentration of 5 × 10³ cells/well. On assay day, 10 µl of the MTT labelling reagent was added at a final concentration 0.5 mg/ml to each well and the cells were labelled for 5h in the incubator. At the end of the incubation period, the media was removed and the converted dye was solubilised with 100 µl solubilising solution (0.01N HCl in 10% SDS in water). The plates were then sealed and placed at 37°C overnight to completely solubilise the purple formazan crystals. The absorbance of the converted dye was measured using Spectra Max 250 multiplate reader at a wavelength of 570 nm with background subtraction at 650 nm. Each treatment was performed at least in triplicate.

### Establishing Artemin depleted cell lines by plasmid-based siRNA.

To test the efficiency of Artemin specific siRNA, assays were performed using the pSilencer-ARTN plasmid encoding siRNA to target the Artemin transcript or the negative control siRNA plasmid pSilencer-CK, together with the pEGFP-C1 vector (EGFP) or the pEGFP-ARTN plasmid in which the Artemin cDNA was inserted within the 3'UTR of EGFP coding sequence. Plasmids were transiently co-transfected into target cells. After 24h of transfection, the expression of EGFP was visualised with a UV-visible fluorescence microscope.

To establish Artemin depleted cell lines, the pSilencer-ARTN plasmid or the pSilencer-CK plasmid was stably transfected into cells. Cell clones were selected by addition of hygromycin in the medium. Transfected cell lines were generated as pools of positive cell clones. The expression of Artemin in the stable clones was determined by RT-PCR and Western blotting.

For each construct, the insert was verified by DNA sequencing.

### Immunoblotting

Cells were washed twice with ice-cold phosphate-buffered saline (PBS) and lysed at 4°C in lysis buffer (20 mM Tris•HCl, pH 7.4, 150 mM NaCl, 1 mM EDTA, 1 mM EGTA, 1% Triton® X-100, 1% Nonidet P-40, 1 µg/ml protease inhibitor cocktail (GE Healthcare) and 0.1 mM PMSF). The lysates were next sonicated and then cleared by centrifugation at 15,000 × g for 15 min at 4°C. SDS-polyacrylamide gel electrophoresis (PAGE) sample buffer (50 mM Tris•HCl, pH 6.8; 2% SDS; 2% β-mercaptoethanol, and bromophenol blue) was added to each sample and the samples were boiled for 5 min. Samples were subjected to discontinuous SDS-PAGE with a 12.5% resolving gel and transferred to nitrocellulose membranes (Hybond™ C-extra) using standard electroblotting procedures. Membranes were blocked with 5% non-fat dry milk in PBS with 0.1% Tween® 20 (PBST) for 1h at room temperature. The blots were then immunolabelled with primary antibodies in PBST containing 1% non-fat dry milk at 4°C overnight.

After incubation with appropriate secondary antibodies at room temperature, immunolabelling was detected by ECL plus™ chemiluminescence as described by the manufacturer (GE Healthcare). Blots were stripped and reprobed with monoclonal antibody against β-actin to ensure equal loading of the cell lysate proteins. Blots were stripped by incubation for 30 min at 50°C in a solution containing 62.5 mM Tris•HCl, pH 6.7; 2% SDS; and 0.7% β-mercaptoethanol. Blots were then washed for 30 min with several changes of PBST at room temperature. Efficacy of stripping was determined by re-exposure of the membranes to ECL plus™. Thereafter, blots were re-blocked and immunolabelled as described above.

### Plasmid constructs

To construct the artemin expression plasmid, the BamHI and BglII fragment containing the coding sequence for human artemin transcript variant 4 (NM_057090) from IMAGE cDNA clone 5453642 was subcloned into pCMV6-XL4 at BglII site. The resultant plasmid was designated pCMV6-XL4-artemin.

In order to construct artemin expression plasmid for subsequent use, two oligos, 5'-atcGCCGCCACCATGGaacttggacttggaggcctctccacgctgtcccactgcccctggc-3' (forward, Kozak sequence in upper case; SEQ ID NO: 35) and 5'-ctaggccaggggcagtgggacagcgtggagaggcctccaagtc caagttccatggcggcggcgat-3' (reverse; SEQ ID NO: 36) were annealed to form the 5' sequence of human artemin with a Kozak sequence at its 5' end, and an AvrII overhang at its 3' end. These annealed oligos, together with the AvrII/AgeI fragment (released from pCMV6-XL4-artemin) containing the rest of the coding sequence for human artemin, were cloned into the pIRESneo3 vector between EcoRV and AgeI sites. The resultant plasmid was designated pIRESneo3-artemin.

The human artemin cDNA fragment coding for the mature C-terminal peptide of 113 amino acids (SEQ ID NO: 4) was subcloned into pQE30 vector (Qiagen) in frame with the N-terminal His tag coding sequence. The resultant construct, pQE30-artemin, encodes an N-terminal peptide of MRGSHHHHHHGS (SEQ ID NO:37) followed by the mature artemin protein comprising 113 amino acids.

To design siRNA oligonucleotides to target Artemin, a DNA sequence, AA(N19), was selected as AACTGGCCTGTACTCACTCAT (SEQ ID NO: 38). This sequence is common for all four Artemin transcript variants. A BLAST search against the human genome sequence showed that only the Artemin gene would be targeted. A pair of oligonucleotides, 5'-gatccgctggcctgtactcactcatttcaagagaatgagtgagtacaggccagttttttggaaa-3' (forward; SEQ ID NO: 39) and 5'- agcttttccaaaaaactggcctgtactcactcattctcttgaaatgagtgagtcaggccagcg-3' (reverse; SEQ ID NO: 40) were cloned into the pSilencer 2.1-U6 hygro vector (Ambion) according to the manufacturer's instructions. The resultant plasmid was designated pSilencer-ARTN. As a negative control, pSilencer-CK was used to encode siRNA having no significant sequence similarity to human gene sequences (Ambion).

### Preparation of total RNA

Total RNA was isolated from cultured cells with Trizol reagent (Invitrogen) at 1 ml/10 cm2 according to the manufacturer's instructions. RNA was resuspended in diethyl pyrocarbonate (DEPC)-treated nuclease-free water. RNA samples were further treated with DNase I for 30 min at 37°C. The reaction was stopped by addition of 25 mM EDTA and incubation at 65°C for 15 min. RNA samples were then purified by extraction in phenol/chloroform (pH 5.2, phenol:chloroform:isoamyl alcohol at 25:24:1) followed by an additional chloroform extraction and ethanol precipitation. Quantification and purity of the RNA was assessed by A260/A280 absorption, and RNA quality was assessed by agarose gel electrophoresis. RNA samples with ratios of A260/A280 greater than 1.6 were stored at - 80°C for further analysis.

### Production of chicken polyclonal antibodies to artemin

Chicken polyclonal antibodies were generated by Commonwealth Biotechnologies, Inc. (Richmond, VA), as follows. Immunization: Chickens were immunized with recombinant human artemin (∼0.5 mg/chicken) with complete Freund's adjuvant on Day 0 and boosted again on days 9, 29, and 39 (∼0.17 mg/chicken). Eggs were collected pre- and post-immunizations.

IgY extraction from egg yolk: The eggs were collected and egg yolk separated and pooled. The purification was performed at 4°C. Egg yolk was diluted and centrifuged. IgY was precipitated from the clear supernatant. The precipitate was separated by centrifugation and dissolved in PBS. The pre- and post-immunization chicken IgY samples were filter sterilised to yield a final concentration of ∼5 mg/ml with purity equal or greater than 90%, and stored at 4°C.

### Production of recombinant human artemin protein in bacteria

*E.coli* Rosetta-gami™ B (DE3) pLysS competent cells transformed with the plasmid pQE30-artemin were grown in 1 litre LB containing 100 µg/ml Carbenicillin at 37°C until A₆₀₀ reached 0.5. Protein expression was then induced by addition of isopropyl-β-D-thiogalactopyranoside (IPTG) to a final concentration of 0.2 mM and the cultures were incubated for an additional 5 to 6h. The cells were harvested and the pellet was resuspended in 50 ml of 20 mM Tris-HCl (pH 8.0), 1 mM EDTA and 0.1 mg/ml lysozyme. The cells were disrupted by sonication followed by a centrifugation at 10,000g for 30 minutes at 4°C to isolate the inclusion bodies. The inclusion bodies were washed by 2% Triton X-100, 20 mM Tris-HCl (pH 8.0) and 1 mM EDTA twice, and then by 20 mM Tris-HCl (pH 8.0) to remove the detergent and EDTA.

Subsequently, the inclusion bodies were solubilised in 50 ml of solubilising buffer (6 M guanidine hydrochloride, 10 mM Tris-HCl and 100 mM NaH₂PO₄, pH 8.0) and rocked overnight at 4°C. After a centrifugation at 10,000g for 30 minutes at 4°C, the supernatant was mixed with 4 ml of Ni-NTA (Qiagen) at 50% slurry and rocked for 1 hour at 4°C. The lysate-resin mixture was then loaded into an empty column, followed by wash with 20 ml of 25 mM imidazole, 10 mM Tri-HCl, 100 mM NaH2PO4, 500 mM NaCl, 8 M Urea (pH 8). The protein was eluted with 1 ml of elution buffer containing 250 mM imidazole, 10 mM Tris-HCl, 100 mM NaH₂PO₄, 150 mM NaCl, 8 M Urea (pH 8.0). The fractions were collected and analysed by SDS-PAGE and visualised by Coomassie blue staining. The yield of purified protein was estimated by Bradford's assay.

### Reagents

### β-estradiol (E₂) and tamoxifen were purchased from Sigma-Aldrich (UK).

### Reverse transcription-PCR

One-step reverse transcription (RT)-PCR kit (Qiagen) was used to determine the presence of artemin transcript using a pair of primers: 5'-CTTTGCAGACTGGACCCTTACC-3 (forward; SEQ ID NO: 41) and 5'-AGCTCCCATGAGTGAGTACAGG-3' (reverse; SEQ ID NO: 42). For RT-PCR, the following procedure was employed. To start, 1 µg of total RNA was diluted to 0.1 µg/µl to minimize the variation of sample handling. This dilution was treated by DNase I for 15 min, followed by inactivation of DNase by adding EDTA to 5 mM and heating to 70°C for 15 min. The DNase-treated RNA was then mixed with a master cocktail containing RT-PCR buffer, forward and reverse primers, dNTPs, RNase inhibitor, an enzyme mixture containing reverse transcriptase (Omniscript and Sensiscript) and HotStart Taq DNA polymerase at the concentrations recommended by the manufacturer to a final volume of 50 µl.

The temperature-cycle protocol included: 60 min at 50°C for the reverse transcription reaction, followed by denaturation and activation of HotStart DNA polymerase for 15 min at 95°C, and PCR amplification for 20 sec at 95°C, 30 sec at 54-62°C, and 1 min at 72°C for 30 cycles. A final extension for 5 min at 72°C was performed at the end of the cycles.

β-actin was similarly amplified by RT-PCR using 0.2 µg of total RNA as an internal control with a pair of primers: 5'-ATGATATCGCCGCGCTCG-3' (forward; SEQ ID NO: 43) and 5'-CGCTCGGTGAGGATCTTCA-3' (reverse; SEQ ID NO: 44).

Ten microlitres of each of the RT-PCR product was fractionated on 1% agarose gels. The identity of RT-PCR product was confirmed by the size, restriction enzyme digestion, and DNA sequencing.

### Soft agar assay for colony formation

For soft agar colony formation assay, cells were cultured in 96-well plates first covered with a layer of agar (0.5%). Cells were grown in medium containing 10% FBS. 5x10³ cells were seeded into 100 µl 0.35% agarose and onto the 0.5% agarose base layer. 100 µl serum-supplemented medium containing drug or antibody was added on the top and changed every 3 days. The plates were incubated at 37°C in a humidified incubator. After 10 days, alarmarBlue® was added (1:10 volume reagent, Invitrogen) and incubated at 37°C for 4 hours, followed by measurement of fluorescence with excitation wavelength at 530 nm and emission wavelength at 590nm.

### Soft agar growth assays

To perform soft agar colony formation assays, 50 µl of 0.5% agarose in serum containing medium was plated onto each well of 96-well flat-bottom microplates, as a base agar layer. Next, 5 × 10³ cells in a 100 µl mixture of 0.35% agarose in serum containing medium were plated onto the base layer. Subsequently, 100 µl of the medium was added to prevent drying of the agarose gels. For antibody treatment, the antibodies at the indicated concentrations were added into the top agar layer and the culture medium to ensure the complete exposure of cells to antibodies. The plates were incubated at 37°C in a humidified incubator. The top culture medium including the antibodies was changed every 3 days. After 10 days, 20 µl of 5 mg/ml MTT (Sigma Chemical Company, St Louis, MO, USA) was added into each well and incubated at 37°C for 4h followed by adding 100 µl of acidified lysis buffer (0.01% HCl in 10% SDS). The plate was incubated at 42°C to ensure a homogenous mixture. The plate was shaken briefly and then read at 595 nm (test wavelength) and 690 nm (reference wavelength) using the Synergy™ 2 Multi-Mode Microplate Reader (BioTek instruments, Inc., Vermont, USA).

### Statistics

All experiments were repeated three to four times. All numerical data are expressed as mean±SEM (standard error of the mean) from a representative experiment performed in triplicate, and statistical analyses were assessed by Student's t test using Microsoft Excel XP.

### 3D-cufture inside Matrigel™

For the 3D Matrigel™ culture, monolayer grown cells were harvested by trypsinization, taken up in the complete medium, and separated into single-cell suspension by several passages through a Pasteur pipette. After this, 50 µl complete medium containing 5,000 cells was added to 150 µl of growth factor reduced Matrigel™ (Becton Dickinson, Bedford, MA) diluted 1:1 with complete media with 20% FBS. This was gently mixed, deposited onto Nunc 4-well slides precoated with the Matrigel, and incubated at 37°C until the Matrigel solidified. The cells were then covered with complete medium and grown at 37°C under 5% CO₂ for up to 24 days with a change of media every 2 days. Organoid formation was counted in randomly chosen fields with light microscopy.

### Western blot analysis

Western blot analysis was performed as described previously (Liu and Lobie, 2007) using the following antibodies: goat anti-artemin polyclonal antibody (R&D systems, Minneapolis, MN) and mouse anti-β-Actin monoclonal antibody (Sigma, St Louis, MO).

### Results

### Antagonism of artemin function reverses acquired tamoxifen resistance

To determine whether antagonism of artemin function reverses tamoxifen resistance in mammary carcinoma cells, a tamoxifen resistant MCF-7 cell line (designated TAMR) was developed. The growth of TAMS and TAMR cells was measured in estrogen free medium containing 10 nM E₂ or 1 µM tamoxifen over a period of 7 days. TAMS cells retained the response to E₂ and tamoxifen sensitivity. In contrast, TAMR cells exhibited loss of response to E₂ and of tamoxifen sensitivity (Figure 1A). Artemin expression in TAMS and TAMR cells was then compared. In the absence of tamoxifen, both mRNA and protein levels of artemin were increased in TAMR cells compared to TAMS cells. Tamoxifen markedly abrogated artemin expression in TAMS cells. However, TAMR cells retained elevated artemin expression at both mRNA and protein levels in response to tamoxifen treatment (Figure 1B).

To further determine the function of artemin in TAMR cells, the anchorage independent growth of TAMR cells treated with tamoxifen alone or in combination with anti-artemin IgY was measured by colony formation in soft agar assay. Figure 1C demonstrated that tamoxifen treatment did not affect colony formation of TAMR cells, thereby confirming the acquired tamoxifen resistance of these cells. Anti-artemin IgY alone suppressed the basal growth of TAMR cells by 14% (Figure 1C). However, combined treatment with anti-artemin antibody and tamoxifen resulted in reduction of colony formation by 28%, producing a similar effect to that observed in TAMS cells treated with tamoxifen alone.

### Discussion

It has been proposed that growth factor driven signalling pathways fundamentally contribute to the development of either *de novo* or acquired endocrine resistance (Arpino *et al.,* 2008; Massarweh and Schiff, 2006). However, the clinical relevance of growth factor signalling pathways in antiestrogen resistance has not been determined.

This work demonstrates for the first time that antagonists of artemin functionality can reverse antiestrogen resistance. The above results suggest, without wishing to be bound by any theory, that antagonists of artemin functionality resensitise cells to the inhibitory effects of anti-estrogenic agents, such as tamoxifen or fulvestrant, enabling cell cycle arrest and drug induced apoptosis.

In the TAMR cells described above, artemin expression was substantially increased and was not affected by tamoxifen treatment. The increase of artemin expression at the time of resistance suggests, again without wishing to be bound by any theory, that artemin signalling becomes reactivated, and may contribute to the growth of TAMR cells.

Notably, inhibition of artemin by treatment with anti-artemin antibody significantly enhanced the growth-inhibitory activity of antiestrogens in chemoresistant MCF-7 cells, restoring tamoxifen sensitivity in tamoxifen resistant cells. Thus, treatment with antagonists of artemin function increased the efficacy of antiestrogens in mammary carcinoma cells, restoring sensitivity to chemotherapy in resistant cells. Inhibition of artemin is considered a novel adjuvant therapeutic approach for the treatment of resistant mammary carcinoma. The present invention recognises that therapeutic targeting of artemin has utility in reversing antiestrogen resistance, which may in turn reduce mammary carcinoma associated mortality.

### EXAMPLE 2

### Materials and methods

### Cell culture

The mammary carcinoma cell line BT549 was obtained from American Type Culture Collection (Manassas, VA) and was cultured in RPMI-1640 supplemented with 10% FBS (FBS, Invitrogen) at 37°C in a humidified atmosphere with 5% CO₂.

The paclitaxel resistant cell line were grown continuously under selective pressure (40nM paclitaxel) for 4 days and then replaced with paclitaxel free culture medium for another 4 days. This was repeated for 4 cycles until resistance was established. Later, paclitaxel resistant cells were transferred to paclitaxel free culture medium for 5-7 days before experiments were performed.

### Cell functions assays

Cell viability, soft agar colony formation assay and 3D matrigel growth assays were performed as described above in Example 1. For the cell viability assay, 3000 cells were seeded into 100ul media(2%FBS) and every 24h viability was determined.

### Reagents

Alamar blue was purchased from Invitrogen. Taxol was from Sigma. Matrigel was purchased from BD Biosciences.

### Western Blot analysis

Western blot analysis was performed as described above in Example 1.

### Results

### Antagonism of artemin function reverses acquired paclitaxel resistance in mammary carcinoma cells

To determine whether antagonism of artemin function reverses paclitaxel resistance in mammary carcinoma cells, a paclitaxel resistant BT549 cell line (designated BT549-PTX Res) was developed. The resistant subline differs from the parental line in its sensitivity to paclitaxel by nearly 2 fold. BT549 PTX Res cells showed more scattered, elongated and mesenchymal cellular morphology in monolayer culture as compared to control BT549-wild type cells. When cultured in 3D matrigel, control BT549 wild type cells produced circumscribed colonies, whereas BT549-PTX Res cells exhibited a more disorganised structure as compared to the control cells and a large number of PTX Res cells spread from the main bulk of the colony (Figure 2A).

Western blot for ARTN protein expression in PTX Res and control wild type cells ± paclitaxel was performed to characterise ARTN expression in these mammary carcinoma cells. Increased expression of ARTN was observed in PTX Res cells compared to control wild type cells with or without the presence of paclitaxel. Furthermore, the presence of paclitaxel also increased ARTN expression in wild type cells as compared to wild type cells without paclitaxel (Figure 2B). Thus, ARTN expression is increased in paclitaxel resistance in mammary carcinoma cells.

Paclitaxel resistant cells exhibited increases in monolayer proliferation, colony formation in soft agar and 3D matrigel growth compared to the paclitaxel sensitive cells. Paclitaxel resistant cells also exhibited less sensitivity to paclitaxel in monolayer proliferation, colony formation in soft agar and 3D matrigel growth compared to the paclitaxel sensitive cells (Fig 2. C, D, E).

siRNA was employed to deplete ARTN in BT549 PTX Res and wild type cells, respectively. The siRNA targeting ARTN efficiently depleted ARTN expression in both cell pairs (Figure 2B), whereas transfection of scrambled siRNA (siCONT) had no effect on ARTN expression.

Depletion of ARTN reduced the basal capacity for monolayer proliferation, colony formation in soft agar, 3D matrigel growth of BT549 wild type cells. Depletion of ARTN also eliminated or largely abrogated the enhanced activity of paclitaxel resistant cells in monolayer proliferation, colony formation in soft agar, 3D matrigel growth of BT549 PTX Res cells both in the absence and presence of paclitaxel (Figure 2C, D and E). Combined depletion of ARTN and administration of paclitaxel further reduced the level of monolayer proliferation, colony formation in soft agar and 3D matrigel growth to that observed in paclitaxel sensitive cells treated with paclitaxel. Thus, functional antagonism of artermin, in this case by depletion of ARTN reversed acquired resistance to paclitaxel.

### EXAMPLE 3

### Materials and methods

### Cell culture

The MDA-MB-231 mammary carcinoma cell line was obtained from American Type Culture Collection (Manassas, VA). Both the MDA-MB-231 and the BT549 cell lines were cultured in RPMI-1640 supplemented with 10% FBS (FBS, Invitrogen) at 37°C in a humidified atmosphere with 5% CO₂.

The ionizing radiation (IR) resistant cell lines were exposed to IR (4Gy Co₆₀ γ-radiation) each day for 4 days, and then grown without radiation for another 4 days. This was repeated for 6 cycles until resistance was established. Later, IR resistant cells were cultured in radiation-free condition for 5-7 days before experiments were performed. The siRNA to ARTN used was as described above in Example 1.

Reagents, Western blot analysis, and cell function and viability assays were all done as described above in Example 2.

### Results

### Antagonism of artemin function reverses ionizing radiation resistance in mammary carcinoma cells

To determine whether antagonism of artemin function reverses resistance to ionizing radiation (IR) in mammary carcinoma cells, IR resistant MDA-MB-231 and BT549 cell lines (designated MDA-MB-231 IR Res, and BT549-IR Res, respectively) were developed.

The IR resistant sublines differed from the parental line in their sensitivity to IR by nearly 1.8-fold.

Western blot for ARTN protein expression in both pairs of IR Res and control wild type cells ± irradiation was performed to characterise ARTN expression in these mammary carcinoma cells. Increased expression of ARTN was observed in both pairs of IR Res cells as compared to respective wild type cells with irradiation (Figure 3A; left panel). Thus, ARTN expression is increased in IR resistance in mammary carcinoma cells.

IR resistant cells exhibited increases in monolayer proliferation, colony formation in soft agar and 3D matrigel growth compared to the IR sensitive cells. IR resistant cells also exhibited less sensitivity to IR in monolayer proliferation, colony formation in soft agar and 3D matrigel growth compared to the IR sensitive cells (Fig 3. B, C, D).

siRNA was then employed to deplete ARTN in BT549 and MDA-MB-231 IR resistant and wild type cells, respectively. The siRNA targeting of ARTN efficiently depleted ARTN expression in each cell pair (Figure 3A; right panel), whereas transfection of scrambeled siRNA (siCONT) had no effect on ARTN expression.

Depletion of ARTN reduced the basal capacity for monolayer proliferation, colony formation in soft agar, 3D matrigel growth of BT549 and MDA-MB-231-wild type (IR sensitive) cells. Depletion of ARTN also eliminated or largely abrogated the enhanced activity of IR resistant cells in monolayer proliferation, colony formation in soft agar, 3D matrigel growth both in the absence and presence of treatment with IR (Figure 3, B, C ,D). Combined depletion of ARTN and treatment with IR further reduced the level of monolayer proliferation, colony formation in soft agar, and 3D matrigel growth to that observed in IR sensitive cells treated with IR. Thus, functional antagonism of artemin, in this case by depletion of ARTN, reversed aquired resistance to IR.

### REFERENCES

Abie Pro 3.0: Peptide Antibody Design (hypertext transfer protocol://world wide web.changbioscience.com/abie/abie.html
Aguilar RC, Retegui LA, Roguin LP Int J Biomed Comput. 1994 Nov-Dec;37(3):225-35
Alix AJ. : Vaccine. 1999 Sep;18(3-4):311-4
Arpino G, Wiechmann L, Osborne CK, Schiff R (2008). Crosstalk between the estrogen receptor and the HER tyrosine kinase receptor family: Molecular mechanism and clinical implications for endocrine therapy resistance. Endocrine Reviews 29: 217-233
Ausubel, F. M. et al. (1989) Current Protocols in Molecular Biology, John Wiley & Sons, New York, NY
Baca, M., L. G. Presta, S. J. O'Connor, and J. A. Wells. 1997. Antibody humanization using monovalent phage display. J. Biol. Chem. 272:10678-10684
Bean, Eric S (2001) Polyclonal Antibodies. In: Basic Methods in Antibody Production and Characterization antibodies
Bird et al. (1988) Science 242:423-426
Boesen et al., 1994, Biotherapy 6:291-302
Bolton and McCarthy, 1962, PNAS 84:1390
Bowie et al., 1990, Science 247, 1306
Chothia & Lesk J. Mol. Biol. 196:901-917, 1987
Chothia et al. Nature 342:878-883, 1989
Doerr, M. E. And J. I. Jones. 1996. The roles of integrins and extracellular matrix proteins in the insulin-like growth factor I-stimulated chemotaxis of human breast cancer cells. J. Biol. Chem. 271:2443-2447
Donnelly JJ, Ulmer JB, Liu MA. DNA vaccines. Life Sci 1997;60:163-172
Fidler, I. J. (1973) Nat. New Biol. 242, 148-149
Folkman, J. And Moscona, A. (1978). Role of cell shape in growth control. Nature 278, 345-349 ftp://ftp.ncbi.nih.gov/blast/
Garber, 2001
Gennaro AR: Remington: The Science and Practice of Pharmacy, 20th ed., Lippincott, Williams & Wilkins, 2000
Ghetie V, Vitetta E. Immunotoxins in the therapy of cancer: from bench to clinic. Pharmacol Ther 1994;63:209-234
Giesen et al., Nucleic Acids Res. 1998 Nov 1;26(21):5004-6
Green, L. L., et al. 1994. Antigen-specific human monoclonal antibodies from mice engineered with human Ig heavy and light chain YACs. Nat. Gen. 7:13-21
Herlyn DM, et al., Inhibition of growth of colorectal carcinoma in nude mice by monoclonal antibody. Cancer Res 1980;40:717-721
Holliger, P., et al. (1993) Proc. Natl. Acad. Sci. USA 90:6444-6448
Hoogenboom, H. R., A. P. de Bruine, S. E. Hufton, R. M. Hoet, J. W. Arends, and R. C. Roovers. 1998. Antibody phage display technology and its applications. Immunotechnology. 4:1-20
Hopp TP, Woods KR. Prediction of protein antigenic determinants from amino acid sequences. Proc Natl Acad Sci U S A. 1981 Jun;78(6):3824-8
Howard, G, and Bethel D. (ed.), CRC Press, 5:21-50, 2000) http:/www.ebi.ac.uk/emboss/align/
Huang, X. (1994) On Global Sequence Alignment. Computer Applications in the Biosciences 10, 227-235
Hunkapiller, M.W.; Hewick, R.M.; Dreyer, W.J. and Hood, L.E.. 1983. High-Sequencing with a Gas-Phase Sequenator. Methods Enzymol. 91: 399
Huston et al. (1988) Proc. Natl. Acad. Sci. USA 85:5879-5883
Jakobovits, A. 1995. Production of fully human antibodies by transgenic mice. Curr. Opin. Biotechnol. 6:561-566
Jespers, L. S., A. Roberts, S. M. Mahler, G. Winter, and H. R. Hoogenboom. 1994. Guiding the selection of human antibodies from phage display repertoires to a single epitope of an antigen. Biotechnology (NY) 12:899-903
Jones PT, Dear PH, Foote J, Neuberger MS, Winter G. 1986. Replacing the complementarity determining regions in a human antibody with those from a mouse. Nature 321:522-25
Kabat Sequences of Proteins of Immunological Interest, National Institutes of Health, Bethesda, MD, 1987 and 1991
Kerbel R. S. What is the optimal rodent model for anti-tumor drug testing? Cancer Metastasis Rev., 17: 301-304, 1998
Killion J. J., Radinsky R., Fidler I. J. Orthotopic models are necessary to predict therapy of transplantable tumours in mice. Cancer Metastasis Rev., 17: 279-284, 1998
Kimmel, A. R. (1987; Methods Enzymol. 152:507-511
Koller and Smithies, 1989, Proc. Natl. Acad. Sci. USA 86:8932-8935
Kozarsky and Wilson, 1993, Current Opinion in Genetics and Development 3:499-503
Kyte J, Doolittle RF. A simple method for displaying the hydropathic character of a protein. J Mol Biol. 1982 May 5;157(1):105-32
Langer et al., 1981, J. Biomed. Mater. Res.: 15: 267), or poly-D-(-)-3-hydroxybutyric acid (EP 133,988)
Liu DX, Lobie PE (2007). Transcriptional activation of p53 by Pitx1. Cell Death and Differentiation 14: 1893-1907
Massarweh S, Schiff R (2006). Resistance to endocrine therapy in breast cancer: Exploiting estrogen receptor/growth factor signaling crosstalk. Endocrine-Related Cancer 13
Mastrangeli et al., 1993, J. Clin. Invest. 91:225-234
Maynard, J., and Georgiou, G. 2000. Antibody engineering. Annu Rev Biomed Eng 2: 339-376
Mendez, M. J., L. L. et al. 1997. Functional transplant of megabase human immunoglobulin loci recapitulates human antibody response in mice. Nat. Gen. 15:146-156
Mesri, E A et al. J Clin Microbiol. 1990 June; 28(6): 1219-1224
Miller et al., 1993, Meth. Enzymol. 217:581-599
Miller RA, Maloney DG, Warnke R, Levy R. Treatment of B-cell lymphoma with monoclonal anti-idiotype antibody. N Engl J Med 1982;306:517-522
Needleman, S. B. And Wunsch, C. D. (1970) J. Mol. Biol. 48, 443-453
Nielsen et al., Science. 1991 Dec 6;254(5037):1497-500
Paul, W., ea., 2nd ed. Raven Press, N.Y. (1989)
Pedersen, J. T., A. H. Henry, S. J. Searle, B. C. Guild, M. Roguska, and A. R. Rees. 1994. Comparison of surface accessible residues in human and murine immunoglobulin Fv domains. Implication for humanization of murine antibodies. J. Mol. Biol. 235:959-973
Poljak, R. J., et al. (1994) Structure 2:1121-1123
Price J. E. The biology of cancer metastasis. Prog. Clin. Biol. Res., 354A: 237-255, 1990
Price J. E. Analyzing the metastatic phenotype. J. Cell. Biochem., 56: 16-22, 1994
Rader, C. And C. F. Barbas, III. 1997. Phage display of combinatorial antibody libraries. Curr. Opin. Biotechnol. 8:503-508
Rader, C., D. A. Cheresh, and C. F. Barbas, III. 1998. A phage display approach for rapid antibody humanization: designed combinatorial V gene libraries. Proc. Natl. Acad. Sci. U. S. A 95:8910-8915 Reichmann, L., M. Clark, H. Waldman, and G. Winter. 1998. Reshaping human antibodies for therapy. Nature 332L323-327
Reisfeld RA, Gillies SD. Recombinant antibody fusion proteins for cancer immunotherapy. Curr Top Microbiol Immunol 1996;213:27-53
Remington's Pharmaceutical Science 16th edition, Osol, A. Ed. 1980
Rice, P. Longden, I. And Bleasby, A. EMBOSS: The European Molecular Biology Open Software Suite, Trends in Genetics June 2000, vol 16, No 6. pp.276-277
Riethmüller G, et al., Monoclonal antibody therapy for resected Dukes' C colorectal cancer: seven-year outcome of a multicenter randomized trial. J Clin Oncol 1998;16:1788-1794
Riethmüller G, et al., Randomized trial of monoclonal antibody for adjuvant therapy of resected Dukes' C colorectal carcinoma. German Cancer Aid 17-1A Study Group. Lancet 1994;343:1177-1183
Rosenblad C, Gronborg M, Hansen C, Blom N, Meyer M, Johansen J et al. (2000). In vivo protection of nigral dopamine neurons by lentiviral gene transfer of the novel GDNF-family member neublastin/artemin. Mol Cell Neurosci 15: 199-214. Erratum in: Mol Cell Neurosci 2001 Sep;18(3):332-3.
Rosenblum MD, Shivers RR (2000). 'Rings' of F-actin form around the nucleus in cultured human MCF7 adenocarcinoma cells upon exposure to both taxol and taxotere. Comp Biochem Physiol C Toxicol Pharmacol 125: 121-31
Rosenfeld et al., 1991, Science 252:431-434
Rosenfeld et al., 1992, Cell 68:143-155
Rosok, M. J., D. E. Yelton, L. J. Harris, J. Bajorath, K. E. Hellstrom, I. Hellstrom, G. A. Cruz, K. Kristensson, H. Lin, W. D. Huse, and S. M. Glaser. 1996. A combinatorial library strategy for the rapid humanization of anticarcinoma BR96 Fab. J. Biol. Chem. 271:22611-22618
Russell ND et al., Production of protective human antipneumococcal antibodies by transgenic mice with human immunoglobulin loci. Infect Immun. 2000 Apr;68(4):1820-6
Sambrook, J. et al. (1989) Molecular Cloning, A Laboratory Manual, Cold Spring Harbor Press, Plainview, NY
Santin AD, Bellone S, Van Stedum S, Bushen W, Palmieri M, Siegel ER et al (2005). Amplification of c-erbB2 oncogene: a major prognostic indicator in uterine serous papillary carcinoma. Cancer 104: 1391-7
Sarkar, G. (1993) PCR Methods Applic. 2:318-322
Schook, Lawrence B eds., Monocolonal Antibody Production Techniques and Applications, Marcel Dekker Inc., New York, 1987
Shaw LE, Sadler AJ, Pugazhendhi D, Darbre PD (2006). Changes in oestrogen receptor-Î± and -Î2 during progression to acquired resistance to tamoxifen and fulvestrant (Faslodex, ICI 182,780) in MCF7 human breast cancer cells. Journal of Steroid Biochemistry and Molecular Biology 99: 19-32 Shurin MR. Dendritic cells presenting tumor antigen. Cancer Immunol Immunother 1996;43:158-164 Sidman et al., 1983, Biopolymers: 22: 547-56
Siegel (2002) Siegel DL, Recombinant monoclonal antibody technology, Transfus Clin Biol, 9(1):15-22 2002
Smith CA, et al. Adoptive immunotherapy for Epstein-Barr virus-related lymphoma. Leuk Lymphoma 1996;23:213-220
Stewart, Sandy J (2001) Monoclonal Antibody Production. In: Basic Methods in Antibody Production and Characterization antibodies
Syrengelas AD, Chen TT, Levy R. DNA immunization induces protective immunity against B-cell lymphoma. Nat Med 1996;2:1038-1041
Tatiana A. Tatusova, Thomas L. Madden (1999), "Blast 2 sequences - a new tool for comparing protein and nucleotide sequences", FEMS Microbiol Lett. 174:247-250
Thomadaki and Scorilas, 2006
Todorovska et al. Design and application of diabodies, triabodies, and tetrabodies for cancer targeting. J. Immunol. Methods. 2001 Feb 1;248(1-2):47-66
Toes RE et al. Peptide vaccination can lead to enhanced tumor growth through specific T-cell tolerance induction. Proc Natl Acad Sci USA 1996
Tomlinson I and Holliger P, Methods for generating multivalent and bispecific antibody fragments, Methods Enzymol, 326, 461-479, 2000
Wahl, G. M. And S. L. Berger (1987; Methods Enzymol. 152:399-407
Walsh et al., 1993, Proc. Soc. Exp. Biol. Med. 204:289-300
Wang et al, J. Immunological Methods 241: 171-184, 2000
Wang, et al., 1995, Gene Therapy 2:775-783
Ward et al., (1989) Nature 341:544-546
Welschof, M., C. Christ, I. Hermes, A. Keller, C. Kleist, and M. Braunagel. 2003. Generation and screening of a modular human scFv expression library from multiple donors. Methods Mol. Biol. 207:103-121
Wu and Wu, 1987, J. Biol. Chem. 262:4429-4432
Zijlstra et al., 1989, Nature 342:435-438

## Claims

1. An antagonist of artemin function for use in reversing, wholly or in part, the resistance of a breast cancer-burdened patient to a cancer therapeutic, wherein the antagonist of artemin function is an antisense polynucleotide, an RNAi polynucleotide, or an antibody capable of inhibiting artemin functionality.

2. The antagonist for use according to claim 1, wherein the antibody is a monoclonal antibody (mAb).

3. The antagonist for use according to claim 1 or 2, wherein the cancer therapeutic to which resistance has been induced or mediated is a chemotherapeutic.

4. The antagonist for use according to claim 3, wherein the chemotherapeutic to which said tumours are resistant is an estrogen receptor antagonist or an aromatase inhibitor.

5. The antagonist for use according to claim 4, wherein the estrogen receptor antagonist is tamoxifen, toremifene, idoxifene, arzoxifene, raloxifene, or fulvestrant, or the aromatase inhibitor is formestane, anastrozole, letrozole, vorozole, or exemestane.

6. The antagonist for use according to claim 1, wherein the cancer therapeutic to which said tumours are resistant is radiation therapy.

7. An antagonist of artemin function for use in pre-treating a tumour-burdened patient prior to administration to the patient of an amount of a cancer therapeutic effective to induce an anti-tumour effect in said patient, wherein the antagonist of artemin function is an antisense polynucleotide, an RNAi polynucleotide, or an antibody capable of inhibiting artemin functionality.

8. An antagonist of artemin function for use according to claim 1, wherein said reversing resistance comprises resensitising to treatment one or more breast cancer cells that are resistant to treatment with a cancer therapeutic.

9. A composition for use in reversing resistance to treatment with a cancer therapeutic in a cancer cell, the composition comprising an antagonist of artemin function, wherein the antagonist of artemin function is an antisense polynucleotide, an RNAi polynucleotide, or an antibody capable of inhibiting artemin functionality.

10. The composition for use according to claim 9 additionally comprising a therapeutic agent, wherein the composition is for use in inhibiting one or more breast cancer cells that are resistant to treatment with a cancer therapeutic.

11. The composition for use according to claim 10, wherein the inhibition is selected from the group consisting of: inhibition of growth, including inhibition of anchorage-independent growth, inhibition of cell survival, inhibition of proliferation, inhibition of mitosis, inhibition of cell-cycle progression, inhibition of metastasis, inhibition of cell motility, and induction of apoptosis.

12. A method of determining or monitoring resistance to a therapeutic agent in one or more cancer cells *in vitro,* the method comprising contacting one or more cancer cells with an antagonist of artemin function and the therapeutic agent, and measuring inhibition of the one or more cancer cells, wherein the antagonist of artemin function is an antisense polynucleotide, an RNAi polynucleotide, or an antibody capable of inhibiting artemin functionality.

13. A method of determining the presence or extent of BCL2-mediated resistance in cancer cells exhibiting resistance or of distinguishing in one or more cancer cells BCL2-mediated resistance to a cancer therapy from non-BCL2-mediated resistance to the cancer therapy, the method comprising administration to one or more cancer cells *in vitro* of an effective amount of an antagonist of artemin function and a cancer therapeutic to which the cancer cells are resistant and measuring one or more of cancer cell survival, cancer cell motility, cancer cell proliferation, cancer cell mitosis, cell-cycle progression, cancer cell metastasis, or cancer cell apoptosis, wherein the antagonist of artemin function is an antisense polynucleotide, an RNAi polynucleotide, or an antibody capable of inhibiting artemin functionality.

## Patentansprüche

1. Antagonist der Arteminfunktion zur Verwendung im Umkehren, vollständig oder teilweise, der Resistenz eines brustkrebsbetroffenen Patienten gegenüber einem Krebstherapeutikum, wobei der Antagonist der Arteminfunktion eine Antisense-Polynukleotid, ein RNAi-Polynukleotid oder ein Antikörper ist, die in der Lage sind, die Arteminfunktionalität zu inhibieren.

2. Antagonist zur Verwendung nach Anspruch 1, wobei der Antikörper ein monoklonaler Antikörper (mAb) ist.

3. Antagonist zur Verwendung nach Anspruch 1 oder 2, wobei das Krebstherapeutikum, wogegen eine Resistenz induziert oder vermittelt wurde, ein Chemotherapeutikum ist.

4. Antagonist zur Verwendung nach Anspruch 3, wobei das Chemotherapeutikum, wogegen die Tumore resistent sind, ein Östrogenrezeptorantagonist oder ein Aromataseinhibitor ist.

5. Antagonist zur Verwendung nach Anspruch 4, wobei der Östrogenrezeptor-antagonist Tamoxifen, Toremifen, Idoxifen, Arzoxifen, Raloxifen oder Fulvestrant ist oder der Aromataseinhibitor Formestan, Anastrozol, Letrozol, Vorozol oder Exemestan ist.

6. Antagonist zur Verwendung nach Anspruch 1, wobei das Krebstherapeutikum, wogegen die Tumore resistent sind, eine Bestrahlungstherapie ist.

7. Antagonist der Arteminfunktion zur Verwendung im Vorbehandeln eines tumorbetroffenen Patienten vor der Verabreichung an den Patienten einer Menge eines Krebstherapeutikums, welche wirksam eine Antitumorwirkung in dem Patienten induzieren kann, wobei der Antagonist der Arteminfunktion ein Antisense-Polynukleotid, ein RNAi-Polynukleotid oder ein Antikörper ist, die in der Lage sind, die Arteminfunktionalität zu inhibieren.

8. Antagonist der Arteminfunktion zur Verwendung nach Anspruch 1, wobei das Umkehren der Resistenz eine Resensibilisierung für eine Behandlung von einer oder mehreren Brustkrebszellen umfasst, die gegenüber einer Behandlung mit einem Krebstherapeutikum resistent sind.

9. Zusammensetzung zur Verwendung im Umkehren einer Resistenz gegenüber einer Behandlung mit einem Krebstherapeutikum in einer Krebszelle, wobei die Zusammensetzung einen Antagonisten der Arteminfunktion umfasst, wobei der Antagonist der Arteminfunktion ein Antisense-Polynukleotid, ein RNAi-Polynukleotid oder ein Antikörper ist, die in der Lage sind, die Arteminfunktionalität zu inhibieren.

10. Zusammensetzung zur Verwendung nach Anspruch 9, weiterhin umfassend ein Therapeutikum, wobei die Zusammensetzung zur Verwendung im Inhibieren von einer oder mehreren Brustkrebszellen ist, die gegenüber einer Behandlung mit einem Krebstherapeutikum resistent sind.

11. Zusammensetzung zur Verwendung nach Anspruch 10, wobei die Inhibierung ausgewählt ist aus der Gruppe bestehend aus: einer Inhibierung von Wachstum, einschließlich einer Inhibierung von verankerungsunabhängigem Wachstum, einer Inhibierung von Zellüberleben, einer Inhibierung von Proliferation, einer Inhibierung von Mitose, einer Inhibierung von Zellzyklusprogression, einer Inhibierung von Metastasierung, einer Inhibierung von Zellmotilität und einer Induktion von Apoptose.

12. Verfahren zum Bestimmen oder Überwachen von Resistenz gegenüber einem Therapeutikum in einer oder mehreren Krebszellen *in vitro,* das Verfahren umfassend ein Kontaktieren von einer oder mehreren Krebszellen mit einem Antagonisten der Arteminfunktion und dem Therapeutikum und Bestimmen der Inhibierung der einen oder mehreren Krebszellen umfasst, wobei der Antagonist der Arteminfunktion ein Antisense-Polynukleotid, ein RNAi-Polynukleotid oder ein Antikörper ist, die in der Lage sind, die Arteminfunktionalität zu inhibieren.

13. Verfahren zum Bestimmen des Vorliegens oder Ausmaßes einer BCL-2-vermittelten Resistenz in Krebszellen, die eine Resistenz aufweisen, oder zum Unterscheiden in einer oder mehreren Krebszellen zwischen einer BCL2-vermittelten Resistenz gegenüber einer Krebstherapie und einer nicht-BCL2-vermittelten Resistenz gegenüber einer Krebstherapie, das Verfahren umfassend ein Verabreichen an eine oder mehrere Krebszellen *in vitro* einer wirksamen Menge eines Antagonisten der Arteminfunktion und eines Krebstherapeutikums, gegenüber welchem die Krebszellen resistent sind, und Bestimmen von einem oder mehreren eines Zellüberlebens, einer Krebszellmotilität, einer Krebszellproliferation, einer Krebszellmitose, einer Zellzyklusprogression, einer Krebstzellmetastasierung oder einer Krebszellapoptose, wobei der Antagonist der Arteminfunktion ein Antisense-Polynukleotid, ein RNAi-Polynukleotid oder ein Antikörper ist, die in der Lage sind, die Arteminfunktionalität zu inhibieren.

## Revendications

1. Antagoniste de la fonction d'artémine à utiliser dans l'inversion, en intégralité ou en partie, de la résistance d'un patient accablé d'un cancer du sein à une thérapie contre le cancer, dans lequel l'antagoniste de la fonction d'artémine est un polynucléotide anti-sens, un polynucléotide ARNi, ou un anticorps capable d'inhiber la fonctionnalité d'artémine.

2. Antagoniste à utiliser selon la revendication 1, dans lequel l'anticorps est un anticorps monoclonal (mAb).

3. Antagoniste à utiliser selon la revendication 1 ou 2, dans lequel la thérapie contre le cancer à laquelle une résistance a été induite ou médiée est un agent chimiothérapeutique.

4. Antagoniste à utiliser selon la revendication 3, dans lequel l'agent chimiothérapeutique auquel lesdites tumeurs sont résistantes est un antagoniste de récepteur d'oestrogène ou un inhibiteur d'aromatase.

5. Antagoniste à utiliser selon la revendication 4, dans lequel l'antagoniste de récepteur d'oestrogène est le tamoxifène, le torémifène, l'idoxifène, l'arzoxifène, le raloxifène, ou le fulvestrant, et l'inhibiteur d'aromatase est le formestane, l'anastrozole, le létrozole, le vorozole, ou l'exémestane.

6. Antagoniste à utiliser selon la revendication 1, dans lequel la thérapie contre le cancer à laquelle lesdites tumeurs sont résistantes est la radiothérapie.

7. Antagoniste de la fonction d'artémine à utiliser dans le prétraitement d'un patient accablé d'une tumeur avant administration au patient d'une quantité d'une thérapie contre le cancer efficace pour induire un effet anti-tumoral chez ledit patient, dans lequel l'antagoniste de la fonction d'artémine est un polynucléotide anti-sens, un polynucléotide ARNi, ou un anticorps capable d'inhiber la fonctionnalité d'artémine.

8. Antagoniste de la fonction d'artémine à utiliser selon la revendication 1, dans lequel ladite inversion de résistance comprend la resensibilisation au traitement d'une ou plusieurs cellules du cancer du sein qui sont résistantes à un traitement avec une thérapie contre le cancer.

9. Composition à utiliser dans l'inversion d'une résistance au traitement avec une thérapie contre le cancer dans une cellule cancéreuse, la composition comprenant un antagoniste de la fonction d'artémine, dans laquelle l'antagoniste de la fonction d'artémine est un polynucléotide anti-sens, un polynucléotide ARNi, ou un anticorps capable d'inhiber la fonctionnalité d'artémine.

10. Composition à utiliser selon la revendication 9, comprenant de surcroît un agent thérapeutique, dans laquelle la composition est destinée à être utilisée dans l'inhibition d'une ou plusieurs cellules du cancer du sein qui sont résistantes à un traitement avec une thérapie contre le cancer.

11. Composition à utiliser selon la revendication 10, dans laquelle l'inhibition est choisie dans le groupe consistant en : l'inhibition de la croissance, dont l'inhibition de la croissance indépendante de l'ancrage, l'inhibition de la survie cellulaire, l'inhibition de la prolifération, l'inhibition de la mitose, l'inhibition de la progression du cycle cellulaire, l'inhibition de la métastase, l'inhibition de la motilité cellulaire, et l'induction d'apoptose.

12. Procédé de détermination ou de contrôle de la résistance à un agent thérapeutique dans une ou plusieurs cellules cancéreuses *in vitro,* le procédé comprenant la mise en contact d'une ou plusieurs cellules cancéreuses avec un antagoniste de la fonction d'artémine et l'agent thérapeutique, et la mesure de l'inhibition des une ou plusieurs cellules cancéreuses, dans lequel l'antagoniste de la fonction d'artémine est un polynucléotide anti-sens, un polynucléotide ARNi, ou un anticorps capable d'inhiber la fonctionnalité d'artémine.

13. Procédé de détermination de la présence ou de l'ampleur d'une résistance médiée par BCL2 dans des cellules cancéreuses affichant une résistance ou de distinction dans une ou plusieurs cellules cancéreuses d'une résistance médiée par BCL2 à une thérapie contre le cancer d'une résistance non médiée par BCL2 à la thérapie contre le cancer, le procédé comprenant l'administration à une ou plusieurs cellules cancéreuses *in vitro* d'une quantité efficace d'un antagoniste de la fonction d'artémine et d'une thérapie contre le cancer à laquelle les cellules cancéreuses sont résistantes et la mesure d'un ou plusieurs éléments parmi la survie de cellules cancéreuses, la motilité de cellules cancéreuses, la prolifération de cellules cancéreuses, la mitose de cellules cancéreuses, la progression du cycle cellulaire, la métastase de cellules cancéreuses, ou l'apoptose de cellules cancéreuses, dans lequel l'antagoniste de la fonction d'artémine est un polynucléotide anti-sens, un polynucléotide ARNi, ou un anticorps capable d'inhiber la fonctionnalité d'artémine.


### SEQUENCE LISTING

<110> Auckland Uniservices Ltd
<120> ANTI-NEOPLASTIC USES OF ARTEMIN ANTAGONISTS
<130> CSC/FP6827349
<150> EP 10823668.8
   <151> 2010-10-15
<140> PCT/NZ2010/000207
   <141> 2010-10-15
<140> US 61/252513
   <141> 2009-10-16
<160> 44
<170> PatentIn version 3.5
<210> 1
   <211> 220
   <212> PRT
   <213> Homo sapiens
<220>
   <221> misc_feature
   <223> Artemin isofom 1 precursor
<400> 1
   Met Glu Leu Gly Leu Gly Gly Leu Ser Thr Leu Ser His Cys Pro Trp
   1               5                   10                  15
   Pro Arg Arg Gln Pro Ala Leu Trp Pro Thr Leu Ala Ala Leu Ala Leu
               20                  25                  30
   Leu Ser Ser Val Ala Glu Ala Ser Leu Gly Ser Ala Pro Arg Ser Pro
           35                  40                  45
   Ala Pro Arg Glu Gly Pro Pro Pro Val Leu Ala Ser Pro Ala Gly His
       50                  55                  60
   Leu Pro Gly Gly Arg Thr Ala Arg Trp Cys Ser Gly Arg Ala Arg Arg
   65                  70                  75                  80
   Pro Pro Pro Gln Pro Ser Arg Pro Ala Pro Pro Pro Pro Ala Pro Pro
                   85                  90                  95
   Ser Ala Leu Pro Arg Gly Gly Arg Ala Ala Arg Ala Gly Gly Pro Gly
               100                 105                 110
   Ser Arg Ala Arg Ala Ala Gly Ala Arg Gly Cys Arg Leu Arg Ser Gln
           115                 120                 125
   Leu Val Pro Val Arg Ala Leu Gly Leu Gly His Arg Ser Asp Glu Leu
       130                 135                 140
   Val Arg Phe Arg Phe Cys Ser Gly Ser Cys Arg Arg Ala Arg Ser Pro
   145                 150                 155                 160
   His Asp Leu Ser Leu Ala Ser Leu Leu Gly Ala Gly Ala Leu Arg Pro
                   165                 170                 175
   Pro Pro Gly Ser Arg Pro Val Ser Gln Pro Cys Cys Arg Pro Thr Arg
               180                 185                 190
   Tyr Glu Ala Val Ser Phe Met Asp Val Asn Ser Thr Trp Arg Thr Val
           195                 200                 205
   Asp Arg Leu Ser Ala Thr Ala Cys Gly Cys Leu Gly
       210                 215                 220
<210> 2
   <211> 237
   <212> PRT
   <213> Homo sapiens
<220>
   <221> misc_feature
   <223> Artemin isoform 2 precursor
<400> 2
   Met Pro Gly Leu Ile Ser Ala Arg Gly Gln Pro Leu Leu Glu Val Leu
   1               5                   10                  15
   Pro Pro Gln Ala His Leu Gly Ala Leu Phe Leu Pro Glu Ala Pro Leu
               20                  25                  30
   Gly Leu Ser Ala Gln Pro Ala Leu Trp Pro Thr Leu Ala Ala Leu Ala
           35                  40                  45
   Leu Leu Ser Ser Val Ala Glu Ala Ser Leu Gly Ser Ala Pro Arg Ser
       50                  55                  60
   Pro Ala Pro Arg Glu Gly Pro Pro Pro Val Leu Ala Ser Pro Ala Gly
   65                  70                  75                  80
   His Leu Pro Gly Gly Arg Thr Ala Arg Trp Cys Ser Gly Arg Ala Arg
                   85                  90                  95
   Arg Pro Pro Pro Gln Pro Ser Arg Pro Ala Pro Pro Pro Pro Ala Pro
               100                 105                 110
   Pro Ser Ala Leu Pro Arg Gly Gly Arg Ala Ala Arg Ala Gly Gly Pro
               115                 120                 125
   Gly Ser Arg Ala Arg Ala Ala Gly Ala Arg Gly Cys Arg Leu Arg Ser
       130                 135                 140
   Gln Leu Val Pro Val Arg Ala Leu Gly Leu Gly His Arg Ser Asp Glu
   145                 150                 155                 160
   Leu Val Arg Phe Arg Phe Cys Ser Gly Ser Cys Arg Arg Ala Arg Ser
                   165                 170                 175
   Pro His Asp Leu Ser Leu Ala Ser Leu Leu Gly Ala Gly Ala Leu Arg
               180                 185                 190
   Pro Pro Pro Gly Ser Arg Pro Val Ser Gln Pro Cys Cys Arg Pro Thr
           195                 200                 205
   Arg Tyr Glu Ala Val Ser Phe Met Asp Val Asn Ser Thr Trp Arg Thr
       210                 215                 220
   Val Asp Arg Leu Ser Ala Thr Ala Cys Gly Cys Leu Gly
   225                 230                 235
<210> 3
   <211> 228
   <212> PRT
   <213> Homo sapiens
<220>
   <221> misc_feature
   <223> Artemin isoform 3 precursor
<400> 3
   Met Glu Leu Gly Leu Gly Gly Leu Ser Thr Leu Ser His Cys Pro Trp
   1               5                   10                  15
   Pro Arg Arg Gln Ala Pro Leu Gly Leu Ser Ala Gln Pro Ala Leu Trp
               20                  25                  30
   Pro Thr Leu Ala Ala Leu Ala Leu Leu Ser Ser Val Ala Glu Ala Ser
           35                  40                  45
   Leu Gly Ser Ala Pro Arg Ser Pro Ala Pro Arg Glu Gly Pro Pro Pro
       50                  55                  60
   Val Leu Ala Ser Pro Ala Gly His Leu Pro Gly Gly Arg Thr Ala Arg
   65                  70                  75                  80
   Trp Cys Ser Gly Arg Ala Arg Arg Pro Pro Pro Gln Pro Ser Arg Pro
                   85                  90                  95
   Ala Pro Pro Pro Pro Ala Pro Pro Ser Ala Leu Pro Arg Gly Gly Arg
               100                 105                 110
   Ala Ala Arg Ala Gly Gly Pro Gly Ser Arg Ala Arg Ala Ala Gly Ala
           115                 120                 125
   Arg Gly Cys Arg Leu Arg Ser Gln Leu Val Pro Val Arg Ala Leu Gly
       130                 135                 140
   Leu Gly His Arg Ser Asp Glu Leu Val Arg Phe Arg Phe Cys Ser Gly
   145                 150                 155                 160
   Ser Cys Arg Arg Ala Arg Ser Pro His Asp Leu Ser Leu Ala Ser Leu
                   165                 170                 175
   Leu Gly Ala Gly Ala Leu Arg Pro Pro Pro Gly Ser Arg Pro Val Ser
               180                 185                 190
   Gln Pro Cys Cys Arg Pro Thr Arg Tyr Glu Ala Val Ser Phe Met Asp
           195                 200                 205
   Val Asn Ser Thr Trp Arg Thr Val Asp Arg Leu Ser Ala Thr Ala Cys
       210                 215                 220
   Gly Cys Leu Gly
   225
<210> 4
   <211> 113
   <212> PRT
   <213> Homo sapiens
<220>
   <221> misc_feature
   <223> 113 amino acid form of mature artemin
<400> 4
   Ala Gly Gly Pro Gly Ser Arg Ala Arg Ala Ala Gly Ala Arg Gly Cys
   1               5                   10                  15
   Arg Leu Arg Ser Gln Leu Val Pro Val Arg Ala Leu Gly Leu Gly His
               20                  25                  30
   Arg Ser Asp Glu Leu Val Arg Phe Arg Phe Cys Ser Gly Ser Cys Arg
           35                  40                  45
   Arg Ala Arg Ser Pro His Asp Leu Ser Leu Ala Ser Leu Leu Gly Ala
       50                  55                  60
   Gly Ala Leu Arg Pro Pro Pro Gly Ser Arg Pro Val Ser Gln Pro Cys
   65                  70                  75                  80
   Cys Arg Pro Thr Arg Tyr Glu Ala Val Ser Phe Met Asp Val Asn Ser
                   85                  90                  95
   Thr Trp Arg Thr Val Asp Arg Leu Ser Ala Thr Ala Cys Gly Cys Leu
               100                 105                 110
   Gly
<210> 5
   <211> 116
   <212> PRT
   <213> Homo sapiens
<220>
   <221> misc_feature
   <223> 116 amino acid form of mature artemin
<400> 5
   Ala Ala Arg Ala Gly Gly Pro Gly Ser Arg Ala Arg Ala Ala Gly Ala
   1               5                   10                  15
   Arg Gly Cys Arg Leu Arg Ser Gln Leu Val Pro Val Arg Ala Leu Gly
               20                  25                  30
   Leu Gly His Arg Ser Asp Glu Leu Val Arg Phe Arg Phe Cys Ser Gly
           35                  40                  45
   Ser Cys Arg Arg Ala Arg Ser Pro His Asp Leu Ser Leu Ala Ser Leu
       50                  55                  60
   Leu Gly Ala Gly Ala Leu Arg Pro Pro Pro Gly Ser Arg Pro Val Ser
   65                  70                  75                  80
   Gln Pro Cys Cys Arg Pro Thr Arg Tyr Glu Ala Val Ser Phe Met Asp
                   85                  90                  95
   Val Asn Ser Thr Trp Arg Thr Val Asp Arg Leu Ser Ala Thr Ala Cys
               100                 105                 110
   Gly Cys Leu Gly
           115
<210> 6
   <211> 140
   <212> PRT
   <213> Homo sapiens
<220>
   <221> misc_feature
   <223> 140 amino acid form of mature artemin
<400> 6
   Pro Pro Pro Gln Pro Ser Arg Pro Ala Pro Pro Pro Pro Ala Pro Pro
   1               5                   10                  15
   Ser Ala Leu Pro Arg Gly Gly Arg Ala Ala Arg Ala Gly Gly Pro Gly
               20                  25                  30
   Ser Arg Ala Arg Ala Ala Gly Ala Arg Gly Cys Arg Leu Arg Ser Gln
           35                  40                  45
   Leu Val Pro Val Arg Ala Leu Gly Leu Gly His Arg Ser Asp Glu Leu
       50                  55                  60
   Val Arg Phe Arg Phe Cys Ser Gly Ser Cys Arg Arg Ala Arg Ser Pro
   65                  70                  75                  80
   His Asp Leu Ser Leu Ala Ser Leu Leu Gly Ala Gly Ala Leu Arg Pro
                   85                  90                  95
   Pro Pro Gly Ser Arg Pro Val Ser Gln Pro Cys Cys Arg Pro Thr Arg
               100                 105                 110
   Tyr Glu Ala Val Ser Phe Met Asp Val Asn Ser Thr Trp Arg Thr Val
           115                 120                 125
   Asp Arg Leu Ser Ala Thr Ala Cys Gly Cys Leu Gly
       130                 135                 140
<210> 7
   <211> 42
   <212> PRT
   <213> Artificial
<220>
   <223> Epitope
<400> 7
   Pro Pro Pro Gln Pro Ser Arg Pro Ala Pro Pro Pro Pro Ala Pro Pro
   1               5                   10                  15
   Ser Ala Leu Pro Arg Gly Gly Arg Ala Ala Arg Ala Gly Gly Pro Gly
               20                  25                  30
   Ser Arg Ala Arg Ala Ala Gly Ala Arg Gly
           35                  40
<210> 8
   <211> 15
   <212> PRT
   <213> Artificial
<220>
   <223> Epitope
<400> 8
   Gly Ala Leu Arg Pro Pro Pro Gly Ser Arg Pro Val Ser Gln Pro
   1               5                   10                  15
<210> 9
   <211> 14
   <212> PRT
   <213> Artificial
<220>
   <223> Epitope
<400> 9
   Pro Pro Pro Gln Pro Ser Arg Pro Ala Pro Pro Pro Pro Ala
   1               5                   10
<210> 10
   <211> 14
   <212> PRT
   <213> Artificial
<220>
   <223> Epitope
<400> 10
   Pro Pro Gln Pro Ser Arg Pro Ala Pro Pro Pro Pro Ala Pro
   1               5                   10
<210> 11
   <211> 14
   <212> PRT
   <213> Artificial
<220>
   <223> Epitope
<400> 11
   Pro Gln Pro Ser Arg Pro Ala Pro Pro Pro Pro Ala Pro Pro
   1               5                   10
<210> 12
   <211> 14
   <212> PRT
   <213> Artificial
<220>
   <223> Epitope
<400> 12
   Gln Pro Ser Arg Pro Ala Pro Pro Pro Pro Ala Pro Pro Ser
   1 5 10
<210> 13
   <211> 14
   <212> PRT
   <213> Artificial
<220>
   <223> Epitope
<400> 13
   Pro Ser Arg Pro Ala Pro Pro Pro Pro Ala Pro Pro Ser Ala
   1 5 10
<210> 14
   <211> 14
   <212> PRT
   <213> Artificial
<220>
   <223> Epitope
<400> 14
   Ser Arg Pro Ala Pro Pro Pro Pro Ala Pro Pro Ser Ala Leu
   1 5 10
<210> 15
   <211> 14
   <212> PRT
   <213> Artificial
<220>
   <223> Epitope
<400> 15
   Arg Pro Ala Pro Pro Pro Pro Ala Pro Pro Ser Ala Leu Pro
   1 5 10
<210> 16
   <211> 14
   <212> PRT
   <213> Artificial
<220>
   <223> Epitope
<400> 16
   Pro Ala Pro Pro Pro Pro Ala Pro Pro Ser Ala Leu Pro Arg
   1 5 10
<210> 17
   <211> 14
   <212> PRT
   <213> Artificial
<220>
   <223> Epitope
<400> 17
   Ala Pro Pro Pro Pro Ala Pro Pro Ser Ala Leu Pro Arg Gly
   1               5                   10
<210> 18
   <211> 14
   <212> PRT
   <213> Artificial
<220>
   <223> Epitope
<400> 18
   Pro Pro Pro Pro Ala Pro Pro Ser Ala Leu Pro Arg Gly Gly
   1               5                   10
<210> 19
   <211> 14
   <212> PRT
   <213> Artificial
<220>
   <223> Epitope
<400> 19
   Pro Pro Pro Ala Pro Pro Ser Ala Leu Pro Arg Gly Gly Arg
   1               5                   10
<210> 20
   <211> 14
   <212> PRT
   <213> Artificial
<220>
   <223> Epitope
<400> 20
   Pro Pro Ala Pro Pro Ser Ala Leu Pro Arg Gly Gly Arg Ala
   1               5                   10
<210> 21
   <211> 14
   <212> PRT
   <213> Artificial
<220>
   <223> Epitope
<400> 21
   Ala Pro Pro Ser Ala Leu Pro Arg Gly Gly Arg Ala Ala Arg
   1 5 10
<210> 22
   <211> 14
   <212> PRT
   <213> Artificial
<220>
   <223> Epitope
<400> 22
   Pro Pro Ser Ala Leu Pro Arg Gly Gly Arg Ala Ala Arg Ala
   1 5 10
<210> 23
   <211> 14
   <212> PRT
   <213> Artificial
<220>
   <223> Epitope
<400> 23
   Pro Ser Ala Leu Pro Arg Gly Gly Arg Ala Ala Arg Ala Gly
   1 5 10
<210> 24
   <211> 14
   <212> PRT
   <213> Artificial
<220>
   <223> Epitope
<400> 24
   Leu Pro Arg Gly Gly Arg Ala Ala Arg Ala Gly Gly Pro Gly
   1 5 10
<210> 25
   <211> 14
   <212> PRT
   <213> Artificial
<220>
   <223> Epitope
<400> 25
   Pro Arg Gly Gly Arg Ala Ala Arg Ala Gly Gly Pro Gly Ser
   1 5 10
<210> 26
   <211> 14
   <212> PRT
   <213> Artificial
<220>
   <223> Epitope
<400> 26
   Arg Gly Gly Arg Ala Ala Arg Ala Gly Gly Pro Gly Ser Arg
   1               5                   10
<210> 27
   <211> 14
   <212> PRT
   <213> Artificial
<220>
   <223> Epitope
<400> 27
   Gly Gly Arg Ala Ala Arg Ala Gly Gly Pro Gly Ser Arg Ala
   1               5                   10
<210> 28
   <211> 14
   <212> PRT
   <213> Artificial
<220>
   <223> Epitope
<400> 28
   Gly Arg Ala Ala Arg Ala Gly Gly Pro Gly Ser Arg Ala Arg
   1               5                   10
<210> 29
   <211> 14
   <212> PRT
   <213> Artificial
<220>
   <223> Epitope
<400> 29
   Arg Ala Ala Arg Ala Gly Gly Pro Gly Ser Arg Ala Arg Ala
   1               5                   10
<210> 30
   <211> 14
   <212> PRT
   <213> Artificial
<220>
   <223> Epitope
<400> 30
   Ala Arg Ala Gly Gly Pro Gly Ser Arg Ala Arg Ala Ala Gly
   1 5 10
<210> 31
   <211> 14
   <212> PRT
   <213> Artificial
<220>
   <223> Epitope
<400> 31
   Ala Gly Gly Pro Gly Ser Arg Ala Arg Ala Ala Gly Ala Arg
   1 5 10
<210> 32
   <211> 14
   <212> PRT
   <213> Artificial
<220>
   <223> Epitope
<400> 32
   Gly Gly Pro Gly Ser Arg Ala Arg Ala Ala Gly Ala Arg Gly
   1 5 10
<210> 33
   <211> 14
   <212> PRT
   <213> Artificial
<220>
   <223> Epitope
<400> 33
   Gly Ala Leu Arg Pro Pro Pro Gly Ser Arg Pro Val Ser Gln
   1 5 10
<210> 34
   <211> 14
   <212> PRT
   <213> Artificial
<220>
   <223> Epitope
<400> 34
   Ala Leu Arg Pro Pro Pro Gly Ser Arg Pro Val Ser Gln Pro
   1 5 10
<210> 35
   <211> 61
   <212> DNA
   <213> Synthetic
<400> 35
   atcgccgcca ccatggaact tggacttgga ggcctctcca cgctgtccca ctgcccctgg 60
   c 61
<210> 36
   <211> 65
   <212> DNA
   <213> Synthetic
<400> 36
   ctaggccagg ggcagtggga cagcgtggag aggcctccaa gtccaagttc catggcggcg 60
   gcgat 65
<210> 37
   <211> 12
   <212> PRT
   <213> Artificial
<220>
   <223> Epitope
<400> 37
   Met Arg Gly Ser His His His His His His Gly Ser
   1 5 10
<210> 38
   <211> 21
   <212> DNA
   <213> Artificial
<220>
   <223> Synthetic - made in laboratory
<400> 38
   aactggcctg tactcactca t 21
<210> 39
   <211> 64
   <212> DNA
   <213> Artificial
<220>
   <223> Synthetic - made in laboratory
<400> 39
   gatccgctgg cctgtactca ctcatttcaa gagaatgagt gagtacaggc cagttttttg 60
   gaaa 64
<210> 40
   <211> 63
   <212> DNA
   <213> Artificial
<220>
   <223> Synthetic - made in laboratory
<400> 40
   agcttttcca aaaaactggc ctgtactcac tcattctctt gaaatgagtg agtcaggcca        60
   gcg                                                                      63
<210> 41
   <211> 22
   <212> DNA
   <213> Artificial
<220>
   <223> Synthetic - made in laboratory
<400> 41
   ctttgcagac tggaccctta cc 22
<210> 42
   <211> 22
   <212> DNA
   <213> Artificial
<220>
   <223> Synthetic - made in laboratory
<400> 42
   agctcccatg agtgagtaca gg 22
<210> 43
   <211> 18
   <212> DNA
   <213> Artificial
<220>
   <223> Synthetic - made in laboratory
<400> 43
   atgatatcgc cgcgctcg 18
<210> 44
   <211> 19
   <212> DNA
   <213> Artificial
<220>
   <223> Synthetic - made in laboratory
<400> 44
   cgctcggtga ggatcttca 19